# EUROPEAN PATENT SPECIFICATION

(11) **EP 1 734 997 B1**
(45) Date of publication and mention of the grant of the patent: **02.09.2015**
(21) Application number: 05768245.2
(22) Date of filing: 30.03.2005
(51) Int. Cl.: A61K 39/395, A61P 1/00, A61P 11/06, A61P 37/00

(54) **NATALIZUMAB FOR USE IN TREATING DISEASES NEEDING STEROID TREATMENT**
NATALIZUMAB ZUR VERWENDUNG BEI DER BEHANDLUNG VON KRANKHEITEN MIT KORTIKOSTEROID-THERAPIE
NATALIZUMAB ET SON UTILISATION DANS LE TRAITEMENT DES MALADIES NECESSITANTS DES CORTICOSTEROIDS.

(30) Priority: 01.04.2004 US 558120 P
(43) Date of publication of application: 27.12.2006
(73) Proprietor: Biogen Idec MA Inc., Cambridge, Massachusetts 02142 (US)
(72) Inventor: LIEBERBURG, Ivan, Berkeley, California 94705 (US)
(74) Representative: Schüssler, Andrea
(86) International application number: PCT/US2005/010848
(87) International publication number: WO 2005/099776

(56) References cited:
- WO-A-02/070007
- WO-A-03/072040
- US-A1- 2004 009 169
- SORBERA L A ET AL: "Natalizumab. Treatment of IBD, treatment of multiple sclerosis: AN100226, AntegrenTM" DRUGS OF THE FUTURE, vol. 25, no. 9, September 2000 (2000-09), pages 917-921, XP002352328 ISSN: 0377-8282
- NEPOM G T: "Therapy of autoimmune diseases: clinical trials and new biologics" CURRENT OPINION IN IMMUNOLOGY, CURRENT BIOLOGY LTD, vol. 14, no. 6, 1 December 2002 (2002-12-01), pages 812-815, XP004390323 ISSN: 0952-7915
- FOX R J ET AL: "New directions in MS therapeutics: vehicles of hope" TRENDS IN IMMUNOLOGY, ELSEVIER, RAHWAY, NJ, US, vol. 25, no. 12, December 2004 (2004-12), pages 632-636, XP004628797 ISSN: 1471-4906
- ULBRICH H ET AL: "Leukocyte and endothelial cell adhesion molecules as targets for therapeutic interventions in inflammatory disease" TRENDS IN PHARMACOLOGICAL SCIENCES, ELSEVIER, AMSTERDAM, NL, vol. 24, no. 12, December 2003 (2003-12), pages 640-647, XP004476617 ISSN: 0165-6147

## Description

### Field of the Invention

This invention relates to the use of natalizumab or an immunologically active fragment thereof for the preparation of a medicament to be administered to a subject in a steroid sparing effective amount to reduce and/or eliminate a need for steroid treatment in the subject with a disease selected from the group consisting of inflammatory bowel disease (IBD), asthma, multiple sclerosis (MS), rheumatoid arthritis (RA), graft versus host disease (GVHD), host versus graft disease, spondyloarthropathies, and combinations thereof, wherein the subject is under treatment with steroids.

### Background of the Invention

Inflammation is a response of vascularized tissues to infection or injury and is affected by adhesion of leukocytes to the endothelial cells of blood vessels and their infiltration into the surrounding tissues. In normal inflammation, the infiltrating leukocytes release toxic mediators to kill invading organisms, phagocytize debris and dead cells, and play a role in tissue repair and the immune response. However, in pathologic inflammation, infiltrating leukocytes are over-responsive and can cause serious or fatal damage. *See, e.g.*, Hickey, Psychoneuroimmunology II (Academic Press 1990).

The integrins are a family of cell-surface glycoproteins involved in cell-adhesion, immune cell migration and activation. Alpha-4 integrin is expressed by all circulating leukocytes except neutrophils, and forms heterodimeric receptors in conjunction with either the beta-1 (β₁) or beta-7 (β₇) integrin subunits; both alpha-4 beta-1 (α₄β₁) and alpha-4 beta-7 (α₄β₇) play a role in migration of leukocytes across the vascular endothelium (Springer et al., Cell 1994, 76: 301-14; Butcher et al., Science 1996,272: 60-6) and contribute to cell activation and survival within the parenchyma (Damle et al., J. Immunol. 1993; 151: 2368-79; Koopman et al., J. Immunol 1994, 152: 3760-7; Leussink et al., Acta Neuropathol. 2002, 103: 131-136). α₄β₁ is constitutively expressed on lymphocytes, monocytes, macrophages, mast cells, basophils and eosinophils.

α₄β₁ (also known as very late antigen-4, VLA-4), binds to vascular cell adhesion molecule-1 (Lobb et al., J. Clin. Invest. 1994, 94: 1722-8), which is expressed by the vascular endothelium at many sites of chronic inflammation (Bevilacqua et al., 1993 Annu. Rev. Immunol. 11: 767-804; Postigo et al., 1993 Res. Immunol. 144: 723-35). α₄β₁ has other ligands, including fibronectin and other extracellular matrix (ECM) components.

The α₄β₇ dimer interacts with mucosal addressin cell adhesion molecule (MAdCAM-1), and mediates homing of lymphocytes to the gut (Farstad et al., 1997 Am. J. Pathol. 150: 187-99; Issekutz, 1991 J. Immunol. 147: 4178-84). Expression of MAdCAM-1 on the vascular endothelium is also increased at sites of inflammation in the intestinal tract of patients with inflammatory bowel disease (IBD) (Briskin et al., 1997 Am. J. Pathol. 151: 97-110).

Adhesion molecules such as α₄ integrins are potential targets for therapeutic agents. For instance, the VLA-4 receptor of which α₄ integrins is a subunit is an important target because of its interaction with a ligand residing on brain endothelial cells. Diseases and conditions resulting from brain inflammation have particularly severe consequences. In another example, the α₄β₇ integrin dimer is an important target due to its involvement in lymphocyte homing and pathological inflammation in the gastrointestinal tract.

α₄β₁ integrin is expressed on the extracellular surface of activated lymphocytes and monocytes, which have been implicated in the pathogenesis of acute inflammatory brain lesions and blood brain barrier (BBB) breakdown associated with multiple sclerosis (MS) (Coles et al., 1999 Ann. Neurol. 46(3): 296-304). Agents against α₄ integrin have been tested for their anti-inflammatory potential both *in vitro* and *in vivo. See* Yednock et al., Nature 1992, 356: 63-66; U.S. Patent No. 5,840,299 to Bendig et al., issued November 24, 1998, and U.S. Patent No. 6,001,809 to Thorsett et al., issued December 14, 1999. The *in vitro* experiments demonstrate that α₄ integrin antibodies block attachment of lymphocytes to brain endothelial cells. Experiments testing the effect of α₄ integrin antibodies on animals having the artificially induced condition simulating multiple sclerosis, experimental autoimmune encephalomyelitis (EAE), have demonstrated that administration of anti-α4 integrin antibodies prevents inflammation of the brain and subsequent paralysis in the animals. Collectively, these experiments identify anti-α4 integrin antibodies as potentially useful therapeutic agents for treating multiple sclerosis and other inflammatory diseases and disorders.

Steroids are often indicated for the treatment of inflammatory conditions, but cannot be used safely for extended periods of time. Steroids reduce inflammation, which weakening the immune system. Patients taking steroids may be come dependent, intolerant or refractory to steroids. Examples of steroids include hydrocortisone, betamethasone, fluorometholone, prednisolone, prednisone, medrysone, dexamethasone, methylprednisolone, rimexolone and triamcinolone.

Many serious side effects are associated with the use of steroids. The long-term use of steroids is discouraged because of the high risk of long-lasting side effects. Some common side effects include immune suppression, diabetes, weight gain, acne, cataracts, hypertension, psychosis, hirsutism, mood swings, gastritis, muscle weakness, easy bruising, osteoporosis, increased risk of infection and aseptic necrosis. Patients who take steroids for more than two months must often take calcium and vitamin D supplements or other medications, such as biphosphonates, to prevent osteoporosis. Long-term steroid use in children carries the risk of a delay in growth, as well as the side effects that occur in adults.

To date, no therapies have been discovered which allow for safe and effective treatment of inflammatory conditions such as Crohn's disease, asthma, multiple sclerosis (MS), rheumatoid arthritis (RA), graft versus host disease (GVHD), host versus graft disease, and various spondyloarthropathies, without the need for steroids or which allow for the tapering and/or discontinuation of steroids. Steroid sparing agents and methods for using these agents to reduce or eliminate the need for steroids in a subject that is unresponsive, intolerant or dependent on treatment with steroids in statistically significant amount are needed and continue to be sought out for the treatment of inflammatory diseases.

US 2004/009169 A1 generally discloses an anti-imflammaory agent that binds to alpha-4 integrin; said inflammation is caused by multiple sclerosis, Crohn's disease, inflammatory bowel disease or ulcerative colitis. US 2004/009169 A1 also discloses the general use of natalizumab in order to ameliorate said inflammatory diseases, as well as combination therapy with infliximab, interferons, non-steroidal anti-inflammatory drugs, methotrexate etc.

WO 03/072040 A2 is directed to the general use of an agent that specifically binds to alpha-4 integrin, more specifically natalizumab, alone or in combination therapy, in order to suppress pathological inflammation related to multiple sclerosis or diseases of the GI-tract such as ulcerative colitis.

Sorbera et al. (2000 Drugs of the Future 25(9): 917-921) generally states that natalizumab is an anti-alpha 4-integrin antibody and a candidate agent for the treatment of multiple sclerosis and inflammatory bowel disease. It discloses the results of double-blind, placebo controlled studies in the treatment of Crohn's disease, wherein a concomitant steroid treatment was not allowed in this study.

WO 02/070007 A1 discloses methods of preventing, treating, or ameliorating multiple sclerosis, ulcerative colitis and Crohn's disease by administering integrin αᵥβ₃ antagonists.

### Summary of the Invention

Based on the above, new compositions and methods of treating inflammatory diseases involving steroid use are needed which will effectively treat or inhibit these diseases such that patients can achieve long life spans and better quality of life.

This invention relates generally to the use of natalizumab or an immunologically active fragment thereof as a steroid sparing agent for the preparation of a medicament to be administered to a subject in a steroid sparing effective amount to reduce and/or eliminate a need for steroid treatment in the subject with a disease selected from the group consisting of inflammatory bowel disease (IBD), asthma, multiple sclerosis (MS), rheumatoid arthritis (RA), graft versus host disease (GVHD), host versus graft disease, spondyloarthropathies, and combinations thereof, wherein the subject is under treatment with steroids.

It has been surprisingly discovered that the agent of the present invention or rather that natalizumab or an immunologically active fragment thereof is steroid sparing. Steroids are often indicated for the treatment of inflammatory conditions, but cannot be used safely for extended periods of time. Steroids reduce inflammation, which weakening the immune system. Patients taking steroids may be come dependent, intolerant or refractory to steroids.

Accordingly, the agent of the present invention allow for safe and effective treatment of inflammatory conditions such as Crohn's disease, asthma, multiple sclerosis (MS), rheumatoid arthritis (RA), graft versus host disease (GVHD), host versus graft disease, and various spondyloarthropathies, without the need for steroids or which allow for the tapering and/or discontinuation of steroids.

The steroid sparing agent of the present invention is an antibody or an immunologically active fragment thereof, more specifically the anti-α₄ immunoglobulin, natalizumab (Tysabri®) or an immunologically active fragment thereof. As such, the anti-α₄ immunoglobulin is to be administered to a subject for treatment of a disease selected from the group consisting of inflammatory bowel diseases (IBD), asthma, multiple sclerosis (MS), rheumatoid arthritis (RA), graft versus host disease (GVHD), host versus graft disease, and various spondyloarthropathies. When administered in a steroid sparing effective amount, the anti-α₄ immunoglobulin permits the subject to be tapered from steroid therapy. Accordingly, it has been surprisingly discovered that when the anti-α₄ immunoglobulin is administered to a subject in a steroid sparing effective amount, the subject requires a therapeutically effective amount of steroids that is

less than would be required in the absence of administering the anti-α₄ immunoglobulin of the present invention.

The invention also relates generally to combination therapies for the treatment of these conditions. As such, the steroid sparing agent of the invention can be administered in combination with an immunosuppressant, wherein the immunosuppressant is not a steroid, an anti-TNF composition, a 5-ASA composition, and combinations thereof.

The invention relates to a pharmaceutical composition comprising a pharmaceutically acceptable carrier and a steroid sparing effective amount of the steroid sparing agent, as disclosed herein, which when administered to a subject in need thereof allows steroid use to be reduced or eliminated.

The composition of the invention may be administered by a variety of modes of administration including oral, parenteral (*e.g*., subcutaneous, subdural, intravenous, intramuscular, intrathecal, intraperitoneal, intracerebral, intraarterial, or intralesional routes of administration), topical, localized (*e.g*., surgical application or surgical suppository), rectal, and pulmonary (*e.g*., aerosols, inhalation, or powder). Preferably, the compositions of this invention are administered parenterally.

These and other objects, advantages, and features of the invention will become apparent to those persons skilled in the art upon reading the details of the methods and formulations as more fully described below.

### Brief Descriptions of the Drawings

Figure 1 shows a graph of the response to natalizumab when given to patients in a Crohn's disease trial *(see* Example 2).
Figure 2 shows a graph of the level of remission in response to natalizumab when given to patients in a Crohn's disease trial (*see* Example 2).
Figure 3 shows a graph of the level of remission in response to natalizumab when given to patients in a Crohn's disease trial (*see* Example 2) in various populations: the intention-to-treat population (ITT), elevated C-reactive protein population (CRP), the population unresponsive or intolerant to immunosuppressives (immuno UI). and the population unresponsive, intolerant to, or dependent upon steroids (steroid UID). These categorizations were based upon patient history of previous use of these medications.

### Detailed Description of the Invention

It is to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller, subject to any specifically excluded limit in the stated range. Where the stated range includes one or both of the limits, ranges excluding either both of those included limits are also included in the invention. Also contemplated are any values that fall within the cited ranges.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

### 1. Abbreviations and Definitions

In accordance with this detailed description, the following abbreviations and definitions apply. It must be noted that as used herein, the singular forms "a", "and", and "the" include plural referents unless the context clearly dictates otherwise. Thus, for example, reference to "an antibody" includes a plurality of such antibodies and reference to "the dosage" includes reference to one or more dosages and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

### 1.1 Abbreviations

The following abbreviations have been used herein.

| | |
|---|---|
| ACTH | adrenocorticotropic hormone |
| ANA | Anti-nuclear antibodies |
| aq or aq. | aqueous |
| BBB | blood brain barrier |
| C | constant region of an immunoglobulin |
| CD | Crohn's disease |
| CDAI | Crohn's disease activity index |
| cDNA | complementary deoxyribonucleic acid |
| CDR | complementarity determining region |
| CDR1 | complementarity determining region 1 |
| CDR2 | complementarity determining region 2 |
| CDR3 | complementarity determining region 3 |
| CNS | central nervous system |
| COX-2 | cyclooxygenase-2 |
| CRP | C-Reactive Protein |
| CS | Cockayne's syndrome |
| CSF | colony stimulating factor |
| DMSO | dimethylsulfoxide |
| DNA | deoxyribonucleic acid |
| EAE | experimental autoimmune encephalomyelitis |
| EBNA2 | Epstein-Barr virus nuclear antigen 2 |
| ECM | extracellular matrix |
| ELAMS | endothelial adhesion molecules |
| EM | electron microscopy |
| FACS | fluorescence activated cell sorter |
| FR | framework region |
| FR1 | framework region 1 |
| FR2 | framework region 2 |
| FR3 | framework region 3 |
| GM-CSF | granulocyte monocyte colony stimulating factor |
| GVHD | graft versus host disease |
| h or hr | hour |
| H | heavy chain of an immunoglobulin |
| HAMA | human anti-mouse antibody |
| H-E | hematoxylin-eosin |
| hex A | hexoaminidase A |
| HIC | hydrophobic interaction chromatography |
| HIG | human immunoglobulin |
| HMSN IV | hereditary motor and sensory neuropathy IV (also known as heredopathia atactica polyneuritiformis) |
| H₂O | water |
| ICAM-1 | intercellular adhesion molecule 1 |
| Ig | immunoglobulin |
| IgG | immunoglobulin G |
| IgM | immunoglobulin M |
| IL | interleukin |
| IL-1 | interleukin-1 |
| IL-2 | interleukin-2 |
| IL-8 | interleukin-8 |
| IBD | inflammatory bowel disease |
| IBDQ | inflammatory bowel disease questionairre |
| immuno UI | population unresponsive or intolerant to immunosuppressives |
| ITT | Intention-to-treat (including all subjects randomized, regardless of whether dosed) |
| L | light chain of an immunoglobulin |
| LFA-1 | lymphocyte function-related antigen 1- (also known as β₂ integrin, CD11a/CD18 and α_{L}β₂) |
| MAbs | monoclonal antibodies |
| Mac-1 | α_{M}β₂ integrin (also known as CD11b/CD18) |
| MAdCAM-1 | mucosal addressin cell adhesion molecule |
| MALDI/TOF MS | matrix-assisted laser desorption ionization/time-of-flight mass spectrometry |
| MCP-1 | monocyte chemotactic protein 1 |
| MeOH | methanol |
| MIP-1α | macrophage inflammatory protein 1 alpha |
| MIP-1β | macrophage inflammatory protein 1 beta |
| MLD | metachromatic leukodystrophy |
| MS | multiple sclerosis |
| N | normal |
| NSAID | nonsteroidal anti-inflammatory |
| PCR | polymerase chain reaction |
| PEG | polyethylene glycol |
| PKU | phenylketonuria |
| PLP | proteolipid protein |
| RNA | ribonucleic acid |
| rt | room temperature |
| RT-PCR | reverse transcription polymerase chain reaction |
| SAE | Serious adverse event |
| SDS PAGE | sodium dodecylsulphate polyacrylamide gel |
| SF-36 | Quality of Life Question |
| SAMIs | selective adhesion molecule inhibitors |
| sat or sat. | saturated |
| scFv | single chain Fv fragment |
| steroid UID | the population unresponsive, intolerant to, or dependent upon steroids |
| TGF-β | tumor growth factor beta |
| TLC or tlc | thin layer chromatography |
| TNF | tumor necrosis factor |
| TNF-α | tumor necrosis factor alpha |
| TNF-β | tumor necrosis factor beta |
| VCAM-1 | vascular cell adhesion molecule 1 |
| V_{H} | heavy chain of the variable domain |
| V_{L} | light chain of the variable domain |
| VLA-4 | very late antigen 4 (also known as alpha-4 beta-1, α₄β₁) |

### 1.2 Definitions

Abbreviations for the twenty naturally occurring amino acids follow conventional usage (IMMUNOLOGY-A SYNTHESIS (2nd ed., E. S. Golub & D. R. Gren, eds., Sinauer Associates, Sunderland, Mass., 1991)). Stereoisomers (*e.g*., D-amino acids) of the twenty conventional amino acids, unnatural amino acids such as α,α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, ω-N-methylarginine, and other similar amino acids and imino acids (*e.g.*, 4-hydroxyproline). Moreover, amino acids may be modified by glycosylation, phosphorylation and the like.

In the polypeptide notation used herein, the left-hand direction is the amino terminal direction and the right-hand direction is the carboxy-terminal direction, in accordance with standard usage and convention. Similarly, unless specified otherwise, the left-hand end of single-stranded polynucleotide sequences is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences."

The phrase "polynucleotide sequence" refers to a single or double-stranded polymer of deoxyribonucleotide or ribonucleotide bases read from the 5' to the 3' end. It includes self-replicating plasmids, infectious polymers of DNA or RNA and non-functional DNA or RNA.

The following terms are used to describe the sequence relationships between two or more polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, or may comprise a complete DNA or gene sequence. Generally, a reference sequence is at least 20 nucleotides in length, frequently at least 25 nucleotides in length, and often at least 50 nucleotides in length. Since two polynucleotides may each (1) comprise a sequence (*i.e.*, a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotides positions wherein a polynucleotide sequence may be compared to a reference sequence of at least 20 contiguous nucleotides and wherein the portion of the polynucleotide sequence in the comparison window may comprise additions or deletions (*i.e.*, gaps) of 20 percent or less as compared to the reference sequence (which does not comprise additions or deletions) for optimal alignment of the two sequences. Optimal alignment of sequences for aligning a comparison window may be conducted by the local homology algorithm of Smith & Waterman, Adv. Appl. Math. 2: 482 (1981), by the homology alignment algorithm of Needleman & Wunsch, J. Mol. Biol. 48: 443 (1970), by the search for similarity method of Pearson & Lipman, Proc. Natl. Acad. Sci. (USA) 85: 2444 (1988) by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (*i.e.*, resulting in the highest percentage of sequence similarity over the comparison window) generated by the various methods is selected. The term "sequence identity" means that two polynucleotide sequences are identical (*i.e.*, on a nucleotide-by-nucleotide basis) over the window of comparison. The term "percentage of sequence identity" is calculated by comparing two optimally aligned sequences over the window of comparison, determining the number of positions at which the identical nucleic acid base (*e.g.*, A, T, C, G, U, or I) occurs in both sequences to yield the number of matched positions, dividing the number of matched positions by the total number of positions in the window of comparison (*i.e.*, the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

As applied to polypeptides, the term "sequence identity" means peptides share identical amino acids at corresponding positions. The term "sequence similarity" means peptides have identical or similar amino acids (*i.e.*, conservative substitutions) at corresponding positions. The term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (*e.g.*, 99 percent sequence identity). Preferably, residue positions that are not identical differ by conservative amino acid substitutions. The term "substantial similarity" means that two peptide sequences share corresponding percentages of sequence similarity.

The term "substantially similar" as used herein is intended to mean any polypeptide that has an alteration in the sequence such that a functionally equivalent amino acid is substituted for one or more amino acids in the polypeptide, thus producing a change that has no or relatively little effect on the binding properties of the polypeptide. For example, one or more amino acid residues within the sequence can be substituted by another amino acid of a similar polarity or similar size.

The term "substantially pure" means an object species is the predominant species present (*i.e.*, on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 to 90 percent of all macromolecular species present in the composition. Most preferably, the object species is purified to essential homogeneity (contaminant species cannot be detected in the composition by conventional detection methods) wherein the composition consists essentially of a single macromolecular species.

For purposes of classifying amino acids substitutions as conservative or non-conservative, amino acids are grouped as follows: Group I (hydrophobic side chains): norleucine, met, ala, val, leu, ile; Group II (neutral hydrophilic side chains): cys, ser, thr; Group III (acidic side chains): asp, glu; Group IV (basic side chains): asn, gln, his, lys, arg; Group V (residues influencing chain orientation): gly, pro; and Group VI (aromatic side chains): trp, tyr, phe. Conservative substitutions involve substitutions between amino acids in the same class. Non-conservative substitutions constitute exchanging a member of one of these classes for another.

Amino acids from the variable regions of the mature heavy and light chains of immunoglobulins are designated Hx and Lxx respectively, where "x" is a number designating the position of an amino acids according to the scheme of Kabat *et al.,* SEQUENCES OF PROTEINS OF IMMUNOLOGICAL INTEREST (National Institutes of Health, Bethesda, Md. (1987) and (1991)) (hereinafter collectively referred to as "Kabat"). Kabat lists many amino acid sequences for antibodies for each subclass, and list the most commonly occurring amino acid for each residue position in that subclass. Kabat uses a method for assigning a residue number to each amino acid in a listed sequence, and this method for assigning residue numbers has become standard in the field. Kabat's scheme is extendible to other antibodies not included in the compendium by aligning the antibody in question with one of the consensus sequences in Kabat. The use of the Kabat numbering system readily identifies amino acids at equivalent positions in different antibodies. For example, an amino acid at the L50 position of a human antibody occupies the equivalence position to an amino acid position L50 of a mouse antibody.

In general, the term "reagent" or "agent" is used to denote a biologically active molecule that binds to a ligand receptor. For example, antibodies or fragments thereof which immunoreact with the VLA-4 receptor or VCAM-1 can eliminate the need for steroids in a subject unresponsive, intolerant or dependent on steroids. Peptides, or peptidomimetics or related compounds, which can act to bind the cell surface receptor, are also contemplated, and can be made synthetically by methods known in the art.

However, with reference to the present invention, the term "reagent" or "agent" or "compound" only includes natalizumab or an immunologically active fragment thereof.

A "steroid sparing agent" generally refers to agents that reduce or eliminate the need for steroids in a subject that is unresponsive, intolerant or dependent on treatment with steroids in a statistically significant amount. For example, such agents include immunoglobulins (*e.g*., antibodies, antibody fragments, and recombinantly produced antibodies or fragments), polypeptides (*e.g*., soluble forms of the ligand proteins for integrins) and small molecules, which when administered in an effective amount, reduces or eliminates the need for steroids in a subject that is unresponsive, intolerant or dependent on treatment with steroids. These agents may be anti-α₄ integrin agents (*e.g*. anti-α₄β₁ or anti-α₄β₇ antagonists) and anti-VCAM-1 agents. However, with reference to the present invention, such anti-α₄ integrin agents only include natalizumab or an immunologically active fragment thereof which when administered in an steroid sparing effective amount reduces or eliminates the need for steroids in a subject that is unresponsive, intolerant or dependent on treatment with steroids.

The term "anti-α₄ integrin agent" generally refers to any agent that binds specifically to an integrin comprising an α₄ subunit and inhibits activity of the integrin.

The term "integrin antagonist" generally refers to any agent that inhibits α₄ subunit-containing integrins from binding with an integrin ligand and/or receptor. For example, the integrin antagonist inhibits the α₄β₁ dimer and/or the α₄β₇ dimer from binding to its cognate ligand(s). Such antagonists can generally include anti-integrin antibodies or antibody homolog-containing proteins, as well as other molecules such as soluble forms of the ligand proteins for integrins. Soluble forms of the ligand proteins for α₄ subunit-containing integrins generally include soluble VCAM-1, VCAM-1 fusion proteins, or bifunctional VCAM-1/Ig fusion proteins.

By "natalizumab" or "Tysabri^{®}" is meant a humanized antibody against VLA-4 as described in commonly owned U.S. Patent Nos. 5,840,299 and 6,033,665. Also known are other VLA-4 specific antibodies. Such immunoglobulins generally include those immunoglobulins described in U.S. Patent Nos. 6,602,503 and 6,551,593, published U.S. Application No. 20020197233 (Relton et al.).

The term "efficacy" as used herein in the context of a chronic dosage regime refers to the effectiveness of a particular treatment regime. Efficacy can be measured based on change the course of the disease in response to an agent of the present invention. For example, in the treatment of MS, efficacy can be measured by the frequency of relapses in relapsing-remitting MS, and by the presence or absence of new lesions in the central nervous system as detected using methods such as MRI.

The term "success" as used herein in the context of a chronic treatment regime refers to the effectiveness of a particular treatment regime. This includes a balance of efficacy, toxicity (*e.g*., side effects and patient tolerance of a formulation or dosage unit), patient compliance, and the like. For a chronic administration regime to be considered "successful" it must balance different aspects of patient care and efficacy to produce the most favorable patient outcome.

The terms "specifically binds" or "binds specifically" as used herein refer to the situation in which one member of a specific binding pair will not show any significant binding to molecules other than its specific binding partner (*e.g*., an affinity of about 1000x or more for its binding partner). In the present invention, the small compounds, such as *N*-[*N*-(3-pyridinesulfonyl)-L-3,3-dimethyl-4-thiaprolyl]-*O-*[1-methylpiperzain-4-ylcarbonyl]-L-tyrosine isopropyl ester, will not show significant binding to any polypeptide other than an α₄ integrin or a receptor comprising an α₄ integrin. For example, the small compounds used in the methods of the invention that bind to an α₄ integrin with a binding affinity of greater than 0.3 nM are said to bind specifically to an α₄ integrin.

The terms "elicits an immune response" and "elicits a host immune response" as used herein refer to the production of an immune response to a receptor comprising an α₄ integrin in a subject upon introduction of an agent of the invention to the subject. An immune response in the subject can be characterized by a serum reactivity with an α₄ integrin receptor that is at least twice that of an untreated subject, more preferably three times the reactivity of an untreated subject, and even more preferably at least four times the reactivity of an untreated subject, with serum immunoreactivity measured using a serum dilution of approximately 1:100.

The term "pharmaceutically acceptable carrier or excipient" is intended to mean any compound used in forming a part of the formulation that is intended to act merely as a carrier, *i.e.*, not intended to have biological activity itself. The pharmaceutically acceptable carrier or excipient is generally safe, non-toxic and neither biologically nor otherwise undesirable. A pharmaceutically acceptable carrier or excipient as used in the specification and claims includes both one and more than one such carrier.

The terms "treating", and "treatment" and the like are used herein to generally mean obtaining a desired pharmacological and physiological effect. More specifically, the reagent of the present invention is used to reduce or eliminate the need for steroids in a subject that is unresponsive, intolerant or dependent on treatment with steroids. Thus, the effect may be prophylactic in terms of preventing or partially preventing a disease, symptom or condition thereof and/or may be therapeutic in terms of a partial or complete cure of a disease, condition, symptom or adverse effect attributed to the disease depending on the condition or disease being treated. The term "treatment", as used herein, covers any treatment of a disease in a mammal, particularly a human, and includes: (a) preventing the disease from occurring in a subject which may be predisposed to the disease but has not yet been diagnosed as having it; (b) inhibiting the disease, *i.e*., arresting its development; or (c) relieving the disease, *i.e*., causing regression of the disease and/or its symptoms or conditions. The invention is directed towards treating a patient's suffering from disease related to pathological inflammation. The present invention is involved in preventing, inhibiting, or relieving adverse effects attributed to pathological inflammation, preferably an inflammatory bowel disease such as Crohn's disease, asthma, MS, RA or various spondyloarthropathies, over long periods of time and/or are such caused by the physiological responses to inappropriate inflammation present in a biological system over long periods of time.

By "therapeutically effective amount" is meant an amount of an agent, a reagent, or a combination of reagents that when administered to a mammal is sufficient to reduce or eliminate the need for steroids in a subject that is unresponsive, intolerant or dependent on treatment with steroids in statistically significant amount.

By the term "steroid sparing effective amount" is meant an amount of the agent, the reagent, or the composition of the present invention effective to that reduces or eliminates the need for steroids in a subject that is unresponsive, intolerant or dependent on treatment with steroids in statistically significant amount. The "steroid sparing effective amount" will vary depending on the compound or composition, the specific disease to be treated and its severity, and the age, weight, etc., of the mammal to be treated.

By "chronic administration" is meant administration of the agent, the reagent, or the combination therapy of the invention in an amount and periodicity to result in the reduction or the elimination of the need for steroids in a subject that is unresponsive, intolerant or dependent on treatment with steroids. Administration is preferably biweekly, weekly, monthly, or every other month, but can be daily. More preferably the treatment is weekly or monthly and is administered for 6 months to several years or the remainder of the patient's life depending on the disease or condition being treated.

Additional definitions relevant to the compounds of formula I to formula IX are as defined therein.

### 2. General Aspects of the Invention

### 2.1 Diseases and conditions

The following disease and/or conditions may be treated and/or prevented using the steroid sparing agent of the present invention.

### 2.1.1 Inflammatory Bowel Diseases

Inflammatory bowel disease (IBD) is the general name given to diseases that cause inflammation in the intestines. Inflammatory bowel diseases include, Crohn's disease and ulcerative colitis.

### 2.1.1.1 Crohn's Disease

Crohn's disease causes inflammation in the small intestine. Crohn's disease usually occurs in the lower part of the small intestine, *i.e.*, the illium, but it can affect any part of the digestive tract. The inflammation extends deep into the lining of the affected organ, causing pain and causing the intestines to empty frequently. Crohn's disease may also be called ileitis or enteritis. Crohn's disease affects men and women equally and may run in some families. About 20 percent of people with Crohn's disease have a blood relative with some form of IBD.

The cause of Crohn's disease is uncertain. One theory is that the immune system reacts to a virus or a bacterium by causing ongoing inflammation in the intestine. Patients suffering from Crohn's disease tend to have abnormalities of the immune system, but it is uncertain whether these abnormalities are a cause or result of the disease.

The most common symptoms of Crohn's disease include abdominal pain, often in the lower right area, and diarrhea. Rectal bleeding, weight loss, and fever may also occur. Bleeding may be serious and persistent, leading to anemia. Children with Crohn's disease may suffer from delayed development and stunted growth.

The most common complication of Crohn's disease is blockage of the intestine. Blockage occurs because Crohn's disease causes a thickening of the intestinal wall with swelling and scar tissue, narrowing the intestinal passage. Crohn's disease may also cause sores and ulcers that tunnel through the affected area into surrounding tissues such as the bladder, vagina, or skin. The tunnels, called fistulas, are a common complication and often become infected. Sometimes fistulas can be treated with medication, but often require surgery.

Nutritional complications, such as deficiencies of proteins, calories and vitamins, are common in Crohn's disease. Other complications associated with Crohn's disease include arthritis, skin problems, inflammation in the eyes or mouth, kidney stones, gallstones, or other diseases of the liver and biliary system.

Treatment for Crohn's disease depends on the location and severity of disease, complications, and response to previous treatment. The goals of treatment are to control inflammation, correct nutritional deficiencies, and relieve symptoms such abdominal pain, diarrhea, and rectal bleeding. Treatment may include drugs, nutritional supplements, surgery, or a combination of these options. At this time, treatment there is no cure. Some patients have long periods of remission, free of symptoms. However, Crohn's disease usually recurs at various times over a person's lifetime. Predicting when a remission may occur or when symptoms will return is not possible.

Most patients are first treated with drugs containing mesalamine, a substance that helps control inflammation. Sulfasalazine is also commonly used. Patients who do not benefit from it, or who cannot tolerate it, may be put on other mesalamine-containing drugs, generally known as 5-ASA agents, such as Dipentum®, or Pentasa®. Possible side effects of mesalamine preparations include nausea, vomiting, heartburn, diarrhea, and headache.

Some patients are administered steroids, such as budesonide, to control inflammation. These drugs are the most effective for active Crohn's disease, but they can cause serious side effects, including greater susceptibility to infection. Drugs that suppress the immune system are also used to treat Crohn's disease. The most common include 6-mercaptopurine and azathioprine. Immunosuppressive agents work by blocking the immune reaction that contributes to inflammation. These drugs may cause side effects such as nausea, vomiting, and diarrhea, and lower a patient's resistance to infection.

Antibiotics are used to treat bacterial overgrowth in the small intestine caused by stricture, fistulas, or prior surgery. For this common problem, the doctor may prescribe antibiotics including ampicillin, sulfonamide, cephalosporin, tetracycline, or metronidazole.

Biologics are also used in the treatment of Crohn's disease. Infliximab (Remicade®) is indicated for the treatment of moderate to severe Crohn's disease that does not respond to standard therapies (*i.e.*, mesalamine substances, corticosteroids and immunosuppressive agents) and for the treatment of open, draining fistulas. Infliximab is an anti-tumor necrosis factor (TNF) substance. TNF is a protein produced by the immune system that may cause the inflammation associated with Crohn's disease.

Surgery to remove part of the intestine can help Crohn's disease but cannot cure it. The inflammation tends to return to the area of intestine adjacent to that has been removed. Many Crohn's disease patients require surgery, either to relieve symptoms that do not respond to medical therapy, or to correct complications such as blockage, perforation, abscess or bleeding in the intestine. Some patients must have their entire colon removed by colectomy. *See* Harrison's PRINCIPLES OF INTERNAL MEDICINE; 13th Ed., (1994) McGraw Hill, New York, pp. 1403-1405; THE PHYSICIAN'S DESK REFERENCE; 58th Ed. (2004) Thomson PDR, Montvale, NJ, pp. 402, 1130, 2707, 3153-3155, 3173.

### 2.1.1.2 Ulcerative Colitis

Ulcerative Colitis is a chronic, inflammatory, and ulcerative disease arising in the colonic mucosa. The cause of ulcerative colitis is unknown. Evidence suggests that a genetic predisposition causes an unregulated intestinal immune response to an environmental, dietary, or infectious agent. However, no inciting antigen has been identified.

Pathologic changes begin with degeneration of the reticulin fibers beneath the mucosal epithelium, occlusion of the subepithelial capillaries, and progressive infiltration of the lamina propria with plasma cells, eosinophils, lymphocytes and mast cells. Crypt abscesses, epithelial necrosis, and mucosal ulceration ultimately develop. The disease usually begins in the rectosigmoid and may extend into the entire colon, or it may involve most of the large bowel.

Symptoms include bloody diarrhea, peritonitis, and profound toxemia. Some cases develop following a documented infection (*i.e.*, by amebiasis or bacillary dysentery). Malaise, fever, anemia, anorexia, weight loss, leukocytosis and hypoalbuminemia may be present. Bleeding is the most common local complication. Another severe complication, toxic colitis, occurs when extension of ulceration results in localized ileus and peritonitis. As toxic colitis progresses, the colon loses muscular tone and begins to dilate within hours or days.

Toxic megacolon (or toxic dilation) exists when the diameter of the transverse colon exceeds 6 centimeters, resulting in a high fever, leukocytosis, abdominal pain, and rebound tenderness. Treatment must be given in the early stages to avoid dangerous complications, such as perforation, generalized peritonitis and septicemia. The incidence of colon cancer is increased when the entire colon is involved and the disease lasts for greater than ten years, independent of disease activity. Although cancer incidence is highest in cases of universal ulcerative colitis, the risk is significantly increased with any extent of ulcerative colitis above the sigmoid.

Other complications include peripheral arthritis, ankylosing spondylitis, sacroiliitis, anterior uveitis, erythema nodosum, skin complications, and in children, retarded growth and development. Liver disease may manifest as fatty liver or more seriously as autoimmune hepatitis, primary sclerosing cholangitis, or cirrhosis.

Ulcerative colitis is chronic with repeated exacerbations and remissions. Nearly one third of patients with extensive ulcerative colitis require surgery. Total proctocolectomy is curative: Life expectancy and quality of life are restored to normal, and the risk of colon cancer is eliminated.

Ulcerative colitis symptoms may respond to anti-diarrheal medications and changes in diet. Moderate to severe symptoms may require one or more medications. For disease in the rectum alone, topical therapy is indicated. Inflammation throughout the colon requires medication that acts on the whole body, such as medications to suppress the immune system (azathioprine, 6-mercaptopurine, or cyclosporine) and to control inflammation (steroids). *See* HARRISON'S PRINCIPLES OF INTERNAL MEDICINE; 13th Ed., (1994) McGraw Hill, New York, pp. 1403-1405; THE PHYSICIAN's DESK REFERENCE; 58th Ed. (2004) Thomson PDR, Montvale, NJ, pp. 402, 1130, 2707, 3153-3155, 3173.

### 2.1.2 Graft versus Host Disease (GVHD) and Host versus Graft Disease

Graft versus Host Disease (GVHD) is a rare disorder that can strike persons whose immune system is suppressed and have either received a blood transfusion or a bone marrow transplant. Host versus Graft Disease occurs in patients with suppressed immune systems and who have received an organ transplant. Symptoms for these conditions may include skin rash, intestinal problems similar to colitis, and liver dysfunction.

With GVHD, immunologically competent donor T cells react against antigens in an immunologically depressed recipient. Symptoms of acute GVHD include fever, exfoliative dermatitis, hepatitis with hyperbilirubinemia, vomiting, diarrhea and abdominal pain, which may progress to an ileus, and weight loss. GVHD continues to be the major cause of mortality and severe morbidity after allogeneic bone marrow transplants (BMT).

About 1/3 to 1/2 of bone marrow transplant recipients develop a chronic form of GVHD. Although the skin, liver, and gut remain the organs primarily affected, other areas of involvement (*i.e*, joint, lung) are also noted. Ultimately, 20 to 40% of patients die of complications associated with GVHD.

One method of treatment is the removal of T cells from the donor marrow with monoclonal antibodies, using a rosetting technique or mechanical separation, before reinfusion of the marrow. T-cell depletion has been very effective in decreasing both the incidence and severity of GVHD. However, the incidences of engraftment failure and relapse are increased. A possible explanation is that the cytokines generated in the graft versus host reaction promote stem cell multiplication and maturation necessary for engraftment. Other agents used to prevent or treat GVHD include methotrexate, corticosteroids, and monoclonal antibodies against antigens expressed on mature T cells.

GVHD may also follow blood transfusions in exceptional cases, because even small numbers of donor T cells can cause GVHD. Such situations include intrauterine fetal blood transfusions and transfusions in immunodepressed patients, such as those with bone marrow transplant recipients, leukemia, lymphoma, neuroblastoma, Hodgkin's and non-Hodgkin's lymphoma. *See* THE MERCK MANUAL OF MEDICAL INFORMATION (1997), Merck Research Laboratories, West Point, PA, pp. 836-837.

### 2.1.3 Multiple Sclerosis

Multiple sclerosis (MS) is a chronic neurologic disease, which appears in early adulthood and progresses to a significant disability in most cases. There are approximately 350,000 cases of MS in the United States alone. Outside of trauma, MS is the most frequent cause of neurologic disability in early to middle adulthood.

The cause of MS is yet to be determined. MS is characterized by chronic inflammation, demyelination and gliosis (scarring). Demyelination may result in either negative or positive effects on axonal conduction. Positive conduction abnormalities include slowed axonal conduction, variable conduction block that occurs in the presence of high-but not low-frequency trains of impulses or complete conduction block. Positive conduction abnormalities include ectopic impulse generation, spontaneously or following mechanical stress and abnormal "cross-talk" between demyelinated exons.

T cells reactive against myelin proteins, either myelin basic protein (MBP) or myelin proteolipid protein (PLP) have been observed to mediate CNS inflammation in experimental allergic encephalomyelitis. Patients have also been observed as having elevated levels of CNS immunoglobulin (Ig). It is further possible that some of the tissue damage observed in MS is mediated by cytokine products of activated T cells, macrophages or astrocytes.

Today, 80% patients diagnosed with MS live 20 years after onset of illness. Therapies for managing MS include (1) treatment aimed at modification of the disease course, including treatment of acute exacerbation and directed to long-term suppression of the disease; (2) treatment of the symptoms of MS; (3) prevention and treatment of medical complications, and (4) management of secondary personal and social problems.

The onset of MS may be dramatic or so mild as to not cause a patient to seek medical attention. The most common symptoms include weakness in one or more limbs, visual blurring due to optic neuritis, sensory disturbances, diplopia and ataxia. The course of disease may be stratified into three general categories: (1) relapsing MS, (2) chronic progressive MS, and (3) inactive MS. Relapsing MS is characterized by recurrent attacks of neurologic dysfunction. MS attacks generally evolve over days to weeks and may be followed by complete, partial or no recovery. Recovery from attacks generally occurs within weeks to several months from the peak of symptoms, although rarely some recovery may continue for 2 or more years.

Chronic progressive MS results in gradually progressive worsening without periods of stabilization or remission. This form develops in patients with a prior history of relapsing MS, although in 20% of patients, no relapses can be recalled. Acute relapses also may occur during the progressive course.

A third form is inactive MS. Inactive MS is characterized by fixed neurologic deficits of variable magnitude. Most patients with inactive MS have an earlier history of relapsing MS.

Disease course is also dependent on the age of the patient. For example, favourable prognostic factors include early onset (excluding childhood), a relapsing course and little residual disability 5 years after onset. By contrast, poor prognosis is associated with a late age of onset (*i.e.*, age 40 or older) and a progressive course. These variables are interdependent, since chronic progressive MS tends to begin at a later age that relapsing MS. Disability from chronic progressive MS is usually due to progressive paraplegia or quadriplegia (paralysis) in patients. In one aspect of the invention, patients will preferably be treated when the patient is in remission rather then in a relapsing stage of the disease.

Short-term use of either adrenocorticotropic hormone or oral corticosteroids (*e.g.*, oral prednisone or intravenous methylprednisolone) is the only specific therapeutic measure for treating patients with acute exacerbation of MS.

Newer therapies for MS include treating the patient with interferon beta-1b, interferon beta-la, and Copaxone^{®} (formerly known as copolymer 1). These three drugs have been shown to significantly reduce the relapse rate of the disease. These drugs are self-administered intramuscularly or subcutaneously.

### 2.1.4 Asthma

Asthma is a disease of the respiratory system that involves inflammation of the bronchial tubes, or airways, which carry air to the lungs. The airways overreact to allergens, as well as to smoke, cold air, and/or other environmental factors. The airways narrow, leading to difficulty breathing. Allergens can cause chronic inflammation.

Asthma often develops in childhood or the teen years, and is the most common chronic childhood disease. Most cases of asthma can be controlled. However, in severe cases, asthma episodes can be fatal. The number of cases of asthma has grown steadily in the past 30 years, making it one of the leading public health problems in the United States and the rest of the world.

Asthma is caused by genetic, environmental, and immunological factors, which combine to cause inflammation that can lead to asthma episodes. In some patients, the inflamed airways overreact to substances in the environment, such as smoke or cold air. In other patients, the immune system releases cells that cause inflammation in response to allergens.

Asthma may develop at different times and from a variety of factors. Cigarette smoke and air pollution may cause an attack. In addition, expressions of strong emotions, such as laughing or crying hard, can cause an attack,

Symptoms of an asthma episode can be mild to severe and may include, but are not limited to, wheezing, coughing, chest tightness, rapid, shallow breathing or difficulty breathing, shortness of breath, and tiring quickly during exercise.

Treatment involves taking medication to control inflammation and asthma episodes, and avoiding substances that increase inflammation. If inflammation is not controlled, asthma can lead to permanent changes in the bronchial tubes.

Inhaled corticosteroids (such as budesonide and fluticasone), reduce inflammation and are a common treatment for persistent asthma. In rare cases, oral corticosteroids (such as prednisone and dexamethasone) may be used to help control asthma. Long-acting beta2-agonists (such as salmeterol and formoterol) may also be indicated. Medications administered for quick relief include short-acting beta2-agonists (such as albuterol and terbutaline), and anticholinergics (such as ipratropium). *See* THE MERCK MANUAL OF MEDICAL INFORMATION (1997), Merck Research Laboratories, West Point, PA, pp. 133-137.

### 2.1.5 Rheumatoid Arthritis

Rheumatoid arthritis (RA) is a chronic syndrome characterized by inflammation of the peripheral joints, resulting in progressive destruction of articular and periarticular structures. The cause of rheumatoid arthritis is unknown. However, a genetic predisposition has been identified and, in white populations, localized to a pentapeptide in the HLA-DR 1 locus of class II histocompatibility genes. Environmental factors may also play a role. Immunologic changes may be initiated by multiple factors. About 1% of all populations are affected, women two to three times more often than men. Onset may be at any age, most often between 25 and 50 yr.

Prominent immunologic abnormalities that may be important in pathogenesis include immune complexes found in joint fluid cells and in vasculitis. Plasma cells produce antibodies that contribute to these complexes. Lymphocytes that infiltrate the synovial tissue are primarily T helper cells, which can produce pro-inflammatory cytokines. Increased adhesion molecules contribute to inflammatory cell emigration and retention in the synovial tissue.

Rheumatoid nodules occur in up to 30% of patients, usually subcutaneously at sites of chronic irritation. Vasculitis can be found in skin, nerves, or visceral organs in severe cases of RA but is clinically significant in only a few cases.

The onset is usually insidious, with progressive joint involvement, but may be abrupt, with simultaneous inflammation in multiple joints. Tenderness in nearly all inflamed joints and synovial thickening are common. Initial manifestations may occur in any joint.

Stiffness lasting less than 30 minutes on arising in the morning or after prolonged inactivity is common. Subcutaneous rheumatoid nodules are not usually an early manifestation. Visceral nodules, vasculitis causing leg ulcers or mononeuritis multiplex, pleural or pericardial effusions, lymphadenopathy, Felty's syndrome, Sjögren's syndrome, and episcleritis are other manifestations. Fever may be present

As many as 75% of patients improve symptomatically with conservative treatment during the first year of disease. However, less than 10% are eventually severely disabled despite full treatment. The disease greatly affects the lives of most RA patients. Complete bed rest is occasionally indicated for a short period during the most active, painful stage of severe disease. In less severe cases, regular rest should be prescribed.

Nonsteroidal anti-inflammatory drugs may provide important symptomatic relief and may be adequate as simple therapy for mild RA, but they do not appear to alter the long-term course of disease. Salicylates, such as aspirin, may be used for treatment.

Gold compounds usually are given in addition to salicylates or other NSAIDs if the latter do not sufficiently relieve pain or suppress active joint inflammation. In some patients, gold may produce clinical remission and decrease the formation of new bony erosions. Parenteral preparations include gold sodium thiomalate or gold thioglucose. Gold should be discontinued when any of the above manifestations appear. Minor toxic manifestations (*e.g*., mild pruritus, minor rash) may be eliminated by temporarily withholding gold therapy, then resuming it cautiously about 2 wk after symptoms have subsided. However, if toxic symptoms progress, gold should be withheld and the patient given a corticosteroid. A topical corticosteroid or oral prednisone 15 to 20 mg/day in divided doses is given for mild gold dermatitis; larger doses may be needed for hematologic complications. A gold chelating drug, dimercaprol 2.5 mg/kg IM, may be given up to four to six times/day for the first 2 days and bid for 5 to 7 days after a severe gold reaction.

Hydroxychloroquine can also control symptoms of mild or moderately active RA. Toxic effects usually are mild and include dermatitis, myopathy, and generally reversible corneal opacity. However, irreversible retinal degeneration has been reported. Sulfasalazine may also be used for treatment of RA.

Oral penicillamine may have a benefit similar to gold and may be used in some cases if gold fails or produces toxicity in patients with active RA. Side effects requiring discontinuation are more common than with gold and include marrow suppression, proteinuria, nephrosis, other serious toxic effects (*e.g*., myasthenia gravis, pemphigus, Goodpasture's syndrome, polymyositis, a lupus-like syndrome), rash, and a foul taste.

Steroids are the most effective short-term anti-inflammatory drugs. However, their clinical benefit for RA often diminishes with time. Steroids do not predictably prevent the progression of joint destruction. Furthermore, severe rebound follows the withdrawal of corticosteroids in active disease. Contraindications to steroid use include peptic ulcer, hypertension, untreated infections, diabetes mellitus, and glaucoma.

Immunosuppressive drugs are increasingly used in management of severe, active RA. However, major side effects can occur, including liver disease, pneumonitis, bone marrow suppression, and, after long-term use of azathioprine and malignancy.

Joint splinting reduces local inflammation and may relieve severe local symptoms. Active exercise to restore muscle mass and preserve the normal range of joint motion is important as inflammation subsides but should not be fatiguing. Surgery may be performed while the disease is active.

### 2.1.6 Spondyloarthropathies

The spondyloarthropathies are a family of diseases including ankylosing spondylitis, psoriatic arthritis, Reiter's syndrome, and arthritis associated with inflammatory bowel disease.

### 2.1.6.1 Ankylosing spondylitis,

Ankylosing Spondylitis (AS) is a form of arthritis that is chronic and most often affects the spine. It causes fatigue, pain and stiffness, with swelling and limited motion in the low back, middle back, neck, and hips. Although there is no cure, treatment can usually control symptoms and prevent the condition from getting worse. Complications of ankylosing spondylitis include iritis, difficulty breathing due to curving of the upper body and stiffening of the chest wall.

In time, continued inflammation of the ligaments and joints of the spine causes the spine to fuse together (ankylosis), leading to loss of motion in the neck and low back. As the spine fuses, or stiffens, a fixed bent-forward deformity (kyphosis) can result, leading to significant disability. The inflammation of ankylosing spondylitis can affect other parts of the body, most commonly other joints and the eyes, but sometimes the lungs and heart valves.

Ankylosing spondylitis affects 1 in every 100 people. It is more common in men than in women, and the condition usually begins in the late teens or early adulthood. Treatment includes exercise and physical therapy to help reduce stiffness and maintain good posture and mobility, and medications for pain and inflammation, including steroids.

### 2.1.6.2 Psoriatic Arthritis

Psoriatic Arthritis (PsA) is characterized by a swelling of the joints that develops in some patients with psoriasis. Psoriatic arthritis displays the symptoms of other types of arthritis, such as stiff, painful and swollen joints. Untreated psoriatic arthritis can cause bone loss and deformation of the joints. The pain and swelling of psoriatic arthritis are caused by an overactive immune system, which enflames the tissues around the joint. Symptoms flare-up and recede periodically. Symmetric arthritis is the most common type of psoriatic arthritis, making up about 50% of all cases. The symptoms occur on both sides of the body. Symptoms are similar to rheumatoid arthritis, and symmetric arthritis can cause permanent damage to the joints. Asymmetric arthritis, the second most common type of psoriatic arthritis, is milder and only causes symptoms on one side of the body.

Distal interphalangeal predominant (DIP), a less common form of psoriatic arthritis, affects the joints close to the fingernails and toenails. The nails are often affected by the condition as well. Spondylitis can make movement painful, especially in the neck and back. It can also cause inflammation of the spinal column. Arthritis mutilans is a frequently debilitating and destructive form of psoriatic arthritis. It often affects the hands and feet, as well as the back and neck, and it can result in permanent deformity.

The symptoms of psoriatic arthritis are similar to those of other kinds of arthritis. They include stiffness in the joints, pain or swelling in the joints, irritation and redness of the eye. The usual symptoms of psoriasis include red, scaly patches of skin.

Common treatments include nonsteroidal anti-inflammatory drugs (NSAIDs. They include a number of over-the-counter pain medications, such as aspirin and ibuprofen. However, chronic usage of these medications can be dangerous and cause gastrointestinal problems. Cox-2 inhibitors are a class of NSAIDs that are often used to treat psoriatic arthritis. Side effects include nausea and headache.

Immunosuppressants are more powerful drugs that are used for cases of psoriatic arthritis that don't respond to milder medications. Drugs in this class are used for systemic therapy of psoriasis, such as methotrexate, which act by suppressing the immune system. They may also cause serious side effects and raise the risk of infection. Azulfidine is also often prescribed. Certain drugs used to prevent malaria can help with symptoms, and they are sometimes prescribed for psoriatic arthritis as well.

Oral steroids are often indicated to help clear acute joint pain, although steroids cannot be used safely for long periods of time. However, stopping treatment with steroids suddenly can also cause a flare-up of symptoms. Biologics are also used to treat psoriasis. They work by targeting the immune system response that causes the symptoms of psoriasis, preventing the joints from becoming inflamed. Biologic medications may also make the immune system more susceptible to infections.

### 2.1.6.3 Reiter's Syndrome

Reiter's syndrome, also called reactive arthritis, is a form of arthritis that, in addition to joints, also affects the eyes, urethra and skin.

Reiter's syndrome is characterized by a number of symptoms in different organs of the body that may or may not appear at the same time. The disease may be acute or chronic, with sudden remissions or recurrences. Reiter's syndrome primarily affects males between the ages of 20 and 40. Those with human immunodeficiency virus (HIV) are at a particularly high risk.

The cause of Reiter's syndrome is unknown, but research suggests the disease is caused by a combination of genetic predisposition and other factors. Reiter's syndrome often follows infection with *Chlamydia trachomatis* or *Ureaplasma urealyticum*.

The first symptoms of Reiter's syndrome are inflammation of the urethra or the intestines, followed by arthritis. The arthritis usually affects the fingers, toes, ankles, hips, and knee joints. Other symptoms include inflammation of the urethra, with painful urination and discharge, mouth ulcers, inflammation of the eye and Keratoderma blennorrhagica (patches of scaly skin on the palms, soles, trunk, or scalp).

There is no specific treatment for Reiter's syndrome. Joint inflammation is usually treated with nonsteroidal anti-inflammatory drugs (NSAIDs). Skin eruptions and eye inflammation can be treated with steroids. The prognosis for Reiter's syndrome varies. Some patients develop complications that include inflammation of the heart muscle, inflammation with stiffening of the spine, glaucoma, progressive blindness, abnormalities of the feet or accumulation of fluid in the lungs.

Other spondyloarthropathies include, but are not limited to, spondylitis of inflammatory bowel Disease (IBD SpA), Undifferentiated Spondyloarthropathy (uSpA), juvenile spondyloarthropathy (JSpA).

*See* THE MERCK MANUAL OF MEDICAL INFORMATION (1997), Merck Research Laboratories, West Point, PA, 243.

### 3. Administration

In a general sense, the method of the invention does not involve any particular mode of administration, because the mode of administration is dependent upon the form of the active agent and the formulation developed to administer the active agent. Modes of administration include oral, parenteral (*e.g*., subcutaneous, subdural, intravenous, intramuscular, intrathecal, intraperitoneal, intracerebral, intraarterial, or intralesional routes of administration), topical, localized (*e.g*., surgical application or surgical suppository), rectal, and pulmonary (*e.g*., aerosols, inhalation, or powder). Preferably, the route of administration is parenteral. The route of administration is based on the composition being administered (*e.g*., immunoglobulin being administered intravenously versus small compound being administered orally), tissue targeting (*e.g*., intrathecal administration to target the site of a spinal cord injury), and the like, as would be known to the artisan of ordinary skill.

Additionally, the anti-α₄ immunoglobulin of the present invention can be combined with other compounds or compositions used to treat, ameliorate or palliate symptoms associated with inflammatory bowel disease such as Crohn's disease, asthma, multiple sclerosis (MS), rheumatoid arthritis (RA), graft versus host disease (GVHD), host versus graft disease, and various spondyloarthropathies. Furthermore, the compound disclosed herein can be administered alone or in combination with other agents, such as immunosuppresants wherein the immunosuppresant is not a steroid 5-ASAs and anti-TNFs. When administered in combination, the anti-α₄ immunoglobulin of the present invention may be administered in the same formulation as these other compounds or compositions, or in a separate formulation, and administered prior to, following, or concurrently with the other compounds and compositions used to treat, ameliorate, or palliate symptoms.

5-aminosalicyclic acid (5-ASAs) are a class of anti-inflammatories commonly used to treat inflammatory bowel disease, such as Crohn's disease and ulcerative colitis. One common 5-ASA is mesalamine, including Pentasa® and Rowasa®. Other 5-ASAs, such as osalazine (Dipentum®) are converted to mesalamine in the body. Sulfasalazine (Azulfidine®) is also commonly administered. Side effects of 5-ASAs include melena, headache, vomiting and rash.

Immunosuppressants weaken or suppress the immune system, which in turn decreases inflammation. Examples include azathioprine, 6-mercaptopurine, methotrexate, and mycophenolate. These medications are used most often to prevent the body from rejecting a newly transplanted organ, or for inflammatory conditions that have not responded to other treatments. It often takes months for these drugs to improve symptoms, and the disease often returns when medication is discontinued. Side effects of immunosuppressants include nausea, vomiting, diarrhea, stomach ulcers, rash, malaise, liver inflammation, bone marrow suppression, fever, pancreatitis, and increased risk of certain types of cancer.

Anti-TNF agents are also indicated for the treatment of inflammatory conditions. Tumor necrosis factor (TNF) is a protein produced by the immune system that may be related to inflammation. Anti-TNF removes TNF from the bloodstream before it reaches the intestines, thereby preventing inflammation. Infliximab (Remicade®) is an anti-TNF agent indicated for the treatment of moderate to severe Crohn's disease that does not respond to standard therapies (mesalamine substances, corticosteroids, immunosuppressive agents) and for the treatment of open, draining fistulas.

### 4. Indications for Treatment

Inflammatory diseases that are included for treatment by the compositions, compound and methods disclosed herein include inflammatory bowel diseases, asthma, multiple sclerosis (MS), rheumatoid arthritis (RA), graft versus host disease (GVHD), host versus graft disease, and various spondyloarthropathies. Additional diseases or conditions contemplated for treatment include those traditionally treated with steroids.

### 5. Immunoglobulins

The agent of the invention is the anti-α₄ immunoglobulin nataliaumab or an immunologically active fragment thereof that when administered to a patient may be used in the diagnosis and treatment of inflammatory bowel disease, asthma, multiple sclerosis (MS), rheumatoid arthritis (RA), graft versus host disease (GVHD), host versus graft disease, and various spondyloarthropathies, such that a patient previously taking steroids may be tapered off the steroids and/or discontinued from them.

"Antibodies" generally includes complete immunoglobulins such as IgG1 (or any IgG subclass) or IgM, or inhibitors derived from antibodies, such as natalizumab.

"Antibody homolog" generally includes intact antibodies consisting of immunoglobulin light and heavy chains linked via disulfide bonds. The term "antibody homolog" generally encompasses a protein comprising one or more polypeptides selected from immunoglobulin light chains, immunoglobulin heavy chains and antigen-binding fragments thereof which are capable of binding to one or more antigens (*i.e.*, integrin or integrin ligand). The component polypeptides of an antibody homolog composed of more than one polypeptide may be disulfide-bound or otherwise covalently crosslinked. Accordingly, therefore, "antibody homologs" generally includes intact immunoglobulins of types IgA, IgG, IgE, IgD, IgM (as well as subtypes thereof, *e.g*., IgG1), wherein the light chains of the immunoglobulin may be of types kappa or lambda. "Antibody homologs" also generally includes portions of intact antibodies that retain antigen-binding specificity, for example Fab fragments, Fab' fragments, F(ab')₂ fragments, Fv fragments, scFv fragments, heavy and light chain monomers or dimers or mixtures thereof.

The agent of the invention is the monoclonal antibody nataliaumab or an immunologically active fragment thereof. In contrast topolyclonal antibody preparations, which typically include different antibodies directed against different epitopes, each monoclonal antibody is directed against a single epitope on the antigen. A second advantage of monoclonal antibodies is that they are synthesized by means that are uncontaminated by other immunoglobulins, *e.g*., by phage display or isolation from a hybridoma.

"Native antibodies and immunoglobulins" are generally heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains (Clothia et al., 1985, J. Mol. Biol., 186: 651-63; Novotny et al., 1985, Proc. Natl. Acad. Sci. USA, 82: 4592-6).

In addition, other antibodies can be identified using techniques available in the art. The monoclonal antibody of the present invention can be produced using phage display technology. Antibody fragments, which selectively bind to an α₄ integrin or a dimer comprising an α₄ integrin, are then isolated. Exemplary preferred methods for producing antibodies via phage display are disclosed in U.S. Patent Nos. 6,225,447; 6,180,336; 6,172,197; 6,140,471; 5,969,108; 5,885,793; 5,872,215; 5,871,907; 5,858,657; 5,837,242; 5,733,743 and 5,565,332.

A "variant" antibody generally refers to an immunoglobulin molecule that differs in amino acid sequence from a "parent" antibody amino acid sequence by virtue of addition, deletion and/or substitution of one or more amino acid residue(s) in the parent antibody sequence. The parent antibody or immunoglobulin can be a polyclonal antibody, monoclonal antibody, humanized antibody or any antibody fragment. A variant comprises one or more amino acid substitution(s) in one or more hypervariable region(s) of the parent antibody. For example, a variant may comprise at least one, *e.g*., from about one to about ten, or from about two to about five, substitutions in one or more hypervariable regions of the parent antibody. Ordinarily, the variant will have an amino acid sequence having at least 75% amino acid sequence identity with the parent antibody heavy or light chain variable domain sequences, e.g. at least 80%, or at least 85%, or at least 90%, or at least 95%. Identity or homology with respect to this sequence is defined as the percentage of amino acid residues in the candidate sequence that are identical with the parent antibody residues, after aligning the sequences and introducing gaps, if necessary, to achieve the maximum percent sequence identity. No N-terminal, C-terminal, or internal extensions, deletions, or insertions into the antibody sequence shall be construed as affecting sequence identity or homology. A variant retains the ability to bind the receptor and preferably has properties that are superior to those of the parent antibody. For example, a variant may have a stronger binding affinity, enhanced ability to activate the receptor, *etc.* To analyze such properties, one should compare a Fab form of the variant to a Fab form of the parent antibody or a full-length form of the variant to a full-length form of the parent antibody. A variant antibody generally displays at least about 10 fold, preferably at least about 20 fold, and most preferably at least about 50 fold, enhancement in biological activity when compared to the parent antibody. A "parent" antibody is one that is encoded by an amino acid sequence used for the preparation of the variant. For example, a parent antibody has a human framework region and has human antibody constant region(s). Generally, a parent antibody may be a humanized or human antibody. An "isolated" antibody is generally one that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. For example, an antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or non-reducing conditions using Coomassie blue or, preferably, silver stain. Isolated antibody includes the antibody *in situ* within recombinant cells since at least one component of the antibody's natural environment will not be present. Ordinarily, however, isolated antibodies will be prepared by at least one purification step.

### 5.1. Monoclonal Antibodies

Monoclonal antibodies can also be produced using the conventional hybridoma methods or genetically engineered. These methods have been widely applied to produce hybrid cell lines that secrete high levels of monoclonal antibodies against many specific antigens, and can also be used to produce monoclonal antibodies of the present invention. For example, mice (*e.g*., Balb/c mice) can be immunized with an antigenic α₄ epitope by intraperitoneal injection. After sufficient time has passed to allow for an immune response, the mice are sacrificed and the spleen cells obtained and fused with myeloma cells, using techniques well known in the art. The resulting fused cells, hybridomas, are then grown in a selective medium, and the surviving cells grown in such medium using limiting dilution conditions. After cloning and recloning, hybridomas can be isolated that secrete antibodies (for example, of the IgG or IgM class or IgG1 subclass) that selectively bind to the target, α₄ or a dimer comprising an α₄ integrin. To produce agents specific for human use, the isolated monoclonal can then be used to produce chimeric and humanized antibodies. Antibodies can also be prepared that are anti-peptide antibodies. Such anti-peptide antibodies would be prepared against peptides of α₄ integrin.

The term "chimeric" generally means that an agent is comprised of a linkage (chemical cross-linkage or covalent or other type) of two or more proteins having disparate structures and/or having disparate sources of origin. Thus, a chimeric α₄ integrin antagonist can include one moiety that is an α₄ integrin antagonist or fragment and another moiety that is not an α₄β₁ integrin antagonist.

A species of "chimeric" protein is a "fusion" or "fusion protein" generally refers to a colinear, covalent linkage of two or more proteins or fragments thereof via their individual peptide backbones, for example through genetic expression of a polynucleotide molecule encoding those proteins. Thus, fusion proteins are *e.g*. chimeric proteins that include an antibody or fragment thereof covalently linked to a second moiety that is not original to the antibody (*i.e.*, which derives from another immuoglobulin or polypeptide). For example, fusion proteins can include portions of intact antibodies that retain antigen-binding specificity, for example, Fab fragments, Fab' fragments, F(ab')₂ fragments, Fv fragments, scFv fragments, heavy chain monomers or dimers, light chain monomers or dimers, dimers consisting of one heavy and one light chain, and the like.

For example, fusion proteins are chimeric and comprise a moiety fused or otherwise linked to all or part of the hinge and constant regions of an immunoglobulin light chain, heavy chain, or both; *e.g*. a molecule which includes: (1) first moiety, (2) a second peptide, *e.g*., one which increases solubility or *in vivo* life time of the moiety, *e.g.*, a member of the immunoglobulin super family or fragment or portion thereof, *e.g.*, a portion or a fragment of IgG, *e.g.*, the human IgG1 heavy chain constant region, *e.g.*, CH₂, CH₃, and hinge regions. Specifically, a "steroid sparing/Ig fusion" is a protein comprising a biologically active steroid sparing moiety. A species of agents is an "integrin /Fc fusion", generally, which is a protein comprising a steroid sparing immunoglobulin linked to at least a part of the constant domain of an immunoglobulin. For example, a Fc fusion comprises an steroid sparing immunoglobulin linked to a fragment of an antibody containing the C terminal domain of the heavy immunoglobulin chains.

Generally, the term "fusion protein" also means a steroid sparing moiety that is chemically linked via a mono- or hetero-functional molecule to a second moiety that is not a steroid sparing moiety (resulting in a "chimeric" molecule) and is made *de novo* from purified protein as described below. Thus, one example of a chemically linked, as opposed to recombinantly linked, chimeric molecule that is a fusion protein may comprise: (1) an α₄ integrin subunit targeting moiety, *e.g*., a VCAM-1 moiety capable of binding to VLA-4) on the surface of VLA-4 bearing cells; (2) a second molecule which increases solubility or *in vivo* life time of the targeting moiety, *e.g.*, a polyalkylene glycol polymer such as polyethylene glycol (PEG). Usually, the α₄ targeting moiety can be any naturally occurring α₄ ligand or fragment thereof, *e.g*., a VCAM-1 peptide or a similar conservatively substituted amino acid sequence.

Chimeric and humanized antibodies can be produced from non-human antibodies, and can have the same or similar binding affinity as the antibody from which they are produced. Techniques developed for the production of chimeric antibodies (Morrison et al., 1984 Proc. Natl. Acad. Sci. 81: 6851; Neuberger et al., 1984 Nature 312: 604; Takeda et al., 1985 Nature 314: 452) by splicing the genes from a mouse antibody molecule of appropriate antigen specificity together with genes from, for example, a human antibody molecule of appropriate biological activity can be used. For example, a nucleic acid encoding a variable (V) region of a mouse monoclonal antibody can be joined to a nucleic acid encoding a human constant (C) region, *e.g*., IgG1 or IgG4. The resulting antibody is thus a species hybrid, generally with the antigen binding domain from the non-human antibody and the C or effector domain from a human antibody.

Humanized antibodies are antibodies with variable regions that are primarily from a human antibody (the acceptor antibody), but which have complementarity determining regions substantially from a non-human antibody (the donor antibody). *See, e.g.*, Queen et al., 1989 Proc. Natl Acad. Sci. USA 86: 10029-33; WO 90/07861; and U.S. Patent Nos. 6,054,297; 5,693,761; 5,585,089; 5,530,101 and 5,224,539. The constant region or regions of these antibodies are generally also from a human antibody. The human variable domains are typically chosen from human antibodies having sequences displaying a high homology with the desired non-human variable region binding domains. The heavy and light chain variable residues can be derived from the same antibody, or a different human antibody. In addition, the sequences can be chosen as a consensus of several human antibodies, such as described in WO 92/22653.

Specific amino acids within the human variable region are selected for substitution based on the predicted conformation and antigen binding properties. This can be determined using techniques such as computer modeling, prediction of the behavior and binding properties of amino acids at certain locations within the variable region, and observation of effects of substitution. For example, when an amino acid differs between a non-human variable region and a human variable region, the human variable region can be altered to reflect the amino acid composition of the non-human variable region. Several examples of humanizing anti-α4 antibodies are described herein.

By "humanized antibody homolog" is generally meant an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain. A "human antibody homolog" is an antibody homolog in which all the amino acids of an immunoglobulin light or heavy chain (regardless of whether or not they are required for antigen binding) are derived from a human source.

Humanized antibodies are disclosed in U.S. Patent No. 5,840,299.

Transgenic mice containing human antibody genes can be immunized with an antigenic α₄ structure and hybridoma technology can be used to generate human antibodies that selectively bind to α₄.

Chimeric, human and/or humanized antibodies can be produced by recombinant expression, *e.g*., expression in human hybridomas (Cole et al., Monoclonal Antibodies and Cancer Therapy, Alan R. Liss, p. 77 (1985)), in myeloma cells or in Chinese Hamster Ovary (CHO) cells. Alternatively, antibody-coding sequences can be incorporated into vectors suitable for introducing into the genome of animal thereby producing a transgenic animal. One example would be to produce such antibodies in the milk of a transgenic animal such as a bovine. *See e.g.*, U.S. Patent Nos. 5,849,992 and 5,304,489. Suitable transgenes include trangenes having a promoter and/or enhancer from a mammary gland specific gene, for example casein or β-lactoglobulin.

### 5.2 Humanized Antibodies

The present invention provides nataliaumab, a humanized immunoglobulin (or antibody specific for the α₄ subunit of VLA-4, which when administered in a steroid sparing effective amount may be used in the treatment and diagnosis of inflammatory bowel disease such as Crohn's disease, asthma, multiple sclerosis (MS), rheumatoid arthritis (RA), graft versus host disease (GVHD), host versus graft disease, and various spondyloarthropathies such that steroids are not necessary. More specifically, this invention relates to the use of natalizumab or an immunologically active fragment thereof for the preparation of a medicament to be administered to a subject in a steroid sparing effective amount to reduce and/or eliminate a need for steroid treatment in the subject with a disease selected from the group consisting of inflammatory bowel disease (IBD), asthma, multiple sclerosis (MS), rheumatoid arthritis (RA), graft versus host disease (GVHD), host versus graft disease, spondyloarthropathies, and combinations thereof, wherein the subject is under treatment with steroids. Humanized antibodies are antibodies of animal (typically mammalian) origin that have been modified using genetic engineering techniques. The techniques are used to replace constant region and/or variable region framework sequences with human sequences, while retaining the original antigen specificity of the antibody. Humanized antibodies are commonly derived from rodent (*e.g.*, mouse and hamster) antibodies with specificity for human antigens (*e.g.*, human VCAM-1 or human VLA-4). By reshaping the donor antibody (the antibody from the animal to which the antigen was administered) to have sequences from the animal to which the antibody will be administered for therapeutic purposes, there will be a reduced host response in the animal upon administration of the antibody. Only the Fc regions or all but the complementarity determining regions (CDRs) can be replaced with acceptor domains, wherein the acceptor is the animal to whom the reshaped antibody is to be administered (*e.g*., mammals such as humans, domesticated animals, agricultural animals and the like).

Natalizumab, anantibody that bind to the α₄ subunit of VLA-4 which when administered to a patient in a steroid sparing effective amount treat inflammatory bowel disease, asthma, multiple sclerosis (MS), rheumatoid arthritis (RA), graft versus host disease (GVHD), host versus graft disease, and various spondyloarthropathies is the object of the present invention.

Typically, CDRs of a murine antibody are transplanted onto the corresponding regions in a human antibody, since it is the CDRs (*i.e.*, three in antibody heavy chains, three in light chains) that are the regions of the mouse antibody (or any other animal antibody), which bind to a specific antigen. Transplantation of CDRs is achieved by genetic engineering, whereby CDR DNA sequences are determined by cloning of murine heavy and light chain variable (V) region gene segments, and are

then transferred to corresponding human V regions by site directed mutagenesis. In the final stage of the process, human constant region gene segments of the desired isotype (usually gamma I for CH and kappa for CL) are added and the humanized heavy and light chain genes are co-expressed in mammalian cells to produce soluble humanized antibody.

The transfer of these CDRs to a human antibody confers on this antibody the antigen binding properties of the original murine antibody. The six CDRs in the murine antibody are mounted structurally on a V region "framework" region. The reason that CDR-grafting is successful is that framework regions between mouse and human antibodies may have very similar 3-D structures with similar points of attachment for CDRS, such that CDRs can be interchanged. Such humanized antibody homologs may be prepared, as exemplified in, *e.g.*, Jones et al., 1986, Nature 321: 522-5; Riechmann et al., 1988, Nature 332: 323-7; Queen et al., 1989, Proc. Nat. Acad. Sci. USA 86: 10029; and Orlandi et al., 1989, Pro. Nat. Acad. Sci. USA 86: 3833.

Nonetheless, certain amino acids within framework regions are thought to interact with CDRs and to influence overall antigen binding affinity. The direct transfer of CDRs from a murine antibody to produce a recombinant humanized antibody without any modifications of the human V region frameworks often results in a partial or complete loss of binding affinity. In several cases, it appears to be critical to alter residues in the framework regions of the acceptor antibody (*e.g*., human antibody) in order to obtain binding activity.

Queen *et al.*, 1989 (*supra*) and WO 90/07861 (Protein Design Labs) have described the preparation of a humanized antibody that contains modified residues in the framework regions of the acceptor antibody by combining the CDRs of a murine MAb (anti-Tac) with human immunoglobulin framework and constant regions. One solution to solve the problem of the loss of binding affinity without any modifications of the human V region framework residues involves two key steps. First, the human V framework regions are chosen by computer analysts for optimal protein sequence homology to the V region framework of the original murine antibody. In the second step, the tertiary structure of the murine V region is modeled by computer in order to visualize framework amino acid residues that are likely to interact with the murine CDRs. These murine amino acid residues are then superimposed on the homologous human framework. For additional detail, *see* U.S. Patent Nos. 5,693,762; 5,693,761; 5,585,089; and 5,530,101 (Protein Design Labs).

Certain α₄ subunit-containing integrin antagonists generally include chimeric and humanized recombinant antibody homologs (*i.e.*, intact immunoglobulins and portions thereof) with B epitope specificity that have been prepared and are described in U.S. Patent No. 5,932,214 (MAb HP1/2). The starting material for the preparation of chimeric (mouse Variable-human Constant) and humanized anti-integrin antibody homologs may be a murine monoclonal anti-integrin antibody as previously described, a monoclonal anti-integrin antibody commercially available (*e.g*., HP2/1, Amae International, Inc., Westbrook, Me). Other humanized anti-VLA-4 antibody homologs are described by Athena Neurosciences, Inc. in PCT/US95/01219 (Jul. 27, 1995), U.S. Patent Nos. 5,840,299 and 6,033,665. Reference is also made to 5,932,214, 5,840,299 and 6,033,665 patents.

Humanized anti-VLA-4 antibodies generally comprise a humanized light chain and a humanized heavy chain. A humanized light chain may comprise three complementarity determining regions (CDRI, CDR2 and CDR3) having amino acid sequences from the corresponding complementarity determining regions of a mouse 21.6 immunoglobulin light chain, and a variable region framework from a human kappa light chain variable region framework sequence except in at least position the amino acid position is occupied by the same amino acid present in the equivalent position of the mouse 21.6 immunoglobulin light chain variable region framework. A humanized heavy chain may comprise three complementarity determining regions (CDR1, CDR2 and CDR3) having amino acid sequences from the corresponding complementarity determining regions of a mouse 21.6 immunoglobulin heavy chain, and a variable region framework from a human heavy chain variable region framework sequence except in at least one position the amino acid position is occupied by the same amino acid present in the equivalent position of the mouse 21.6 immunoglobulin heavy chain variable region framework. *See,* U.S. Patent Nos. 5,840,299 and 6,033,665

Fragments of an isolated α₄ integrin antagonist (*e.g*., fragments of antibody homologs) can also be produced efficiently by recombinant methods, by proteolytic digestion, or by chemical synthesis using methods known to those of skill in the art. In recombinant methods, internal or terminal fragments of a polypeptide can be generated by removing one or more nucleotides from one end (for a terminal fragment) or both ends (for an internal fragment) of a DNA sequence which encodes for the isolated hedgehog polypeptide. Expression of the mutagenized DNA produces polypeptide fragments. Digestion with certain endonucleases can also generate DNAs, which encode an array of fragments. DNAs that encode fragments of a protein can also be generated by random shearing, restriction digestion, or a combination thereof. Protein fragments can be generated directly from intact proteins. Peptides can be cleaved specifically by proteolytic enzymes, including, but not limited to plasmin, thrombin, trypsin, chymotrypsin, or pepsin. Each of these enzymes is specific for the type of peptide bond it attacks. Trypsin catalyzes the hydrolysis of peptide bonds in which the carbonyl group is from a basic amino acid, usually arginine or lysine. Pepsin and chymotrypsin catalyze the hydrolysis of peptide bonds from aromatic amino acids, such as tryptophan, tyrosine, and phenylalanine. Alternative sets of cleaved protein fragments are generated by preventing cleavage at a site which is susceptible to a proteolytic enzyme. For instance, reaction of the ε-amino acid group of lysine with ethyltrifluorothioacetate in mildly basic solution yields blocked amino acid residues whose adjacent peptide bond is no longer susceptible to hydrolysis by trypsin. Proteins can be modified to create peptide linkages that are susceptible to proteolytic enzymes. For instance, alkylation of cysteine residues with β-haloethylamines yields peptide linkages that are hydrolyzed by trypsin (Lindley, 1956, Nature 178: 647). In addition, chemical reagents that cleave peptide chains at specific residues can be used. For example, cyanogen bromide cleaves peptides at methionine residues (Gross et al., 1961, J. Am. Chem. Soc. 83: 1510). Thus, by treating proteins with various combinations of modifiers, proteolytic enzymes and/or chemical reagents, the proteins may be divided into fragments of a desired length with no overlap of the fragments, or divided into overlapping fragments of a desired length.

### 5.3 Natalizumab and Related Humanized Antibodies

The invention provides for a humanized immunoglobulin that specifically bind to a VLA-4 ligand either alone or in combination for use in diagnosing and/or treating inflammatory bowel disease such as Crohn's disease, asthma, multiple sclerosis (MS), rheumatoid arthritis (RA), graft versus host disease (GVHD), host versus graft disease, and various spondyloarthropathies.

More specifically, this invention relates to the use of natalizumab or an immunologically active fragment thereof for the preparation of a medicament to be administered to a subject in a steroid sparing effective amount to reduce and/or eliminate a need for steroid treatment in the subject with a disease selected from the group consisting of inflammatory bowel disease (IBD), asthma, multiple sclerosis (MS), rheumatoid arthritis (RA), graft versus host disease (GVHD), host versus graft disease, spondyloarthropathies, and combinations thereof, wherein the subject is under treatment with steroids.

In general, humanized antibodies comprise a humanized light chain and a humanized heavy chain. In one aspect, the humanized light chain can comprise three complementarity determining regions (*i.e.*, CDR1, CDR2 and CDR3) having amino acid sequences from the corresponding complementarity determining regions of a mouse 21-6 immunoglobulin light chain, and a variable region framework from a human kappa light chain variable region framework sequence except in at least one position selected from a first group consisting of positions L45, L49, L58 and L69, wherein the amino acid position is occupied by the same amino acid present in the equivalent position of the mouse 21.6 immunoglobulin light chain variable region framework.

Moreover, the humanized heavy chain can comprise three complementarity determining regions (*i.e*., CDR1, CDR2 and CDR3) having amino acid sequences from the corresponding complementarity determining regions of a mouse 21-6 immunoglobulin heavy chain, and a variable region framework from a human heavy chain variable region framework sequence except in at least one position selected from a group consisting of H27, H28, H29, H30, H44, H71, wherein the amino acid position is occupied by the same amino acid present in the equivalent position of the mouse 21-6 immunoglobulin heavy chain variable region framework. Such immunoglobulins specifically bind to VLA-4 with an affinity having a lower limit of about 10⁷ M⁻¹ and an upper limit of about five times the affinity of the mouse 21-6 immunoglobulin.

Usually, the humanized light and heavy chain variable region frameworks are from RE1 and 21/28'CL variable region framework sequences respectively. When the humanized light chain variable region framework is from RE1, at least two framework amino acids are replaced. One amino acid is from the first group of positions described *supra.* The other amino acids are from a third group consisting of positions L104, L105 and L107. This position is occupied by the same amino acid present in the equivalent position of a kappa light chain from a human immunoglobulin other than RE1.

Some humanized immunoglobulins have a mature light chain variable region sequence designated La or Lb, or a mature heavy chain variable region sequence designated Ha, Hb or Hc. Preferred humanized immunoglobulins include those having a La light chain and an Ha, Hb or Hc heavy chain.

Generally, humanized immunoglobulins have variable framework regions substantially from a human immunoglobulin (termed an acceptor immunoglobulin) and complementarity determining regions substantially from a mouse immunoglobulin termed mu MAb 21.6 (referred to as the donor immunoglobulin). The constant region(s), if present, are also substantially from a human immunoglobulin. The humanized antibodies exhibit a specific binding affinity for VLA-4 of at least 10⁷, 10⁸, 10⁹, or 10¹⁰ M⁻¹. Usually the upper limit of binding affinity of the humanized antibodies for VLA-4 is within a factor of three or five of that of mu MAb 21.6 (about 10⁹ M⁻¹). Often the lower limit of binding affinity is also within a factor of three or five of that of mu MAb 21.6.

Usually, humanized antibodies can be produced as exemplified, for example, with the mouse MAb 21.6 monoclonal antibody. The starting material for production of humanized antibodies is mu MAb 21.6. The isolation and properties of this antibody are described in U.S. Patent No. 6,033,655 (assigned to Elan Pharmaceuticals, Inc.). Briefly, mu MAb 21.6 is specific for the α₄ subunit of VLA-4 and has been shown to inhibit human lymphocyte binding to tissue cultures of rat brain cells stimulated with tumor necrosis factor. From N-terminal to C-terminal, both light and heavy chains comprise the domains FR1, CDR1, FR2, CDR2, FR3, CDR3 and FR4. The assignment of amino acids to each domain is in accordance with the numbering convention of Kabat.

The next step involves selecting human antibodies to supply framework residues. The substitution of mouse CDRs into a human variable domain framework is most likely to result in retention of their correct spatial orientation if the human variable domain framework adopts the same or similar conformation to the mouse variable framework from which the CDRs originated. This can be achieved by obtaining the human variable domains from human antibodies whose framework sequences exhibit a high degree of sequence identity with the murine variable framework domains from which the CDRs were derived. The heavy and light chain variable framework regions can be derived from the same or different human antibody sequences. The human antibody sequences can be the sequences of naturally occurring human antibodies or can be consensus sequences of several human antibodies. *See* Kettleborough et al., Protein Engineering 4: 773 (1991); Kolbinger et al., Protein Engineering 6: 971 (1993).

Generally, suitable human antibody sequences are identified by computer comparisons of the amino acid sequences of the mouse variable regions with the sequences of known human antibodies. The comparison can be performed separately for heavy and light chains but the principles are similar for each. This comparison reveals that the mu 21.6 light chain shows greatest sequence identity to human light chains of subtype kappa 1; the mu 21.6 heavy chain shows greatest sequence identity to human heavy chains of subtype one, as defined by Kabat, *supra.* Thus, light and heavy human framework regions are usually derived from human antibodies of these subtypes, or from consensus sequences of such subtypes. Generally, light and heavy chain human variable regions showing greatest sequence identity to the corresponding regions from mu MAb 21.6 are from antibodies RE1 and 21/28'CL respectively.

Usually, computer modeling can then be used to further enhance the humanized antibody's ability to bind to its cognate antigen. The unnatural juxtaposition of murine CDR regions with human variable framework region can result in unnatural conformational restraints, which, unless corrected by substitution of certain amino acid residues, lead to loss of binding affinity. The selection of amino acid residues for substitution is determined, in part, by computer modeling. Computer hardware and software for producing three-dimensional images of immunoglobulin molecules are widely available. In general, molecular models are produced starting from solved structures for immunoglobulin chains or domains thereof. The chains to be modeled are compared for amino acid sequence similarity with chains or domains of solved three dimensional structures, and the chains or domains showing the greatest sequence similarity is/are selected as starting points for construction of the molecular model. For example, for the light chain of mu MAb 21.6, the starting point for modeling the framework regions, CDR1 and CDR2 regions, was the human light chain RB1. For the CDR3 region, the starting point was the CDR3 region from the light chain of a different human antibody HyHEL-5. The solved starting structures are modified to allow for differences between the actual amino acids in the immunoglobulin chains or domains being modeled, and those in the starting structure. The modified structures are then assembled into a composite immunoglobulin. Finally, the model is refined by energy minimization and by verifying that all atoms are within appropriate distances from one another and that bond lengths and angles are within chemically acceptable limits.

As noted *sura*, humanized antibodies can comprise variable framework regions substantially from a human immunoglobulin and complementarity determining regions substantially from a mouse immunoglobulin termed mu MAb 21.6. Having identified the complementarity determining regions (CDRs) of mu MAb 21.6 and appropriate human acceptor immunoglobulins, the next step is to determine which, if any, residues from these components should be substituted to optimize the properties of the resulting humanized antibody. In general, substitution of human amino acid residues with murine should be minimized, because introduction of murine residues increases the risk of the antibody eliciting a HAMA response in humans. Amino acids are selected for substitution based on their possible influence on CDR conformation and/or binding to antigen. Investigation of such possible influences is by modeling, examination of the characteristics of the amino acids at particular locations, or empirical observation of the effects of substitution or mutagenesis of particular amino acids.

When an amino acid differs between a mu MAb 21.6 variable framework region and an equivalent human variable framework region, the human framework amino acid should usually be substituted by the equivalent mouse amino acid if it is reasonably expected that the amino acid:
(1) non-covalently binds antigen directly (*e.g.*, amino acids at positions L49, L69 of mu MAb 21.6),
(2) is adjacent to a CDR region, is part of a CDR region under the alternative definition proposed by Chothia *et al.*, *supra*, or otherwise interacts with a CDR region (*e.g.*, is within about 3 A of a CDR region) (*e.g.*, amino acids at positions L45, L58, H27, H28, H29, H30 and H71 of mu MAb 21.6), or
(3) participates in the V_{L}-V_{H} interface (*e.g.*, amino acids at position H44 of mu MAb 21.6).

Generally, other candidates for substitution are acceptor human framework amino acids that are unusual for a human immunoglobulin at that position (*e.g*., amino acids at positions L104, L105 and L107 of mu MAb 21.6). These amino acids can be substituted with amino acids from the equivalent position of more typical human immunoglobulins. Alternatively, amino acids from equivalent positions in the mouse MAb 21.6 can be introduced into the human framework regions when such amino acids are typical of human immunoglobulin at the equivalent positions.

In general, substitution of all or most of the amino acids fulfilling the above criteria is desirable. Occasionally, however, there is some ambiguity about whether a particular amino acid meets the above criteria, and alternative variant immunoglobulins are produced, one of which has that particular substitution, the other of which does not. Humanized antibodies will usually contain a substitution of a human light chain framework residue with a corresponding mu MAb 21.6 residue in at least 1, 2 or 3, and more usually 4, of the following positions: L45, L49, L58 and L69. Humanized antibodies also usually contain a substitution of a human heavy chain framework residue in at least 1, 2, 3, 4, or 5, and sometimes 6, of the following positions: H27, H28, H29, H30 H44 and H71. Optionally, H36 may also be substituted. In general, when the human light chain acceptor immunoglobulin is RE1, the light chain also contains substitutions in at least 1 or 2, and more usually 3, of the following positions: L104, L105 and L107. These positions are substituted with the amino acid from the equivalent position of a human immunoglobulin having a more typical amino acid residues.

Usually the CDR regions in humanized antibodies are substantially identical; and more usually, identical to the corresponding CDR regions in the mu MAb 21.6 antibody. Occasionally, however, one of the residues in a CDR region can be changed. Amino acid similarity exists between the mu MAb 21.6 CDR3 and the VCAM-1 ligand. This observation suggests that the binding affinity of humanized antibodies might be improved by redesigning the heavy chain CDR3 region to resemble VCAM-1 even more closely. Accordingly, one or more amino acids from the CDR3 domain can usually be substituted with amino acids from the VCAM-1 binding domain. Although not usually desirable, it is sometimes possible to make one or more conservative amino acid substitutions of CDR residues without appreciably affecting the binding affinity of the resulting humanized immunoglobulin.

The framework regions of humanized immunoglobulins can be substantially identical, or, identical to the framework regions of the human antibodies from which they were derived. Of course, many of the amino acids in the framework region make little or no direct contribution to the specificity or affinity of an antibody. Thus, many individual conservative substitutions of framework residues can usually be tolerated without appreciable change of the specificity or affinity of the resulting humanized immunoglobulin. However, in general, such substitutions are undesirable.

### 5.3.1 Production of Variable Regions

Having conceptually selected the CDR and framework components of humanized immunoglobulins, a variety of methods are available for producing humanized immunoglobulins. Because of the degeneracy of the code, a variety of nucleic acid sequences will encode each immunoglobulin amino acid sequence. Nucleic acid sequences can be produced by *de novo* solid-phase DNA synthesis or by PCR mutagenesis of an earlier prepared variant of the desired polynucleotide. Oligonucleotide-mediated mutagenesis is a preferred method for preparing substitution, deletion and insertion variants of target polypeptide *DNA. See* Adelman et al., DNA 2: 183 (1983). Briefly, the target polypeptide DNA is altered by hybridizing an oligonucleotide encoding the desired mutation to a single-stranded DNA template. After hybridization, a DNA polymerase is used to synthesize an entire second complementary strand of the template that incorporates the oligonucleotide primer, and encodes the selected alteration in the target polypeptide DNA.

### 5.3.2 Selection of Constant Region

The variable segments of humanized antibodies are typically linked to at least a portion of an immunoglobulin constant region (Fc), typically that of a human immunoglobulin. Human constant region DNA sequences can be isolated in accordance with well-known procedures from a variety of human cells, but preferably immortalized B-cells (*see* Kabat *et al., supra*, and WO 87/02671) (each of which is incorporated by reference in its entirety for all purposes). Ordinarily, the antibody will contain both light chain and heavy chain constant regions. The heavy chain constant region usually includes CH1, hinge, CH2, CH3, and CH4 regions.

Generally, humanized antibodies include antibodies having all types of constant regions, including IgM, IgG, IgD, IgA and IgE, and any isotype, including IgG1, IgG2, IgG3 and IgG4. When it is desired that the humanized antibody exhibit cytotoxic activity, the constant domain is usually a complement-fixing constant domain and the class is typically IgG₁. When such cytotoxic activity is not desirable, the constant domain may be of the IgG₂ class. Usually, the humanized antibody comprises sequences from more than one class or isotype.

### 5.3.3 Other Anti-VLA-4 Antibodies

Other known anti-VLA-4 antibodies are HP1/2, HP-2/1, HP2/4, L25, and P4C2.

Frequently, monoclonal antibodies created in mice are later humanized to avoid the human anti-mouse antibody (MAMA) immune response in a human subject injected with a mouse antibody. This occurs by CDR grafting or reshaping. Thus, typically the antibodies are first mouse monoclonal antibodies that through CDR grafting or reshaping become humanized, as discussed above for the 21.6 antibody. Generally, humanized antibodies that have specificity for VLA-4 are derived from sources (*e.g*., mouse typically) that at least one or more of the complementarity determining regions (CDRs) of the variable domains are derived from a donor non-human anti-VLA-4 antibody, and in which there may or may not have been minimal alteration of the acceptor antibody heavy and/or light variable framework region in order to retain donor antibody binding specificity. Usually, the antigen binding regions of the CDR-grafted heavy chain variable domain comprise the CDRs corresponding to positions 31-35 (CDR1), 50-65 (CDR2) and 95-102 (CDR3). The heavy chain can further includes non-human residues at framework positions 27-30 (Kabat numbering). The heavy chain can further include non-human residues at framework position 75 (Kabat numbering). The heavy chain can further include non-human residues at framework position(s) 77-79 or 66-67 and 69-71 or 84-85 or 38 and 40 or 24 (Kabat numbering). For example, the antigen binding regions of the CDR-grafted light chain variable domain comprise CDRs corresponding to positions 24-34 (CDR1), 50-56 (CDR2) and 89-97 (CDR3). Specifically, the light chain can further include non-human residues at framework positions 60 and 67 (Kabat numbering). These residue designations are numbered according to the Kabat numbering (Kabat et al., 5th ed. 4 vol. SEQUENCES OF PROTEINS OF IMMUNOLOGICAL INTEREST, U.S. Department of Health Human Services, NIH, USA (1991)).

Synthesis and Humanization of Mouse Antibody HP1/2. HP1/2 is another antibody that is directed against VLA-4. A method of preparing a humanized version of an antibody is described in U.S. Patent No. 6,602,503 assigned to Biogen, Inc. The sequences of the humanized antibodies are provided as follows. The HP1/2 V*_{H}* DNA sequence and its translated amino acid sequence are:

5'-gtc aaa ctg cag cag tct ggg gca gag ctt gtg aag cca ggg gcc tca 48
N-Val Lys Leu Gln Gln Ser Gly Ala Glu Leu Val Lys Pro Gly Ala Ser
1 5 10 15

gtc aag ttg ttc tgc aca gct tct ggc ttc aac att aaa gac acc tat 96
Val Lys Leu Phe Cys Thr Ala Ser Gly Phe Asn Ile Lys Asp Thr Tyr
20 25 30

atg cac tgg gtg aag cag agg cct caa cag ggc ctg gag tgg att gga 144
Met His Trp Val Lys Gln Arg Pro Gln Gln Gly Leu Glu Trp Ile Gly
35 40 45

agg att gat cct gcg agt ggc gat act aaa tat gac ccg aag ttc cag 192
Arg Ile Asp Pro Ala Ser Gly Asp Thr Lys Tyr Asp Pro Lys Phe Gln
50 55 60

gtc aag gcc act att aca gcg gac acg tcc tcc aac aca gcc tgg ctg 240
Val Lys Ala Thr Ile Thr Ala Asp Thr Ser Ser Asn Thr Ala Trp Leu
65 70 75 80

cag ctc agc agc ctg aca tct gag gac act gcc gtc tac tac tgt gca 288
Gln Leu Ser Ser Leu Thr Ser Glu Asp Thr Ala Val Tyr Tyr Cys Ala
85 90 95

gac gga atg tgg gta tca acg gga tat gct ctg gac ttc tgg ggc caa 336
Asp Gly Met Trp Val Ser Thr Gly Tyr Ala Leu Asp Phe Trp Gly Gln
100 105 110

ggg acc acg gtc acc gtc tcc tca-3' 360
Gly Thr Thr Val Thr Val Ser Ser-C
115 120

A comparison between HP 1/2 V*_{H}* the two sequences and a consensus sequence of family IIC revealed that the only unusual residues are at amino acid positions 80, and 121 (*i.e.*, 79, 94, and 121 in Kabat numbering). Although Tyr-80 is invariant in subgroup IIC other sequenced murine V_{H} regions have other aromatic amino acids at this position, although none have Trp. The majority of human and murine V_{H}S have an arginine residue at Kabat position 94. The presence of Asp-94 in HIP1/2 V*_{H}* is extremely rare; there is only one reported example of a negatively charged residue at this position. Proline at Kabat position 113 is also unusual but is unlikely to be important in the conformation of the CDRs because of its distance from them. The amino acids making up CDR1 have been found in three other sequenced murine V*_{H}* regions. However, CDR2 and CDR3 are unique to HP1/2 and are not found in any other reported murine V*_{H}*.

The HP1/2 V*_{K}* DNA sequence and its translated amino acid sequence are as follows:

5'-agt att gtg atg acc cag act ccc aaa ttc ctg ctt gtt tca gca gga 48
N-Ser Ile Val Met Thr Gln Thr Pro Lys Phe Leu Leu Val Ser Ala Gly
1 5 10 15

gac agg gtt acc ata acc tgc aag gcc agt cag agt gtg act aat gat 96
Asp Arg Val Thr Ile Thr Cys Lys Ala Ser Gln Ser Val Thr Asn Asp
20 25 30

gta gct tgg tac caa cag aag cca ggg cag tct cct aaa ctg ctg ata 144
Val Ala Trp Tyr Gln Gln Lys Pro Gly Gln Ser Pro Lys Leu Leu Ile
35 40 45

tat tat gca tcc aat cgc tac act gga gtc cct gat cgc ttc act ggc 192
Tyr Tyr Ala Ser Asn Arg Tyr Thr Gly Val Pro Asp Arg Phe Thr Gly
50 55 60

agt gga tat ggg acg gat ttc act ttc acc atc agc act gtg cag gct 240
Ser Gly Tyr Gly Thr Asp Phe Thr Phe Thr Ile Ser Thr Val Gln Ala
65 70 75 80

gaa gac ctg gca gtt tat ttc tgt cag cag gat tat agc tct ccg tac 288
Glu Asp Leu Ala Val Tyr Phe Cys Gln Gln Asp Tyr Ser Ser Pro Tyr
85 90 95

acg ttc gga ggg ggg acc aag ctg gag atc-3' 318
Thr Phe Gly Gly Gly Thr Lys Leu Glu Ile-C
100 105

Hop1/2 V*_{K}* is a member of Kabat family V (Kabat et al., 5th ed., 4 vol., SEQUENCES OF PROTEINS OF IMMUNOLOGICAL INTEREST, U.S. Department of Health Human Services (1991)) and has no unusual residues. The amino acids of CDR1 and CDR3 are unique. The amino acids making up CDR2 have been reported in one other murine V*_{K}*.

Design of a CDR-grafted Anti-VLA-4 Antibody. To design a CDR-grafted anti-VLA-4 antibody, it is generally necessary to determine which residues of murine HP1/2 comprise the CDRs of the light and heavy chains. Three regions of hypervariability amid the less variable framework sequences are found on both light and heavy chains (Wu and Kabat, J. Exp. Med. 132: 211-250 (1970); Kabat *et al.,* (1991)). In most cases these hypervariable regions correspond to, but may extend beyond, the CDR. CDRs of murine HP1/2 can be elucidated in accordance with Kabat *et al.,* (1991) by alignment with other V*_{H}* and V*_{K}* sequences. The CDRs of murine HP 1/2 V*_{H}* usually correspond to the residues identified in the humanized V*_{H}* sequences as follows:

| | |
|---|---|
| CDR1 | AA₃₁-AA₃₅ |
| CDR2 | AA₅₀-AA₆₆ |
| CDR3 | AA₉₉-AA₁₁₀ |

These correspond to AA₃₁-AA₃₅, AA₅₀-AA₆₅, and AA₉₅-AA₁₀₂, respectively, in Kabat numbering. The CDRs of murine HP 1/2 V*_{K}* were identified and correspond to the residues identified in the humanized V*_{K}* sequences as follows:

| | |
|---|---|
| CDR1 | AA₂₄-AA₃₄ |
| CDR2 | AA₅₀-AA₅₆ |
| CDR3 | AA₈₉-AA₉₇ |

These correspond to the same numbered amino acids in Kabat numbering. Thus, only the boundaries of the V*_{K}*, but not V*_{H}*, CDRs corresponded to the Kabat CDR residues. The human frameworks chosen to accept the HP1/2 (donor) CDRs were NEWM and RE1 for the heavy and light chains, respectively. The NEWM and the RE1 sequences have been published in Kabat *et al.,* (1991).

The DNA and corresponding amino acid sequence of the humanized heavy chain variable region of the humanized HP1/2 antibody is usually:

5'-atg gac tgg acc tgg agg gtc ttc tgc ttg ctg gct gta gca cca ggt 48
N-Met Asp Trp Thr Trp Arg Val Phe Cys Leu Leu Ala Val Ala Pro Gly
1 5 10 15

gcc cac tcc cag gtc caa ctg cag gag tcc ggt gct gaa gtt gtt aaa 96
Ala His Ser Gln Val Gln Leu Gln Glu Ser Gly Ala Glu Val Val Lys
20 25 30

ccg ggt tcc tcc gtt aaa ctg tcc tgc aaa gct tcc ggt ttc aac atc 144
Pro Gly Ser Ser Val Lys Leu Ser Cys Lys Ala Ser Gly Phe Asn Ile
35 40 45

aaa gac acc tac atg cac tgg gtt aaa cag cgt ccg ggt cag ggt ctg 192
Lys Asp Thr Tyr Met His Trp Val Lys Gln Arg Pro Gly Gln Gly Leu
50 55 60

gaa tgg atc ggt cgt atc gac ccg gct tcc ggt gac acc aaa tac gac 240
Glu Trp Ile Gly Arg Ile Asp Pro Ala Ser Gly Asp Thr Lys Tyr Asp
65 70 75 80

ccg aaa ttc cag gtt aaa gct acc atc acc gct gac gaa tcc acc tcc 288
Pro Lys Phe Gln Val Lys Ala Thr Ile Thr Ala Asp Glu Ser Thr Ser
85 90 95

acc gct tac ctg gaa ctg tcc tcc ctg cgt tcc gaa gac acc gct gtt 336
Thr Ala Tyr Leu Glu Leu Ser Ser Leu Arg Ser Glu Asp Thr Ala Val
100 105 110

tac tac tgc gct gac ggt atg tgg gtt tcc acc ggt tac gct ctg gac 384
Tyr Tyr Cys Ala Asp Gly Met Trp Val Ser Thr Gly Tyr Ala Leu Asp
115 120 125

ttc tgg ggt cag ggt acc acg gtc acc gtc tcc tca ggt gag tcc-3' 429
Phe Trp Gly Gln Gly Thr Thr Val Thr Val Ser Ser Gly Glu Ser-C
130 135 140

The DNA and corresponding amino acid sequence of the humanized light chain variable region of the humanized HP1/2 antibody:

5'-atg ggt tgg tcc tgc atc atc ctg ttc ctg gtt gct acc gct acc ggt 48
N-Met Gly Trp Ser Cys Ile Ile Leu Phe Leu Val Ala Thr Ala Thr Gly
1 5 10 15

gtt cac tcc atc gtt atg acc cag tcc ccg gac tcc ctg gct gtt tcc 96
Val His Ser Ile Val Met Thr Gln Ser Pro Asp Ser Leu Ala Val Ser
20 25 30

ctg ggt gaa cgt gtt acc atc aac tgc aaa gct tcc cag tcc gtt acc 144
Leu Gly Glu Arg Val Thr Ile Asn Cys Lys Ala Ser Gln Ser Val Thr
35 40 45

aac gac gtt gct tgg tac cag cag aaa ccg ggt cag tcc ccg aaa ctg 192
Asn Asp Val Ala Trp Tyr Gln Gln Lys Pro Gly Gln Ser Pro Lys Leu
50 55 60

ctg atc tac tac gct tcc aac cgt tac acc ggt gtt ccg gac cgt ttc 240
Leu Ile Tyr Tyr Ala Ser Asn Arg Tyr Thr Gly Val Pro Asp Arg Phe
65 70 75 80

tcc ggt tcc ggt tac ggt acc gac ttc acc ttc acc atc tcc tcc gtt 288
Ser Gly Ser Gly Tyr Gly Thr Asp Phe Thr Phe Thr Ile Ser Ser Val
85 90 95

cag gct gaa gac gtt gct gtt tac tac tgc cag cag gac tac tcc tcc 336
Gln Ala Glu Asp Val Ala Val Tyr Tyr Cys Gln Gln Asp Tyr Ser Ser
100 105 110
ccg tac acc ttc ggt ggt ggt acc aaa ctg gag atc taa ggatcctc-3' 383
Pro Tyr Thr Phe Gly Gly Gly Thr Lys Leu Glu Ile-C
115 120

Usually, in addition to the above humanized HP1/2 antibody light and heavy chains, other acceptor heavy and light chains regions can also be utilized for insertion of the donor HP1/2 regions. All the following constructs contain Ser-75 (Kabat numbering). The STAW construct further contains Gln to Thr at position 77, Phe to Ala at position 78, and Ser to Trp at position 79 (Kabat numbering). The V*_{H}* DNA sequence and its translated amino acid sequence are set forth below:

5'-atg gac tgg acc tgg agg gtc ttc tgc ttg ctg gct gta gca cca ggt 48
N-Met Asp Trp Thr Trp Arg Val Phe Cys Leu Leu Ala Val Ala Pro Gly
1 5 10 15

gcc cac tcc cag gtc caa ctg cag gag agc ggt cca ggt ctt gtg aga 96
Ala His Ser Gln Val Gln Leu Gln Glu Ser Gly Pro Gly Leu Val Arg
20 25 30

cct agc cag acc ctg agc ctg acc tgc acc gtg tct ggc ttc aac att 144
Pro Ser Gln Thr Leu Ser Leu Thr Cys Thr Val Ser Gly Phe Asn Ile
35 40 45

aaa gac acc tat atg cac tgg gtg aga cag cca cct gga cga ggt ctt 192
Lys Asp Thr Tyr Met His Trp Val Arg Gln Pro Pro Gly Arg Gly Leu
50 55 60

gag tgg att gga agg att gat cct gcg agt ggc gat act aaa tat gac 240

Glu Trp Ile Gly Arg Ile Asp Pro Ala Ser Gly Asp Thr Lys Tyr Asp
65                  70                  75                  80

ccg aag ttc cag gtc aga gtg aca atg ctg gta gac acc agc agc aac     288
Pro Lys Phe Gln Val Arg Val Thr Met Leu Val Asp Thr Ser Ser Asn
                85                  90                  95

aca gcc tgg ctg aga ctc agc agc gtg aca gcc gcc gac acc gcg gtc     336
Thr Ala Trp Leu Arg Leu Ser Ser Val Thr Ala Ala Asp Thr Ala Val
            100                 105                 110

tat tat tgt gca gac gga atg tgg gta tca acg gga tat gct ctg gac     384
Tyr Tyr Cys Ala Asp Gly Met Trp Val Ser Thr Gly Tyr Ala Leu Asp
        115                 120                 125

ttc tgg ggc caa ggg acc acg gtc acc gtc tcc tca ggt gag tcc-3'     429
Phe Trp Gly Gln Gly Thr Thr Val Thr Val Ser Ser Gly Glu Ser-C
    130                 135                 140

The KAITAS construct contains the additional changes of Arg to Lys (position 66), Val to Ala (position 67), Met to Ile (position 69), Leu to Thr (position 70) and Val to Ala (position 71) (Kabat numbering. The KAITAS V*_{H}* DNA sequence and its translated amino acid sequence are set forth below:

5'-atg gac tgg acc tgg agg gtc ttc tgc ttg ctg gct gta gca cca ggt 48
N-Met Asp Trp Thr Trp Arg Val Phe Cys Leu Leu Ala Val Ala Pro Gly
1 5 10 15

gcc cac tcc cag gtc caa ctg cag gag agc ggt cca ggt ctt gtg aga 96
Ala His Ser Gln Val Gln Leu Gln Glu Ser Gly Pro Gly Leu Val Arg
20 25 30

cct agc cag acc ctg agc ctg acc tgc acc gtg tct ggc ttc aac att 144
Pro Ser Gln Thr Leu Ser Leu Thr Cys Thr Val Ser Gly Phe Asn Ile
35 40 45

aaa gac acc tat atg cac tgg gtg aga cag cca cct gga cga ggt ctt 192
Lys Asp Thr Tyr Met His Trp Val Arg Gln Pro Pro Gly Arg Gly Leu
50 55 60

gag tgg att gga agg att gat cct gcg agt ggc gat act aaa tat gac 240
Glu Trp Ile Gly Arg Ile Asp Pro Ala Ser Gly Asp Thr Lys Tyr Asp
65 70 75 80

ccg aag ttc cag gtc aaa gcg aca att acg gca gac acc agc agc aac 288
Pro Lys Phe Gln Val Lys Ala Thr Ile Thr Ala Asp Thr Ser Ser Asn
85 90 95

cag ttc agc ctg aga ctc agc agc gtg aca gcc gcc gac acc gcg gtc 336
Gln Phe Ser Leu Arg Leu Ser Ser Val Thr Ala Ala Asp Thr Ala Val
100 105 110

tat tat tgt gca gac gga atg tgg gta tca acg gga tat gct ctg gac 384
Tyr Tyr Cys Ala Asp Gly Met Trp Val Ser Thr Gly Tyr Ala Leu Asp
115 120 125

ttc tgg ggc caa ggg acc acg gtc acc gtc tcc tca ggt gag tcc-3' 429
Phe Trp Gly Gln Gly Thr Thr Val Thr Val Ser Ser Gly Glu Ser-C
130 135 140

The SSE construct comprises the additional changes of Ala to Ser (position 84) and Ala to Glu (position 85) (Kabat numbering). The SSE V*_{H}* DNA sequence and its translated amino acid sequence are set forth below:

5'-cag gtc caa ctg cag gag agc ggt cca ggt ctt gtg aga cct agc cag 48
N-Gln Val Gln Leu Gln Glu Ser Gly Pro Gly Leu Val Arg Pro Ser Gln
1 5 10 15

acc ctg agc ctg acc tgc acc gtg tct ggc ttc aac att aaa gac acc 96
Thr Leu Ser Leu Thr Cys Thr Val Ser Gly Phe Asn Ile Lys Asp Thr
20 25 30

tat atg cac tgg gtg aga cag cca cct gga cga ggt ctt gag tgg att 144
Tyr Met His Trp Val Arg Gln Pro Pro Gly Arg Gly Leu Glu Trp Ile
35 40 45

gga agg att gat cct gcg agt ggc gat act aaa tat gac ccg aag ttc 192
Gly Arg Ile Asp Pro Ala Ser Gly Asp Thr Lys Tyr Asp Pro Lys Phe
50 55 60

cag gtc aga gtg aca atg ctg gta gac acc agc agc aac cag ttc agc 240
Gln Val Arg Val Thr Met Leu Val Asp Thr Ser Ser Asn Gln Phe Ser
65 70 75 80

ctg aga ctc agc agc gtg aca tct gag gac acc gcg gtc tat tat tgt 288
Leu Arg Leu Ser Ser Val Thr Ser Glu Asp Thr Ala Val Tyr Tyr Cys
85 90 95

gca gac gga atg tgg gta tca acg gga tat gct ctg gac ttc tgg ggc 336
Ala Asp Gly Met Trp Val Ser Thr Gly Tyr Ala Leu Asp Phe Trp Gly
100 105 110

caa ggg acc acg gtc acc gtc tcc tca ggt gag tcc-3' 372
Gln Gly Thr Thr Val Thr Val Ser Ser Gly Glu Ser-C
115 120

The KRS construct comprises the additional changes of Arg to Lys (position 38) and Pro to Arg (position 40) (Kabat numbering). The KRS V*_{H}* DNA sequence and its translated amino acid sequence are set forth below:

5'-atg gac tgg acc tgg agg gtc ttc tgc ttg ctg gct gta gca cca ggt 48
N-Met Asp Trp Thr Trp Arg Val Phe Cys Leu Leu Ala Val Ala Pro Gly
1 5 10 15

gcc cac tcc cag gtc caa ctg cag gag agc ggt cca ggt ctt gtg aga 96
Ala His Ser Gln Val Gln Leu Gln Glu Ser Gly Pro Gly Leu Val Arg
20 25 30

cct agc cag acc ctg agc ctg acc tgc acc gtg tct ggc ttc aac att 144
Pro Ser Gln Thr Leu Ser Leu Thr Cys Thr Val Ser Gly Phe Asn Ile
35 40 45

aaa gac acc tat atg cac tgg gtg aaa cag cga cct gga cga ggt ctt 192
Lys Asp Thr Tyr Met His Trp Val Lys Gln Arg Pro Gly Arg Gly Leu
50 55 60

gag tgg att gga agg att gat cct gcg agt ggc gat act aaa tat gac 240
Glu Trp Ile Gly Arg Ile Asp Pro Ala Ser Gly Asp Thr Lys Tyr Asp
65 70 75 80

ccg aag ttc cag gtc aga gtg aca atg ctg gta gac acc agc agc aac 288
Pro Lys Phe Gln Val Arg Val Thr Met Leu Val Asp Thr Ser Ser Asn
85 90 95

cag ttc agc ctg aga ctc agc agc gtg aca gcc gcc gac acc gcg gtc 336
Gln Phe Ser Leu Arg Leu Ser Ser Val Thr Ala Ala Asp Thr Ala Val
100 105 110

tat tat tgt gca gac gga atg tgg gta tca acg gga tat gct ctg gac 384
Tyr Tyr Cys Ala Asp Gly Met Trp Val Ser Thr Gly Tyr Ala Leu Asp
115 120 125

ttc tgg ggc caa ggg acc acg gtc acc gtc tcc tca ggt gag tcc-3' 429
Phe Trp Gly Gln Gly Thr Thr Val Thr Val Ser Ser Gly Glu Ser-C
130 135 140

The AS construct comprises the change Val to Ala at position 24 (Kabat numbering). The AS V*_{H}* DNA sequence and its translated amino acid sequence are:

5'-atg gac tgg acc tgg agg gtc ttc tgc ttg ctg gct gta gca cca ggt 48
N-Met Asp Trp Thr Trp Arg Val Phe Cys Leu Leu Ala Val Ala Pro Gly
1 5 10 15

gcc cac tcc cag gtc caa ctg cag gag agc ggt cca ggt ctt gtg aga 96
Ala His Ser Gln Val Gln Leu Gln Glu Ser Gly Pro Gly Leu Val Arg
20 25 30

cct agc cag acc ctg agc ctg acc tgc acc gcg tct ggc ttc aac att 144
Pro Ser Gln Thr Leu Ser Leu Thr Cys Thr Ala Ser Gly Phe Asn Ile
35 40 45

aaa gac acc tat atg cac tgg gtg aga cag cca cct gga cga ggt ctt 192
Lys Asp Thr Tyr Met His Trp Val Arg Gln Pro Pro Gly Arg Gly Leu
50 55 60

gag tgg att gga agg att gat cct gcg agt ggc gat act aaa tat gac 240
Glu Trp Ile Gly Arg Ile Asp Pro Ala Ser Gly Asp Thr Lys Tyr Asp
65 70 75 80

ccg aag ttc cag gtc aga gtg aca atg ctg gta gac acc agc agc aac 288
Pro Lys Phe Gln Val Arg Val Thr Met Leu Val Asp Thr Ser Ser Asn
85 90 95

cag ttc agc ctg aga ctc agc agc gtg aca gcc gcc gac acc gcg gtc 336
Gln Phe Ser Leu Arg Leu Ser Ser Val Thr Ala Ala Asp Thr Ala Val
100 105 110

tat tat tgt gca gac gga atg tgg gta tca acg gga tat gct ctg gac 384
Tyr Tyr Cys Ala Asp Gly Met Trp Val Ser Thr Gly Tyr Ala Leu Asp
115 120 125

ttc tgg ggc caa ggg acc acg gtc acc gtc tcc tca ggt gag tcc-3' 429
Phe Trp Gly Gln Gly Thr Thr Val Thr Val Ser Ser Gly Glu Ser-C
130 135 140

The humanized light chain generally requires few, if any, modifications. However, in the preparation of humanized anti-VLA-4 antibodies, several empirical changes improve the immunological activity of the antibody towards its ligand. For example, the humanized heavy chain with the Ser mutation with the murine light chain is about 2.5 fold lower potency than murine HP1/2. The same humanized heavy chain with a humanized light chain is about 4-fold lower potency.

In general, a humanized V*_{K}* construct (VK1) comprises a Ser to Asp substitution at position 60, and a Ser for a Tyr at position 67. The DNA sequence and its translated amino acid sequence are set forth below:

5'-atg ggt tgg tcc tgc atc atc ctg ttc ctg gtt gct acc gct acc ggt 48
N-Met Gly Trp Ser Cys Ile Ile Leu Phe Leu Val Ala Thr Ala Thr Gly
1 5 10 15

gtt cac tcc gac atc cag ctg acc cag agc cca agc agc ctg agc gcc 96
Val His Ser Asp Ile Gln Leu Thr Gln Ser Pro Ser Ser Leu Ser Ala
20 25 30

agc gtg ggt gac aga gtg acc atc acc tgt aag gcc agt cag agt gtg 144
Ser Val Gly Asp Arg Val Thr Ile Thr Cys Lys Ala Ser Gln Ser Val
35 40 45

act aat gat gta gct tgg tac cag cag aag cca ggt aag gct cca aag 192
Thr Asn Asp Val Ala Trp Tyr Gln Gln Lys Pro Gly Lys Ala Pro Lys
50 55 60

ctg ctg atc tac tat gca tcc aat cgc tac act ggt gtg cca agc aga 240
Leu Leu Ile Tyr Tyr Ala Ser Asn Arg Tyr Thr Gly Val Pro Ser Arg
65 70 75 80

ttc agc ggt agc ggt agc ggt acc gac ttc acc ttc acc atc agc agc 288
Phe Ser Gly Ser Gly Ser Gly Thr Asp Phe Thr Phe Thr Ile Ser Ser
85 90 95

ctc cag cca gag gac atc gcc acc tac tac tgc cag cag gat tat agc 336
Leu Gln Pro Glu Asp Ile Ala Thr Tyr Tyr Cys Gln Gln Asp Tyr Ser
100 105 110

tct ccg tac acg ttc ggc caa ggg acc aag gtg gaa atc aaa cgt aag tg-3'
386
Ser Pro Tyr Thr Phe Gly Gln Gly Thr Lys Val Glu Ile Lys Arg Lys-C
115 120 125

Another V*_{K}* construct (*i*.*e.,* VK2) has the DQMDY sequences of the original RE1 framework restored. The DNA and corresponding amino acid sequence are provided below:

5'-atg ggt tgg tcc tgc atc atc ctg ttc ctg gtt gct acc gct acc ggt 48
N-Met Gly Trp Ser Cys Ile Ile Leu Phe Leu Val Ala Thr Ala Thr Gly
1 5 10 15

gtc cac tcc agc atc gtg atg acc cag agc cca agc agc ctg agc gcc 96
Val His Ser Ser Ile Val Met Thr Gln Ser Pro Ser Ser Leu Ser Ala
20 25 30

agc gtg ggt gac aga gtg acc atc acc tgt aag gcc agt cag agt gtg 144
Ser Val Gly Asp Arg Val Thr Ile Thr Cys Lys Ala Ser Gln Ser Val
35 40 45

act aat gat gta gct tgg tac cag cag aag cca ggt aag gct cca aag 192
Thr Asn Asp Val Ala Trp Tyr Gln Gln Lys Pro Gly Lys Ala Pro Lys

50 55 60

ctg ctg atc tac tat gca tcc aat cgc tac act ggt gtg cca gat aga 240
Leu Leu Ile Tyr Tyr Ala Ser Asn Arg Tyr Thr Gly Val Pro Asp Arg
65 70 75 80

ttc agc ggt agc ggt tat ggt acc gac ttc acc ttc acc atc agc agc 288
Phe Ser Gly Ser Gly Tyr Gly Thr Asp Phe Thr Phe Thr Ile Ser Ser
85 90 95

ctc cag cca gag gac atc gcc acc tac tac tgc cag cag gat tat agc 336
Leu Gln Pro Glu Asp Ile Ala Thr Tyr Tyr Cys Gln Gln Asp Tyr Ser
100 105 110

tct ccg tac acg ttc ggc caa ggg acc aag gtg gaa atc aaa cgt aag tg-3'
386
Ser Pro Tyr Thr Phe Gly Gln Gly Thr Lys Val Glu Ile Lys Arg Lys-C
115 120 125

A third V*_{K}* construct is VK3 has SVM versus DQM in the amino terminus and two other residue changes. The DNA and corresponding amino acid sequence are:

5'-atg ggt tgg tcc tgc atc atc ctg ttc ctg gtt gct acc gct acc ggt 48
N-Met Gly Trp Ser Cys Ile Ile Leu Phe Leu Val Ala Thr Ala Thr Gly
1 5 10 15

gtc cac tcc gac atc cag atg acc cag agc cca agc agc ctg agc gcc 96
Val His Ser Asp Ile Gln Met Thr Gln Ser Pro Ser Ser Leu Ser Ala
20 25 30

agc gtg ggt gac aga gtg acc atc acc tgt aag gcc agt cag agt gtg 144
Ser Val Gly Asp Arg Val Thr Ile Thr Cys Lys Ala Ser Gln Ser Val
35 40 45

act aat gat gta gct tgg tac cag cag aag cca ggt aag gct cca aag 192
Thr Asn Asp Val Ala Trp Tyr Gln Gln Lys Pro Gly Lys Ala Pro Lys
50 55 60

ctg ctg atc tac tat gca tcc aat cgc tac act ggt gtg cca gat aga 240
Leu Leu Ile Tyr Tyr Ala Ser Asn Arg Tyr Thr Gly Val Pro Asp Arg
65 70 75 80

ttc agc ggt agc ggt tat ggt acc gac ttc acc ttc acc atc agc agc 288
Phe Ser Gly Ser Gly Tyr Gly Thr Asp Phe Thr Phe Thr Ile Ser Ser
85 90 95

ctc cag cca gag gac atc gcc acc tac tac tgc cag cag gat tat agc 336
Leu Gln Pro Glu Asp Ile Ala Thr Tyr Tyr Cys Gln Gln Asp Tyr Ser
100 105 110

tct ccg tac acg ttc ggc caa ggg acc aag gtg gaa atc aaa cgt aag tg-3'
386
Ser Pro Tyr Thr Phe Gly Gln Gly Thr Lys Val Glu Ile Lys Arg Lys-C
115 120 125

Details regarding the preparation of each of these light and heavy chain sequences are provided in U.S. Patent No. 6,602,503. In general, various combinations of the above light and heavy chains can be prepared based on computer modeling as known in the art.

Additional antibodies that recognize and bind to α₄ integrin are known in the art. For example, GG5/3 (Keszthelyi et al., Neurology 47(4): 1053-1059 (1996)), FW3-218-1 (ATCC No.: HB-261; an IgG2b antibody against sheep α₄ integrin), and R1-2 (ATCC No.: HB-227; IgG2b antibody developed in *Rattus norvegicus*). Whether the antibodies are developed in mouse or other animals, each of the sequences can be genetically engineered such that they are humanized based on what is known in the art and with the aid of computer modeling. The anti-a4 integrin humanized antibodies can then be assessed for their ability to diagnose and/or treat inflammatory bowel conditions on the *in vitro* and *in vivo* assays disclosed herein.

### 5.4 Antibody Fragments

Also contemplated for use in diagnosing and/or treating inflammatory bowel conditions are antibody fragments of antibodies that bind to anti-α4 or VCAM-1 such that they inhibit VLA-4 and VCAM-1 interaction. More specifically, this invention relates to the use of natalizumab or an immunologically active fragment thereof for the preparation of a medicament to be administered to a subject in a steroid sparing effective amount to reduce and/or eliminate a need for steroid treatment in the subject with a disease selected from the group consisting of inflammatory bowel disease (IBD), asthma, multiple sclerosis (MS), rheumatoid arthritis (RA), graft versus host disease (GVHD), host versus graft disease, spondyloarthropathies, and combinations thereof, wherein the subject is under treatment with steroids. Antibody fragments include Fab, F(ab')₂, scFv and Fv fragments which can be used in the compositions disclosed herein.

The term "Fab fragment" as used herein refers to a partial antibody molecule containing a single antigen-binding region, which consists of a portion of both the heavy and light chains of the molecule.

The term "F(ab')₂ fragment" as used herein refers to a partial antibody molecule containing both antigen binding regions, and which consists of the light chains and a portion of the heavy chains of the molecule.

The term "Fv fragment" as used herein refers to the portion of the antibody molecule involved in antigen recognition and binding.

The term "scFv" as used herein refers to single chain Fv (scFv) fragments. These scFv fragments are recombinant antibody derivatives that consist only of the variable domains of antibody heavy and light chains connected by a flexible linker. scFv antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the scFv to form the desired structure for antigen binding. For a review of scFv *see* Pluckthun in The Pharmacology of Monoclonal Antibodies, vol. 113, 269-315 (Rosenburg and Moore eds., Springer-Verlag, New York 1994).

Also included in antibody fragments are diabodies. The term "diabodies" refers to small antibody fragments with two antigen-binding sites, which fragments comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., 1993 Proc. Natl. Acad. Sci. USA 90: 6444-8.

Antibody fragments also include linear antibodies. The expression "linear antibodies" when used throughout this application refers to the antibodies described in, *e.g.*, Zapata et al., 1995 Protein Eng. 8(10): 1057-62. Briefly, these antibodies comprise a pair of tandem Fd segments (V_{H} -C_{H}1-V_{H}-C_{H}1), which form a pair of antigen binding regions. Linear antibodies can be bispecific or monospecific.

Papain digestion of antibodies produces two identical antigen binding fragments, called "Fab" fragments, each with a single antigen binding site, and a residual "Fc" fragment, whose name reflects its ability to crystallize readily. Pepsin treatment yields an F(ab')₂ fragment that has two antigen combining sites and is still capable of cross-linking antigen.

Several mouse anti-VLA-4 monoclonal antibodies have been previously described. *See, e.g.*, U.S. Patent Nos. 6,602,503; 6,033,665; and 5,840,299; Sanchez-Madrid et al., 1986, Eur. J. Immunol. 16: 1343-9; Hemler et al., 1987, J. Biol. Chem. 262: 11478-85; Pulido et al., 1991, J. Biol. Chem., 266: 10241-45; Issekutz et al., 1991, J. Immunol., 147: 109 (TA-2 MAb)). For example, anti - VLA-4 antibodies that recognize the VLA-4 α₄ chain epitopes involved in binding to VCAM-1 and fibronectin ligands (*i.e*., antibodies which can bind to VLA-4 at a site involved in ligand recognition and block VCAM-1 and fibronectin binding). Such antibodies have been defined as B epitope-specific antibodies (B1 or B2) (Pulido *et al.*, 1991, *supra*).

Moreover, there are fully human monoclonal antibody homologs against VLA-4 that can block or coat VLA-4 ligands. In their intact form these may be prepared using *in vitro*-primed human splenocytes, as described by Boerner et al., 1991, J. Immunol., 147: 86-95. Alternatively, they may be prepared by repertoire cloning as described by Persson et al., 1991, Proc. Nat. Acad. Sci. USA, 88: 2432-36 or by Huang et al., 1991, J. Immunol. Meth., 141: 227-236. U.S. Patent No. 5,798,230 (Aug. 25, 1998, "Process for the preparation of human monoclonal antibodies and their use") describes preparation of human monoclonal antibodies from human B cells. According to this process, human antibody-producing B cells are immortalized by infection with an Epstein-Barr virus, or a derivative thereof, that expresses Epstein-Barr virus nuclear antigen 2 (EBNA2). EBNA2 function, which is required for immortalization, is subsequently shut off, which results in an increase in antibody production. Additional methods are known in the art.

For yet another method for producing fully human antibodies, *see, e.g.,* U.S. Patent No. 5,789,650, which describes transgenic non-human animals capable of producing heterologous antibodies and transgenic non-human animals having inactivated endogenous immunoglobulin genes. Endogenous immunoglobulin genes are suppressed by antisense polynucleotides and/or by antiserum directed against endogenous immunoglobulins. Heterologous antibodies are encoded by immunoglobulin genes not normally found in the genome of that species of non-human animal. One or more transgenes containing sequences of unrearranged heterologous human immunoglobulin heavy chains are introduced into a non-human animal thereby forming a transgenic animal capable of functionally rearranging transgenic immunoglobulin sequences and producing a repertoire of antibodies of various isotypes encoded by human immunoglobulin genes. Such heterologous human antibodies are produced in B-cells, which are thereafter immortalized, *e.g*., by fusing with an immortalizing cell line such as a myeloma or by manipulating such B-cells by other techniques to perpetuate a cell line capable of producing a monoclonal, heterologous, fully human antibody homolog. In general, large non-immunized human phage display libraries can also be used to isolate high affinity antibodies that can be developed as human therapeutics using standard phage technology.

Following the early methods for the preparation of true "chimeric antibodies" (*i.e.*, where the entire constant and entire variable regions are derived from different sources), a new approach was described in EP 0239400 (Winter et al.) whereby antibodies are altered by substitution (within a given variable region) of their complementarity determining regions (CDRs) for one species with those from another. Usually, this process can be used, for example, to substitute the CDRs from human heavy and light chain Ig variable region domains with alternative CDRs from murine variable region domains. These altered Ig variable regions can subsequently be combined with human Ig constant regions to created antibodies, which are totally human in composition except for the substituted murine CDRs. Such CDR-substituted antibodies would be predicted to be less likely to elicit an immune response in humans compared to true chimeric antibodies, because the CDR-substituted antibodies contain considerably less non-human components. The process for humanizing monoclonal antibodies via CDR "grafting" has been termed "reshaping" (Riechmann et al., 1988, Nature 332: 323-7; and Verhoeyen et al., 1988, Science 239: 1534-6).

### 5.5 Antibody Purification

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10: 163-7 (1992) describe a procedure for isolating antibodies, which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. In instances when the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells is preferably subjected to at least one purification step prior to LPHIC. Examples of suitable purification steps include hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., 1983 J. Immunol. Meth. 62: 1-13). Protein G is recommended for all mouse isotypes and for human γ3 (Guss et al., 1986 EMBO J. 5: 1567-75). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H}3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™, chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminant(s) is subjected to LPHIC. Often, the antibody composition to be purified will be present in a buffer from the previous purification step. However, it may be necessary to add a buffer to the antibody composition prior to the LPHIC step. Many buffers are available and can be selected by routine experimentation. The pH of the mixture comprising the antibody to be purified and at least one contaminant in a loading buffer is adjusted to a pH of about 2.5-4.5 using either an acid or base, depending on the starting pH. Preferably, the loading buffer has a low salt concentration *(i.e.,* less than about 0.25 M salt).

The mixture is loaded on the HIC column. HIC columns normally comprise a base matrix (*e.g.*, cross-linked agarose or synthetic copolymer material) to which hydrophobic ligands (*e.g.*, alkyl or aryl groups) are coupled. A preferred HIC column comprises an agarose resin substituted with phenyl groups (*e.g.*, a Phenyl SEPHAROSE™ column). Many HIC columns are available commercially. Examples include, but are not limited to, Phenyl SEPHAROSE 6 FAST FLOW™ column with low or high substitution (Pharmacia LKB Biotechnology, AB, Sweden); Phenyl SEPHAROSE™ High Performance column (Pharmacia LKB Biotechnology, AB, Sweden); Octyl SEPHAROSE™ High Performance column (Pharmacia LKB Biotechnology, AB, Sweden); FRACTOGEL™ EMD Propyl or FRACTOGEL™ EMD Phenyl columns (E. Merck, Germany); MACRO-PREP™ Methyl or MACRO-PREP™ t-Butyl Supports (Bio-Rad, California); WP HI-Propyl (C₃)™ column (J. T. Baker, New Jersey); and TOYOPEARL™ ether, phenyl or butyl columns (TosoHaas, PA).

The antibody is eluted from the column using an elution buffer, which is normally the same as the loading buffer. The elution buffer can be selected using routine experimentation. The pH of the elution buffer is between about 2.5-4.5 and has a low salt concentration (*i.e.*, less than about 0.25 M salt). It has been discovered that it is not necessary to use a salt gradient to elute the antibody of interest; the desired product is recovered in the flow through fraction, which does not bind significantly to the column.

The LPHIC step provides a way to remove a correctly folded and disulfide bonded antibody from unwanted contaminants (*e.g.,* incorrectly associated light and heavy fragments). In particular, the method provides a means to substantially remove an impurity characterized herein as a correctly folded antibody fragment whose light and heavy chains fail to associate through disulfide bonding.

Diagnostic or therapeutic formulations of the purified protein can be made by providing the antibody composition in the form of a physiologically acceptable carrier, examples of which are provided below.

To remove contaminants (*e.g.,* unfolded antibody and incorrectly associated light and heavy fragments) from the HIC column so that it can be re-used, a composition including urea (*e.g.,* 6.0 M urea, 1% MES buffer pH 6.0, 4 mM ammonium sulfate) can be flowed through the column. Other methods are known in the art.

### 5.6 Immunoglobulin Formulations

The antibody and immunoglobulin having the desired therapeutic effect may be administered in a physiologically acceptable carrier to a subject. The antibody may be administered in a variety of ways including but not limited to parenteral administration, including subcutaneous, subdural, intravenous, intramuscular, intrathecal, intraperitoneal, intracerebral, intraarterial, or intralesional routes of administration, localized (*e.g.,* surgical application or surgical suppository), and pulmonary (*e.g.*, aerosols, inhalation, or powder) and as described further below.

Depending upon the manner of introduction, the immunoglobulin may be formulated in a variety of ways. The concentration of therapeutically active immunoglobulin in the formulation (*i.e.*, a formulation sufficient to diagnose and/or treat inflammatory bowel conditions) may vary from 1 mg/ml to 1 g/ml. Preferably, the immunoglobulin composition, when administered to a subject in need thereof, reaches a blood level of immunoglobulin in the subject of 10 ng/ml or more.

Preferably, the immunoglobulin is formulated for parenteral administration in a suitable inert carrier, such as a sterile physiological saline solution. For example, the concentration of immunoglobulin in the carrier solution is typically between 1-100 mg/ml. The dose administered will be determined by route of administration. Preferred routes of administration include parenteral or intravenous administration.

For parenteral administration, the antibody of the invention can be administered as injectable dosages of a solution or suspension of the substance in a physiologically acceptable diluent with a pharmaceutical carrier which can be a sterile liquid such as water and oils with or without the addition of a surfactant and other pharmaceutically preparations are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. The antibody of this invention can be administered in the form of a depot injection or implant preparation, which can be formulated in such a manner as to permit a sustained release of the active ingredient. A preferred composition comprises the monoclonal antibody at 5 mg/mL, formulated in aqueous buffer consisting of 50 mM L-histidine, 150 mM NaCl, adjusted to pH 6.0 with HCl.

According to an important feature of the invention, the immunoglobulin that recognizes and binds to VLA-4 may be administered alone, or in combination with an another agent which is typically used to treat inflammatory bowel disease such as Crohn's disease, asthma, multiple sclerosis (MS), rheumatoid arthritis (RA), graft versus host disease (GVHD), host versus graft disease, and various spondyloarthropathies. Administration of these agents can occur prior to, concurrent with or after administration with the immunoglobulin, wherein the other agent is not a steroid.

A therapeutically effective amount of the antibody or immunoglobulin, i.e., natalizumab, can be estimated by comparison with established effective doses for known antibodies, taken together with data obtained for natalizumab in both *in vivo* and *in vitro* models. Preferably the data is from studies of treatment of inflammatory bowel disease such as Crohn's disease, asthma, multiple sclerosis (MS), rheumatoid arthritis (RA), graft versus host disease (GVHD), host versus graft disease, and spondyloarthropathies, as appropriate. As is known in the art, adjustments in the dose may be necessary due to immunoglobulin degeneration or metabolism, systemic versus localized delivery, as well as the age, body weight, general health, sex, diet, time of administration, drug interactions and the severity of the condition of the subject to whom the immunoglobulin is administered. Such adjustments may be made and appropriate doses determined by one of skill in the art through routine experimentation.

Therapeutic formulations of the immunoglobulin are prepared for storage by mixing the immunoglobulin having the desired degree of purity with optional physiologically acceptable carriers, excipients, or stabilizers (Remington's Pharmaceutical Sciences, 16th ed., A. Osol, Ed., 1980 and more recent editions), in the form of lyophilized cake or aqueous solutions. Acceptable immunoglobulin carriers, excipients or stabilizers are nontoxic, non-therapeutic and/or non-immunogenic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG). Specific examples of carrier molecules include but are not limited to glycosaminoglycans (e.g., heparin sulfate), hyaluronic acid, keratan-sulfate, chondroitin 4-sulfate, chondroitin 6-sulfate, heparan sulfate and dermatin sulfate, perlecan and pentopolysulfate.

Pharmaceutical compositions comprising the immunoglobulin can also include if desired, pharmaceutically acceptable, non-toxic carriers or diluents, which are vehicles commonly used to formulate pharmaceutical compositions for animal or human administration. The diluent is selected so as not to affect the biological activity of the combination. Examples include but are not limited to distilled water, physiological phosphate-buffered saline, Ringer's solutions, dextrose solution, and Hank's solution.

The agent of the invention can be formulated into preparations for injections by dissolving, suspending or emulsifying them in an aqueous or nonaqueous solvent, such as vegetable or other similar oils, synthetic aliphatic acid glycerides, esters of higher aliphatic acids or propylene glycol. The formulation may also contain conventional additives, such as solubilizers, isotonic agents, suspending agents, emulsifying agents, stabilizers and preservatives.

The immunoglobulin may also be utilized in aerosol formulation to be administered via inhalation or pulmonary delivery. The agent of the present invention can be formulated into pressurized acceptable propellants such as dichlorodifluoromethane, propane, nitrogen and the like.

The immunoglobulin also may be entrapped in microcapsules prepared, for example, by coacervation techniques or by interfacial polymerization (*e.g.,* hydroxymethylcellulose or gelatin-microcapsules and poly-methylmethacylate microcapsules), in colloidal drug delivery systems (*e.g.,* liposomes, albumin microspheres, microemulsions, nano-particles and nanocapsules), or in macroemulsions. Such techniques are disclosed in *Remington's Pharmaceutical Sciences, supra.*

The immunoglobulin to be used for *in vivo* administration must be sterile. This is readily accomplished by filtration through sterile filtration membranes, prior to or following lyophilization and reconstitution. The immunoglobulin ordinarily will be stored in lyophilized form or in solution.

Therapeutic immunoglobulin compositions generally are placed into a container having a sterile access port, for example, an intravenous solution bag or vial having a stopper pierceable by a hypodermic injection needle or similar sharp instrument.

Suitable examples of sustained-release preparations include semipermeable matrices of solid hydrophobic polymers containing the protein, which matrices are in the form of shaped articles, *e.g*., films, or microcapsules. Examples of sustained-release matrices include polyesters, hydrogels (*e.g.*, poly(2-hydroxyethylmethacrylate) as described by Langer et al., J. Biomed. Mater. Res. 15: 167-277 (1981) and Langer, Chem. Tech. 12: 98-105 (1982) or poly(vinylalcohol)), polylactides (U.S. Patent No. 3,773,919), copolymers of L-glutamic acid and gamma ethyl-L-glutamate (Sidman et al., Biopolymers 22: 547-556, 1983), non-degradable ethylene-vinyl acetate (Langer *et al., supra*), degradable lactic acid-glycolic acid copolymers such as the LUPRON DEPOT™ (*i.e.,* injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C., resulting in a loss of biological activity and possible changes in immunogenicity. Rational strategies can be devised for immunoglobulin stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, developing specific polymer matrix compositions, and the like.

Sustained-release immunoglobulin compositions also include liposomally entrapped immunoglobulin. Liposomes containing the immunoglobulin are prepared by methods known *per se. See, e.g.,* Epstein et al., Proc. Natl. Acad. Sci. USA 82: 3688-92 (1985); Hwang et al., Proc. Natl. Acad. Sci. USA 77:4030-4 (1980); U.S. Patent Nos. 4,485,045; 4,544,545; 6,139,869; and 6,027,726. Ordinarily, the liposomes are of the small (200 to 800 Angstroms), unilamellar type in which the lipid content is greater than about 30 mole percent (mol. %) cholesterol; the selected proportion being adjusted for the optimal immunoglobulin therapy.

The immunoglobulin of this invention can be administered in a sustained release form, for example a depot injection, implant preparation, or osmotic pump, which can be formulated in such a manner as to permit a sustained release of the active ingredient. Implants for sustained release formulations are well-known in the art. Implants are formulated as microspheres, slabs, etc. with biodegradable or non-biodegradable polymers. For example, polymers of lactic acid and/or glycolic acid form an erodible polymer that is well-tolerated by the host. The implant is placed in proximity to the site of protein deposits (*e.g.,* the site of formation of amyloid deposits associated with neurodegenerative disorders), so that the local concentration of active agent is increased at that site relative to the rest of the body.

In addition, the immunoglobulin of the invention may be provided by administering a polynucleotide encoding a whole or partial antibody (*e.g.*, a single chain Fv) to a subject. The polynucleotide is administered to a subject in an appropriate vehicle to allow the expression of the immunoglobulin in the subject in a therapeutically effective amount.

A typical daily dosage might range for the immunoglobulin ranges from about 1 µg/kg to up to about 10 mg/kg or more, depending on the factors mentioned herein. Typically, the clinician will administer immunoglobulin until a dosage is reached that achieves the desired effect. The progress of this therapy is easily monitored by conventional assays.

A "stable" antibody or antibody fragment formulation is one in which the protein therein essentially retains its physical stability and/or chemical stability and/or biological activity upon storage. Various analytical techniques for measuring protein stability are available in the art and are reviewed in Peptide and Protein Drug Delivery, 247-301, (Vincent Lee Ed., Marcel Dekker, Inc., New York, N.Y., Pubs. 1991) and A. Jones, Adv. Drug Delivery Rev. 10: 29-90 (1993), for example. Stability can be measured at a selected temperature for a selected time period. Preferably, the formulation is stable at room temperature (about 30°C) or at 40°C for at least 1 month and/or stable at about 2-8°C for at least 1 year for at least about 2 years. Furthermore, the formulation is preferably stable following freezing (to, *e.g*., -70°C) and thawing of the formulation.

A protein "retains its physical stability" in a pharmaceutical formulation if it shows no signs of aggregation, precipitation and/or denaturation upon visual examination of color and/or clarity, or as measured by UV light scattering or by size exclusion chromatography.

A protein "retains its chemical stability" in a pharmaceutical formulation, if the chemical stability at a given time is such that the protein is considered to still retain its biological activity as defined below. Chemical stability can be assessed by detecting and quantifying chemically altered forms of the protein. Chemical alteration may involve size modification (*e.g.,* clipping) that can be evaluated using size exclusion chromatography, SDS-PAGE and/or matrix-assisted laser desorption ionization/time-of-flight mass spectrometry (MALDI/TOF MS), for example. Other types of chemical alteration include charge alteration (*e.g.*, occurring as a result of deamidation) that can be evaluated by, *e.g*., ion-exchange chromatography.

An immunoglobulin "retains its biological activity" in a pharmaceutical formulation, if the biological activity of the immunoglobulin at a given time is within about 10% (within the errors of the assay) of the biological activity exhibited at the time the pharmaceutical formulation was prepared as determined in an antigen-binding assay, for example.

### 5.7 Routes of Administration of Immunoglobulin Compositions

The pharmaceutical compositions discussed *supra* can be administered for diagnosis, prophylactic and/or therapeutic treatments of inflammatory bowel diseases, asthma, multiple sclerosis (MS), rheumatoid arthritis (RA), graft versus host disease (GVHD), host versus graft disease, and various spondyloarthropathies. In therapeutic applications, compositions are administered to a patient suspected of, or already suffering from a disease, in an amount sufficient to provide treatment. An amount adequate to accomplish this is defined as a therapeutically or pharmaceutically effective dose.

The pharmaceutical compositions will be administered by parenteral, topical, intravenous, oral, or subcutaneous, intramuscular local administration, such as by aerosol or transdermally, for prophylactic and/or therapeutic treatment. Although the proteinaceous substances of this invention may survive passage through the gut following oral administration (p.o.), subcutaneous (s.c.), intravenous (i.v.), intramuscular (i.m.), intraperitoneal administration by depot injection; or by implant preparation are preferred.

The pharmaceutical compositions can be administered in a variety of unit dosage forms depending upon the method of administration. For example, unit dosage forms suitable for oral administration include powder, tablets, pills, capsules, and lozenges.

Effective doses of the compositions of the present invention, for the treatment of the above described conditions will vary depending upon many different factors, including means of administration, target site, physiological state of the patient, and other medicaments administered. Thus, treatment dosages will need to be titrated to optimize safety and efficacy. These compositions may be administered to mammals for veterinary use and for clinical use in humans in a manner similar to other therapeutic agents, *i.e.*, in a physiologically acceptable carrier. In general, the administration dosage will range from 0.0001 to 100 mg/kg, and more usually 0.01 to 0.5 mg/kg of the host body weight.

In a preferred treatment regime, the antibody is administered by intravenous infusion or subcutaneous injection at a dose from 1 to 5 mg antibody per kilo of patient bodyweight. The dose is repeated at interval from 2 to 8 weeks. Within this range, the preferred treatment regimen is 3 mg antibody per kilo of bodyweight repeated at a 4-week interval.

### 6.0 Drug Combinations

Other drugs are currently used for the treatment of inflammatory bowel diseases, asthma, multiple sclerosis (MS), rheumatoid arthritis (RA), graft versus host disease (GVHD), host versus graft disease, and various spondyloarthropathies. These and other drugs are also contemplated for use in combination with the compound and compositions of the present invention. Selection of one or more agent to be utilized in a cocktail and/or combination with the compounds and compositions disclosed herein will be dependent on the management of the disease. For example, the compound and compositions disclosed herein can be administered with immunosuppressant agents to further treat Crohn's disease and to suppress symptoms, wherein the immunosupressant is not a steroid. More specifically, the present invention relates to the use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of inflammatory bowel disease (IBD), asthma, multiple sclerosis (MS), rheumatoid arthritis (RA), graft versus host disease (GVHD), host versus graft disease, spondyloarthropathies, and combinations thereof. Dosage forms of the agents to be used in combination with the compounds and compositions disclosed herein would vary depending on the subject and drug combination being utilized.

### 7.0 Chronic Administration Dosage Regimens

The chronic treatment regimen of the present invention provides natalizumab or an immunologically active fragment thereof at a level that will maintain sufficient receptor saturation to suppress pathological inflammation in a patient in need of such. The methods of the invention entails administration once per every two weeks or once a month to once every two months, with repeated dosings taking place over a period

of at least six months, and more preferably for a year or longer. The methods of the invention involve obtaining and maintaining a receptor saturation level in a human patient of a dimer comprising α₄ integrin (*e.g.*, VLA-4) in a range of from 65% to 100%, more preferably between 75%, to 100%, and even more preferably between 80-100%. These receptor saturation levels are maintained at these levels chronically (e.g., over a period of 6 months or so) to allow for continued suppression of pathological inflammation.

In a specific embodiment, the dosing of the agent of the invention is on a monthly basis. Levels of receptor saturation can be monitored to determine the efficacy of the dosing regime, and physiological markers measured to confirm the success of the dosage regime. As a confirmation, serum levels of the antibody can be monitored to identify clearance of the antibody and to determine the potential effect of half-life on the efficacy of the treatment.

For treatment with the agent of the invention, the dosage ranges from 0.0001 to 100 mg/kg, and more usually 0.01 to 5 mg/kg, of the host body weight. For example dosages can be 1 mg/kg body weight or 10 mg/kg body weight. Dosage and frequency vary depending on the half-life of the agent in the patient. The dosage and frequency of administration can vary depending on whether the treatment is prophylactic or therapeutic. For immunoglobulin administration, each dosing injection is generally between 2.0 to 8.0 mg/kg dosage. For a compound administration, each dosing injection is generally between 1.0 to 50.0 mg/kg dosage. In accordance with the teachings provided herein, effective dosages can be monitored by obtaining a fluid sample from a patient. For this, generally a blood serum or cerebrospinal fluid sample is taken and integrin receptor saturation is determined using methods well known in the art. Ideally, a sample is taken prior to initial dosing; subsequent samples are taken and measured prior to and/or after each treatment.

As an alternative to chronic administration comprised of repeated individual dosings, the agent for the treatment of inflammatory bowel diseases, asthma, multiple sclerosis (MS), rheumatoid arthritis (RA), graft versus host disease (GVHD), host versus graft disease, and various spondyloarthropathies can be administered as a sustained release formulation, provided the dosage is such that the levels of receptor saturation remain sufficient to suppress inflammation. For example, controlled release systems can be used to chronically administer the agent within the scope of this invention. Discussions of appropriate controlled release dosage forms may be found in Lesczek Krowczynski, EXTENDED-RELEASE DOSAGE FORMS, 1987 (CRC Press, Inc.).

The various controlled release technologies cover a very broad spectrum of drug dosage forms. Controlled release technologies include, but are not limited to physical systems and chemical systems. Physical systems include, but not limited to, reservoir systems with rate-controlling membranes, such as microencapsulation, macroencapsulation, and membrane systems; reservoir systems without rate-controlling membranes, such as hollow fibers, ultra microporous cellulose triacetate, and porous polymeric substrates and foams; monolithic systems, including those systems physically dissolved in non-porous, polymeric, or elastomeric matrices (e.g., non-erodible, erodible, environmental agent ingression, and degradable), and materials physically dispersed in non-porous, polymeric, or elastomeric matrices (*e.g.*, non-erodible, erodible, environmental agent ingression, and degradable); laminated structures, including reservoir layers chemically similar or dissimilar to outer control layers; and other physical methods, such as osmotic pumps, or adsorption onto ion-exchange resins.

Chemical systems include, but are not limited to, chemical erosion of polymer matrices (*e.g.,* heterogeneous, or homogeneous erosion), or biological erosion of a polymer matrix (*e.g.,* heterogeneous, or homogeneous). Additional discussion of categories of systems for controlled release may be found in Agis F. Kydonieus, CONTROLLED RELEASE TECHNOLOGIES: METHODS, THEORY AND APPLICATIONS, 1980 (CRC Press, Inc.).

The methods of the invention can be used to treat a patient that is affected with a disorder involving or arising from a pathological inflammation of the present invention or to prophylactically treat a patient at risk for a particular disorder. The dosage regimens necessary for prophylactic versus therapeutic treatment can vary, and will need to be designed for the specific use and disorder treated.

In some methods, two or more agents (*e.g*., the monoclonal antibody and a compound as described above) are administered concurrently, in which case the dosage of each agent administered falls within the ranges indicated. Combination therapies can also occur where the agents are administered consecutively to the patient with a desired time interval been periods of administration. Intervals can also be irregular as indicated by measuring receptor saturation levels or by following other indicia of the disease process.

Those of skill will readily appreciate that dose levels can vary as a function of the specific agent, the severity of the symptoms and the susceptibility of the subject to side effects. Some of the specific agents are more potent than others. Preferred dosages for a given agent are readily determinable by those of skill in the art by a variety of means. A preferred means is to measure the physiological potency of a given agent.

In prophylactic applications, pharmaceutical compositions are chronically administered to a patient susceptible to, or otherwise at risk of, a particular disease in an amount sufficient to eliminate or reduce the risk or delay the outset of the disease. Such an amount is defined to be a prophylactically effective dose. In patients with multiple sclerosis in remission, risk may be assessed by NMR imaging or, in some cases, by pre-symptomatic indications observed by the patient.

Effective dosage regimes of the compositions of the present invention, for the treatment of the above described conditions will vary depending upon many different factors, including means of administration, target site, physiological state of the patient, and other medicaments administered. Thus, treatment dosages will need to be titrated to optimize safety and efficacy. In general, each administration of the dosage regimen will range from 0.0001 to 100 mg/kg, usually 0.01 to 50, and more usually from 0.1 to 30 mg/kg of the host body weight.

### 8.0 Testing Reagents

In general, reagents can be tested *in vitro* and *in vivo*. Many *in vitro* models exist to test whether a reagent binds to the α₄ subunit, as would be known in the art. Testing whether a reagent has activity *in vivo* at diagnosis and/or treatment of inflammatory bowel conditions, as well as other inflammatory conditions, can be performed using the experimental autoimmune encephalomyelitis (EAE) animal model. EAE is an inflammatory condition of the central nervous system with similarities to multiple sclerosis (Paterson, IN TEXTBOOK OF IMMUNOPATHOLOGY, eds. Miescher and Mueller-Eberhard, 179-213, Grune and Stratton, N.Y. 1976).

Sections of EAE brain can be tested for their ability to support leukocyte attachment using, for example, an *in vitro* binding assay described in Stamper and Woodruff, J. Exp. Med. 144: 828-833 (1976). Reagents against leukocyte adhesion receptors can be examined for inhibitory activity in the *in vitro* section assay. The attachment of U937 cells (a human monocytic cell line) is almost completely blocked by antibodies against human VLA-4 integrin. The antibody of the present invention produced significantly greater blocking effect as compared to antibodies against other adhesion molecules.

In general, antibodies that selectively inhibit the fibronectin binding activity of α₄ integrin (P4G9 and HP1/7) enhanced U937 attachment to the EAE vessels. These results suggest that fibronectin-binding activity of α₄ integrin is not directly involved in U937 adhesion to EAE vessels *in vitro.* Given the *in vitro* results using the α₄β₁ reagents described above, the effect of these antibodies on the progression of EAE can also be tested *in vivo* by measuring the delay in the onset of paralysis or reduction in severity of the paralysis.

In general, additional reagents can be identified by use of adhesion assays. Using HP2/1 or *N*-[*N-*(3-pyridinesulfonyl)-L-3,3-dimethyl-4-thiaprolyl]-*O*-[1-methylpiperazin-4-ylcarbonyl]-L-tyrosine isopropyl ester as a control for example, other antibodies or reagents can be screened for their ability to inhibit the binding of lymphocytes to a known ligand for α₄β₁ integrin.

Monoclonal antibodies are for example HP2/1, TY21.6, TY21.12, and L25 as discussed in U.S. Patent No. 6,033,665. These antibodies react with the α chain of VLA-4 and block binding to VCAM-1, fibronectin and inflamed brain endothelial cells, but do not affect the activity of the other members of the β₁ integrin family.

These are other reagents which selectively react against the VLA-4/VCAM-1 These reagents further do not affect matrix interactions (mediated by all members of the β₁ integrins) nor affect normal intestinal immunity (mediated by α₄β₇). The production of this and other such reagents are well within the skill of the art.

In general, assays for determining whether agents exhibit α4β1 and/or α4β7 activity are known to those of skill in the art.

For example, in an assay, compounds can be bound to a solid support and the α4β7 integrin sample added thereto. The amount of α4β1 or α4β7 integrin in the sample can be determined by conventional methods such as use of a sandwich ELISA assay. In addition, certain compounds inhibit, *in vivo*, adhesion of leukocytes to endothelial cells and epithelial cells in mucosal organs mediated by α4β1 or α4β7 integrin and, accordingly, can be used in the treatment of diseases mediated by α4β1 or α4β7 integrin.

The biological activity of compounds can usually be assayed in a variety of systems. For example, a compound can be immobilized on a solid surface and adhesion of cells expressing α4β1 or α4β7 integrin can be measured. Using such formats, large numbers of compounds can be screened. Cells suitable for this assay include any leukocytes known to express α4β1 or α4β7 integrin such as memory T cells and eosinophils. A number of leukocyte cell lines can also be used, examples include the cell line RPMI-8866.

Compounds may also be tested for the ability to competitively inhibit binding between α4β1 or α4β7 integrin and MAdCAM-1, or between α4β1 or α4β7 integrin and a labeled compound known to bind α4β1 integrin such as the compound of this invention or antibodies to α4β7 integrin. In these assays, the MAdCAM-1 can be immobilized on a solid surface. MAdCAM-1 may also be expressed as a recombinant fusion protein having an Ig tail (e.g., IgG Fc) so that binding to α4β7 integrin may be detected in an immunoassay. Alternatively, MAdCAM-1 expressing cells, such as activated endothelial cells or MAdCAM-1 transfected fibroblasts can be used.

Both α4β7 and α4β1 can mediate adhesion to VCAM-1 and to fibronectin. For assays which measure the ability to block adhesion to VCAM-1 and to fibronectin, these are assays described in International Patent Application Publication No. WO US98/15324.

Many assay formats employ labeled assay components. The labeling systems can be in a variety of forms. The label may be coupled directly or indirectly to the desired component of the assay according to methods well known in the art. A wide variety of labels may be used. The component may be labeled by any one of several methods. The most common method of detection is the use of autoradiography with ³H, ¹²⁵I, ³⁵S, ¹⁴C, or ³²P labeled compounds or the like. Non-radioactive labels include ligands which bind to labeled antibodies, fluorophores, chemiluminescent agents, enzymes and antibodies which can serve as specific binding pair members for a labeled ligand. The choice of label depends on sensitivity required, ease of conjugation with the compound, stability requirements, and available instrumentation.

Appropriate *in vivo* models for demonstrating efficacy in treating inflammatory responses include EAE (experimental autoimmune encephalomyelitis) in mice, rats, guinea pigs or primates, as well as other inflammatory models dependent upon α4 integrins.

The compound of the invention may be modified as necessary to provide desired properties such as improved pharmacological properties (e.g., in vivo stability, bio-availability), or the ability to be detected in diagnostic applications. For instance, inclusion of one or more D-amino acids in the sulfonamides of this invention typically increases in vivo stability. Stability can be assayed in a variety of ways such as by measuring the half-life of the proteins during incubation with peptidases or human plasma or serum. A number of such protein stability assays have been described (*see, e.g.*, Verhoef et al., Eur. J. Drug Metab. Pharmacokinet., 1990, 15(2):83-93).

### Examples

The following examples are put forth so as to provide those of ordinary skill in the art with a complete disclosure and description of how to make and use the present invention. Efforts have been made to ensure accuracy with respect to numbers used (*e.g.*, amounts, temperature, etc.) but some experimental errors and deviations should be accounted for.

### Example 1

### Soluble MadCAM-1 FACS Assay

This assay measures the interaction of recombinant soluble MadCAM-1 with RPMI-8866 cells in suspension. Recombinant soluble MadCAM-1 ("rsMadCAM-1") is expressed as a fusion protein with a human IgG Fc tail (Tidswell et al., J. Immunol. (1997) 159(3):1497-1505). Soluble MadCAM-1 is mixed with RPMI-8866 cells in the presence and absence of small molecule inhibitors. 1 mM MnCl₂ is included in the assay buffer to increase the activity of α4β7 integrin and to promote its interaction with the MadCAM-1 construct. After 30 minutes at room temperature, the cells are washed with buffer containing 1 mM MnCl₂ and are exposed to fluorescent-labeled antibody against the Fc tail of the MadCAM-1 fusion protein in the presence of 1 mM MnCl₂ for 30 minutes at 4°C. The cells are washed, resuspended in MnCl₂ containing buffer and examined by FACS analysis. An identical assay can be performed to measure the interaction of recombinant soluble VCAM-1 with cells that express α₄β₁, such as the Jurkat T cell line.

### Example 2

### Cell Free ELISA Assay

This assay measures the interaction of solubilized α₄β₇ integrin with MadCAM-1 which has been immobilized on plastic. RPMI-8866 cells are lysed with a detergent to solubilize α₄β₇ integrin. Antibody against β₇ integrin, 2G3 (Tidswell et al. 1997 J. Immunol. 159(3): 1497-1505), is added to the lysate. This antibody serves two purposes, first, it is a tag by which α₄β₇ integrin can be detected in the assay and, second, 2G3 is an antibody that stabilizes a ligand occupied conformation of β₇ integrin and promotes β₇ integrin-dependent interactions. Cell lysate, 2G3, and test reagent are added to microtiter wells that have been coated with MadCAM-1. The mixture is allowed to incubate for 30 minutes at room temperature. The plate is washed, blocked with 1% BSA, and exposed to HRP-conjugated goat anti-mouse Ig, which recognizes 2G3 associated with α₄β₇ integrin that has bound MadCAM-1 on the assay well. After 30 minutes at room temperature, the wells are washed and exposed to a substrate for HRP to quantify the amount of α₄β₇ integrin that has bound MadCAM-1.

### Example 3

### FACS Assay for Receptor Occupancy

This assay measures the interaction of antibody 2G3 with RPMI-8866 cells or with lymphocytes. The antibody recognizes a ligand-occupied epitope of either rat or human β₇ integrin. Increasing concentrations of small molecule ligand induce the 2G3 epitope on β₇ integrin and will allow higher levels of antibody binding to the surface of the cells. The concentration of ligand required for receptor occupancy is directly related to the ligand's affinity for α₄β₇ integrin. A similar assay has been described for examining the interaction of ligands with α₄β₁ integrin, which utilizes an analogous antibody against a ligand occupied epitope of β₁ integrin (antibody 15/7; Yednock et al. (1995) J. Biol. Chem. 270: 28740-50). The β₁ integrin assay relies on cells that express α₄β₁ integrin, rather than α₄β₇ integrin (such as Jurkat cells). In both assays, the appropriate cells are mixed with either 2G3 or 15/7 in the presence of the small molecule ligand. The cells are incubated at room temperature for 30 minutes and washed to remove unbound antibody. The cells are exposed to a fluorescently-labeled antibody against mouse IgG, which detects cell-associated 2G3 or 15/7 and the cells are examined by FACS analysis.

### Example 4

### Ex Vivo Cell Adhesion Assay

This assay can be used to measure the adhesion of lymphocytes or RPMI-8866 cells to high endothelial venules exposed in tissue sections of Peyer's Patches (lymphoid tissue associated with the intestine). These vessels express high levels of MadCAM-1. This assay is described by Yednock et al., JCB (1987) 104: 725-731.

### Example 5

### In Vivo Migration Assay

Migration of ¹¹¹In-labeled or fluorescently-labeled lymphocytes to Peyer's Patches *in vivo.* In this assay, lymphocytes are isolated from one group of animals and are labeled with a radioactive or fluorescent tracer. The cells are injected intravenously into a second group of animals. After 1 to 24 hours, the localization of the labeled cells to different tissues can be monitored by either determining the number of radioactive counts associated with different tissues in a gamma counter, or by isolating lymphocytes from the tissue and determining the number of cells that carry a fluorescent tag (determined by FACS analysis). This type of assay is described by Rosen et al., 1989 J. Immunol. 142: 1895-1902.

### Example 6

### In vitro Assay For Determining Binding of Candidate Reagents to VLA-4

An *in vitro* assay was used to assess binding of candidate reagents to α₄β₁ integrin. Reagents which bind in this assay can be used to assess VCAM-1 levels in biological samples by conventional assays (*e.g*., competitive binding assays). This assay is sensitive to IC₅₀ values as low as about 1 nM.

The activity of α₄β₁ integrin was measured by the interaction of soluble VCAM-1 with Jurkat cells (*e.g*., American Type Culture Collection Nos. TIB 152, TIB 153, and CRL 8163), a human T-cell line which expresses high levels of α₄β₁ integrin. VCAM-1 interacts with the cell surface in an α₄β₁ integrin-dependent fashion (Yednock et al., J. Bio. Chem., 1995, 270:28740).

Recombinant soluble VCAM-1 was expressed as a chimeric fusion protein containing the seven extracellular domains of VCAM-1 on the N-terminus and the human IgG₁ heavy chain constant region on the C-terminus. The VCAM-1 fusion protein was made and purified by the manner described by Yednock, *supra.*

Jurkat cells were grown in RPMI 1640 supplemented with 10% fetal bovine serum, penicillin, streptomycin and glutamine as described by Yednock, *supra.*

Jurkat cells were incubated with 1.5 mM MnCl₂ and 5 µg/mL 15/7 antibody for 30 minutes on ice. Mn⁺² activates the receptor to enhance ligand binding, and 15/7 is a monoclonal antibody that recognizes an activated/ligand occupied conformation of α₄β₁ integrin and locks the molecule into this conformation thereby stabilizing the VCAM-1/α₄β₁ integrin interaction. Yednock *et al*., *supra*. Antibodies similar to the 15/7 antibody have been prepared by other investigators (Luque *et al*., 1996, *J. Bio. Chem*., 271:11067) and may be used in this assay.

Cells were then incubated for 30 minutes at room temperature with candidate reagents, in various concentrations ranging from 66 µg/mL to 0.01 µg/mL using a standard 5-point serial dilution. 15 µL soluble recombinant VCAM-1 fusion protein was then added to Jurkat cells and incubated for 30 minutes on ice. (Yednock *et al*., *supra*.).

Cells were then washed two times and resuspended in PE-conjugated goat F(ab')₂ anti-mouse IgG Fc (Immunotech, Westbrook, ME) at 1:200 and incubated on ice, in the dark, for 30 minutes. Cells were washed twice and analyzed with a standard fluorescence activated cell sorter ("FACS") analysis as described in Yednock *et al*., *supra.*

Reagents having an IC₅₀ of less than about 15 µM possess binding affinity to α₄β₁.

### Example 7

### In vitro Saturation Assay For Determining Binding of Candidate Reagents to α₄β₁

The following describes an *in vitro* assay to determine the plasma levels needed for a reagent to be active in the Experimental Autoimmune Encephalomyelitis ("EAE") model, described in the next example, or in other *in vivo* models.

Log-growth Jurkat cells are washed and resuspended in normal animal plasma containing 20 µg/ml of the 15/7 antibody (described in the above example).

The Jurkat cells are diluted two-fold into either normal plasma samples containing known candidate reagent amounts in various concentrations ranging from 66 µg/mL to 0.01 µg/mL, using a standard 12 point serial dilution for a standard curve, or into plasma samples obtained from the peripheral blood of candidate reagent-treated animals.

Cells are then incubated for 30 minutes at room temperature, washed twice with phosphate-buffered saline ("PBS") containing 2% fetal bovine serum and 1 mM each of calcium chloride and magnesium chloride (assay medium) to remove unbound 15/7 antibody.

The cells are then exposed to phycoerythrin-conjugated goat F(ab')₂ anti-mouse IgG Fc (Immunotech, Westbrook, ME), which has been adsorbed for any non-specific cross-reactivity by co-incubation with 5% serum from the animal species being studied, at 1:200 and incubated in the dark at 4°C for 30 minutes.

Cells are washed twice with assay medium and resuspended in the same. They are then analyzed with a standard fluorescence activated cell sorter analysis as described in Yednock et al., 1995, J. Bio. Chem., 270: 28740.

The data is then graphed as fluorescence versus dose, *e.g*., in a normal dose-response fashion. The dose levels that result in the upper plateau of the curve represent the levels needed to obtain efficacy in an *in vivo* model.

This assay may also be used to determine the plasma levels needed to saturate the binding sites of other integrins, such as the α₉β₁ integrin, which is the integrin most closely related to α₄β₁ (Palmer *et al*., 1993, *J. Cell Bio.,* 123: 1289).

Accordingly, the above-described assay may be performed with a human colon carcinoma cell line, SW 480 (ATTC #CCL228) transfected with cDNA encoding α₉ integrin (Yokosaki *et al*., 1994, *J*. *Bio. Chem*., 269:26691), in place of the Jurkat cells, to measure the binding of the α₉β₁ integrin. As a control, SW 480 cells which express other α and β₁ subunits may be used.

Using this assay, the plasma levels necessary to obtain efficacy in *in vivo* models for α₄β₁ and α₉β₁ have been established for reagents of the present invention tested in this assay.

### Example 8

### Construction of Humanized 21.6 Antibody

Chimeric light and heavy chains were constructed by linking the PCR-cloned cDNAs of mouse 21.6 V*_{L}* and V*_{H}* regions to human constant regions. The 5'- and 3'-ends of the mouse cDNA sequences were modified using specially designed PCR primers. The 5'-end PCR-primers (Table 1), which hybridize to the DNA sequences coding for the beginnings of the leader sequences, were designed to create the DNA sequences essential for efficient translation (Kozak, 1987, Mol. Biol. 196: 947-950), and to create a *Hind*III restriction site for cloning into an expression vector. The 3'-end primers, which hybridize to the DNA sequences coding for the ends of J regions, were designed to create the DNA sequences essential for splicing to the constant regions, and to create a *Bam*HI site for cloning into an expression vector. The products of PCR amplification were digested with *Hind*III and *Bam*HI, cloned into a pUC19 vector, and sequenced to confirm that no errors had occurred during PCR amplification. The adapted mouse 21.6 variable regions were then subcloned into mammalian cells expression vectors containing either the human kappa or gamma-1 constant regions.

**TABLE 1**

| **PCR Primers for the Construction of Chimeric 21.6 Antibody** | | |
|---|---|---|
| A. Light Chain Variable Region | | |
| 1. Primer for reconstruction of the 5'-end (37-mer) | | |
| 5' C AGA AAG CTT GCC GCC ACC ATG AGA CCG TCT ATT CAG 3' HindIII Kozak M R P S I Q Consensus Sequence | | |
| 2. Primer for reconstruction of the 3'-end (35-mer) | | |
| 5' CC GAG GAT CCA CTC ACG TTT GAT TTC CAG CTT GGT 3' BamHI Splice donor site | | |

| B. Heavy chain variable region | | |
|---|---|---|
| 1. Primer for reconstruction of the 5'-end (37-mer) | | |
| 5' C AGA AAG CTT GCC GCC ACC ATG AAA TGC AGC TGG GTC 3' HindIII Kozak M K C S W V Consensus Sequence | | |
| 2. Primer for reconstruction of the 3'-end (33-mer) | | |
| 5' CC GAG GAT CCA CTC ACC TGA GGA GAC GGT GAC T 3' BamHI Splice donor site | | |

Modeling the Structure of the Mouse 21.6 Variable Regions. A molecular model of the V*_{L}* and V*_{H}* regions of mouse 21.6 antibody was built. The model was built on a Silicon Graphics IRIS 4D workstation running under the UNIX operating system and using the molecular modeling package QUANTA (Polygen Corp., USA). The structure of the FRs of mouse 21.6 V*_{L}* region was based on the solved structure of human Bence-Jones immunoglobulin RE1 (Epp et al., 1975 Biochemistry 14: 4943-4952). The structure of the FRs of mouse 21.6 V*_{H}* region was based on the solved structure of mouse antibody Gloop2. Identical residues in the FRs were retained; non-identical residues were substituted using the facilities within QUANTA. CDR1 and CDR2 of mouse 21.6 V*_{L}* region were identified as belonging to canonical structure groups 2 and 1, respectively (Chothia et al., 1987, J. Mol. Biol. 196: 901-917). Since CDR1 and CDR2 of RE1 belong to the same canonical groups, CDR1 and CDR2 of mouse 21.6, V*_{L}* region were modeled on the structures of CDR1 and CDR2 of RE1. CDR3 of mouse 21.6 V*_{L}* region did not appear to correspond to any of the canonical structure groups for CDR3s of V*_{L}* regions. A database search revealed, however, that CDR3 in mouse 21.6 V*_{L}* region was similar to CDR3 in mouse HyHEL-5 V*_{L}* region (Sheriff et al., 1987, Proc. Natl. Acad. Sci. USA 84: 8075-8079). Thus, the CDR3 of mouse 21.6 V*_{L}* region was modeled on the structure of CDR3 in mouse HyHEL-5 V*_{L}* region. CDR1 and CDR2 of mouse 21.6 V*_{H}* region were identified as belonging to canonical structure groups 1 and 2, respectively. CDR1 of mouse 21.6 V*_{H}* region was modeled on CDR1 of Gloop2 V*_{H}* region, which closely resembles members of canonical group 1 for CDR1s of V*_{H}* regions. CDR2 of mouse 21.6 V*_{H}* region was modeled on CDR2 of mouse HyHEL-5 (Sheriff *et al*., *supra*), which is also a member of canonical group 2 for CDR2 for V*_{H}* regions. For CDR3s of V*_{H}* regions, there are no canonical structures. However, CDR3 in mouse 21.6 V*_{H}* region was similar to CDR3 in mouse R19.9 V*_{H}* region (Lascombe et al., 1989, Pro. Natl. Acad. Sci. USA 86: 607-611) and was modeled on this CDR3 by removing an extra serine residue present at the apex of the CDR3 loop of mouse R19.9 V*_{H}* region and annealing and refining the gap. The model was finally subjected to steepest descents and conjugate gradients energy minimization using the CHARMM potential (Brooks et al., 1983, J. Comp. Chem. 4: 187-217), as implemented in QUANTA in order to relieve unfavorable atomic contacts and to optimize van der Waals and electrostatic interactions.

Design of Reshaped Human 21.6 Variable Regions -Selection of Homologous Human Antibodies for Framework Sequence. Human variable regions whose FRs showed a high percent identity to those of mouse 21.6 were identified by comparison of amino acid sequences. Tables 3 and 4 compare the mouse 21.6 variable regions to all known mouse variable regions and then to all known human variable regions. The mouse 21.6 V*_{L}* region was identified as belonging to mouse kappa V*_{L}* region subgroup 5 as defined by Kabat. Individual mouse kappa V*_{L}* regions were identified that had as much as 93.4% identity to the mouse 21.6 kappa V*_{L}* region (38C13V'CL and PC613'CL). Mouse 21.6 V*_{L}* region was most similar to human kappa V*_{L}* regions of subgroup 1, as defined by Kabat. Individual human kappa V*_{L}* regions were identified that had as much as 72.4% identity to the mouse 21.6 kappa V*_{L}* region. The framework regions (FRs) from one of the most similar human variable regions, RE1, were used in the design of reshaped human 21.6 V*_{L}* region. Mouse 21.6 V*_{H}* region was identified as belonging to mouse V*_{H}* region subgroup 2c as defined by Kabat. Individual mouse heavy chain variable regions were identified that have as much as 93.3% identity to the mouse 21.6 V*_{H}* region (17.2.25'CL and 87.92.6'CL). Mouse 21.6 V*_{H}* region was most similar to human V*_{H}* regions of subgroup 1 as defined by Kabat *et al*., *supra.* Individual human V*_{H}* regions were identified that had as much as 64.7% identity to the mouse 21.6 V*_{H}* region. The FRs from one of the most similar human variable regions, 21/28'CL, was used in the design of reshaped human 21.6 V*_{H}* region.

### Substitution of Amino Acids in Framework Regions

(A) Light Chain. The next step in the design process for the reshaped human 21.6 V*_{L}* region was to join the CDRs from mouse 21.6 V*_{L}* region to the FRs from human RE1 (Palm et al., 1975, Physiol. Chem. 356: 167-191). In the first version of reshaped human 21.6 V*_{L}* region (La), seven changes were made in the human FRs. At positions 104, 105, and 107 in FR4, amino acids from RE1 were substituted with more typical human J region amino acids from another human kappa light chain (Riechmann et al., 1988, Nature 332: 323-327).

At position 45 in FR2, the lysine normally present in RE1 was changed to an arginine as found at that position in mouse 21.6 V*_{L}* region. The amino acid residue at this position was thought to be important in the supporting the CDR2 loop of the mouse 21.6 V*_{L}* region.

At position 49 in FR2, the tyrosine normally present in RE1 was changed to a histidine as found at that position in mouse 21.6 V*_{L}* region. The histidine at this position in mouse 21.6 V*_{L}* region was observed in the model to be located in the middle of the binding site and could possibly make direct contact with antigen during antibody-antigen binding.

At position 58 in FR3, the valine normally present in RE1 was changed to an isoleucine as found at that position in mouse 21.6 V*_{L}* region. The amino acid residue at this position was thought to be important in the supporting the CDR2 loop of the mouse 21.6 V*_{L}* region.

At position 69 in FR3, the threonine normally present in RE1 was changed to an arginine as found at that position in mouse 21.6 V*_{L}* region. The arginine at this position in mouse 21.6 V*_{L}* region was observed in the model to be located adjacent to the CDR1 loop of mouse 21.6 V*_{L}* region and could possibly make direct contact with the antigen during antibody-antigen binding.

A second version of reshaped human 21.6 V*_{L}* region (termed Lb) was designed containing the same substitutions as above except that no change was made at position 49 in FR2 of RE1.

(B) Heavy Chain. The next step in the design process for the reshaped human 21.6 V*_{H}* region was to join the CDRs from mouse 21.6 V*_{H}* region to the FRs from 21/28'CL (Dersimonian et al., 1987, J. Immunol. 139: 2496-2501). In the first version of reshaped human 21.6 V region (Ha), five changes were made in the human framework regions. The five changes in the human FRs were at positions 27, 28, 29, 30, and 71.

At positions 27, 28, 29, and 30 in FR1, the amino acids present in human 21/28'CL were changed to the amino acids found at those positions in mouse 21.6 V*_{H}* region. Although these positions are designated as being within FR1 (Kabat *et al*., *supra*), positions 26 to 30 are part of the structural loop that forms the CDR1 loop of the V*_{H}* region. It is likely, therefore, that the amino acids at these positions are directly involved in binding to antigen. Indeed, positions 27 to 30 are part of the canonical structure for CDR1 of the V*_{H}* region as defined by Chothia *et al*., *supra*.

At position 71 in FR3, the arginine present in human 21/28'CL was changed to a alanine as found at that position in mouse 21.6 V*_{H}* region. Position 71 is part of the canonical structure for CDR2 of the V*_{H}* region as defined by Chothia *et al*., *supra.* From the model of the mouse 21.6 variable regions, it appears that the alanine at position 71 is important in supporting the CDR2 loop of the V*_{H}* region. A substitution of an arginine for an alanine at this position would very probably disrupt the placing of the CDR2 loop.

A second version (Hb) of reshaped human 21.6 V*_{H}* region contains the five changes described above for version Ha were made plus one additional change in FR2.

At position 44 in FR2, the arginine present in human 21/28'CL was changed to a glycine as found at that position in mouse 21.6 V*_{H}* region. Based on published information on the packing of V*_{L}*-V*_{H}* regions and on the model of the mouse 21.6 variable regions, it was thought that the amino acid residue at position 44 might be important in the packing of the V*_{L}*-*V_{H}* regions.

Reshaped human 21.6 V region version Hc was designed to make the CDR3 loop look more similar to human VCAM-1. Both mouse 21.6 antibody and human VCAM-1 bind to the α₄β₁ integrin. The CDR3 loop of the V*_{H}* region of antibodies is the most diverse of the six CDR loops and is generally the most important single component of the antibody in antibody-antigen interactions (Chothia *et al*., *supra*; Hoogenboom & Winter, 1992, J. Mol. Biol. 227: 381-388); Barbas et al., 1992, Proc. Natl. Acad. Sci. USA 89: 4457-4461). Some sequence similarity was identified between the CDR3 of mouse 21.6 V*_{H}* region and amino acids 86 to 94 of human VCAM-1, particularly, between the YGN (Tyrosine-Glycine-Asparagine) sequence in the CDR3 loop and the FGN (*i.e.,* Phenylalanine-Glycine-Asparagine) sequence in VCAM-1. These sequences are thought to be related to the RGD (*i.e.,* Arginine-Glycine-Aspartic acid) sequences important in various cell adhesion events (Main et al., 1992, Cell 71: 671-678). Therefore, at position 98 in CDR3, the tyrosine present in mouse 21.6 V*_{H}* region was changed to a phenylalanine as found in the sequence of human VCAM-1.

Possible substitution at position 36 in FR2 was also considered. The mouse 21.6 V_{H} chain contains an unusual cysteine residue at position 36 in FR2. This position in FR2 is usually a tryptophan in related mouse and human sequences. Although cysteine residues are often important for conformation of an antibody, the model of the mouse 21.6 variable regions did not indicate that this cysteine residue was involved either directly or indirectly with antigen binding so the tryptophan present in FR2 of human 21/28'CL V*_{H}* region was left unsubstituted in all three versions of humanized 21.6 antibody.

Construction of Reshaped Human 21.6 Antibodies. The first version of reshaped human 21.6 V*_{L}* region (resh21.6VLa) was constructed from overlapping PCR fragments essentially as described by Daugherty et al., 1991, Nucleic Acids Res. 19: 2471-2476. The mouse 21.6 V*_{L}* region, adapted as described *supra* and inserted into pUC19, was used as a template. Four pairs of primers, APCR1-vlal, vla2-vla3, vla4-vla5, and vla6-vla7 were synthesized. Adjacent pairs overlapped by at least 21 bases. The APCR1 primer is complementary to the pUC19 vector. The appropriate primer pairs (0.2 µmoles) were combined with 10 ng of template DNA, and 1 unit of AmpliTaq DNA polymerase (Perkin Elmer Cetus) in 50 µL of PCR buffer containing 10 mM Tris-HCl (pH 8.3), 50 mM KCI, 200 µM dNTPs, and 1.5 mM MgCl₂. Each reaction was carried out for 25 cycles. After an initial melt at 94°C for 5 min, the reactions were cycled at 94°C for 1 min, 55°C for 1 min, and 72°C for 2 min, and finally incubated at 72°C for a further 10 min. The ramp time between the primer-annealing and extension steps was 2.5 min. The products of the four reactions (A, B, C, and D) from the first round of PCR reactions were phenol-extracted and ethanol-precipitated.

**TABLE 2**

| **PCR primers for the construction of reshaped human 21.6 variable regions** |
|---|
| A. Light chain variable region |
| 1. Primers for the synthesis of version "a" |
| 21.6VLa1 (39-mer): |
| 5' GAT GGT GAC TCT ATC TCC TAC AGA TGC AGA CAG TGA GGA 3' |
| |
| 21.6VLa2 (32-mer): |
| 5' CTG TAG GAG ATA GAG TCA CCA TCA CTT GCA AG 3' |
| |
| 21.6VLa3 (39-mer): |
| 5' AGG AGC TTT TCC AGG TGT CTG TTG GTA CCA AGC CAT ATA 3' |
| |
| 21.6VLa4 (41-mer): |
| 5' ACC AAC AGA CAC CTG GAA AAG CTC CTA GGC TGC TCA TAC AT 3' |
| |
| 21.6VLa5 (40-mer): |
| 5' GCA GGC TGC TGA TGG TGA AAG TAT AAT CTC TCC CAG ACC C 3' |
| |
| 21.6VLa6 (42-mer): |
| 5' ACT TTC ACC ATC AGC AGC CTG CAG CCT GAA GAT ATT GCA ACT 3' |
| |
| 21.6VLa7 (59-mer): |
| 5' CCG AGG ATC CAC TCA CGT TTG ATT TCC ACC TTG GTG CCT TGA CCG AAC GTC CAC AGA TT 3' |
| |
| |
| 2. Primers for the synthesis of version "b" |
| 21.6VLb1 (33-mer): changes H-49 to Y-49 |
| 5' GGA AAA GCT CCT AGG CTG CTC ATA TAT TAC ACA 3' |
| |
| 21.6VLb2 (38-mer): changes ACC-101 to ACA-101 to destroy an *Sty*I site |
| 5' CCG AGG ATC CAC TCA CGT TTG ATT TCC ACC TTT GTG CC 3' |
| |
| B. Heavy chain variable region |
| 1. Primers for the synthesis of version "a" |
| 21.6VHa1 (51-mer): |
| 5' AAC CCA GTG TAT ATA GGT GTC TTT AAT GTT GAA ACC GCT AGC TTT ACA GCT 3' |
| |
| 21.6VHa2 (67-mer): |
| 5' AAA GAC ACC TAT ATA CAC TGG GTT AGA CAG GCC CCT GGC CAA AGG CTG GAG TGG ATG GGA AGG ATT G 3' |
| |
| 21.6VHa3 (26-mer): |
| 5' GAC CCG GCC CTG GAA CTT CGG GTC AT 3' |
| |
| 21.6VHa4 (66-mer): |
| 5' GAC CCG AAG TTC CAG GGC CGG GTC ACC ATC ACC GCA GAC ACC TCT GCC AGC ACC GCC TAC ATG GAA 3' |
| 21.6VHa5 (64-mer): |
| 5' CCA TAG CAT AGA CCC CGT AGT TAC CAT AAT ATC CCT CTC TGG CGC AGT AGT AGA CTG CAG TGT C 3' |
| |
| 21.6VHa6 (63-mer): |
| 5' GGT AAC TAC GGG GTC TAT GCT ATG GAC TAC TGG GGT CAA GGA ACC CTT GTC ACC GTC TCC TCA 3' |
| |
| 2. Primer for the synthesis of version "b" |
| 21.6VHb (37-mer): changes R-44 to G-44 |
| 5'CCA GGG CCG GGTCAC CAT CAC CAG AGA CAC CTC TGC C 3' |
| |
| 3. Primer for the synthesis of version "c" |
| 21.6VHc (27-mer): changes Y-98 to F-98 |
| 5'CAG GCC CCT GGC CAA GGG CTG GAG TGG 3' |
| |
| C. Both light and heavy chain variable regions |
| Primers hybridizing to the flanking pUC19 vector DNA APCR1 (17-mer, sense primer) |
| 5'TAC GCA AAC CGC CTC TC 3' |
| |
| APCR4 (18-mer, anti-sense primer) |
| 5' GAG TGC ACC ATA TGC GGT 3' |

PCR products A and B, and C and D were joined in a second round of PCR reactions. PCR products A and B, and C and D, (50 ng of each) were added to 50 µL PCR reactions (as described *supra*) and amplified through 20 cycles as described above, except that the annealing temperature was raised to 60°C. The products of these reactions were termed E and F. The pairs of PCR primers used were APCR1-vla3 and vla4-vla7, respectively. PCR products E and F were phenol-extracted and ethanol-precipitated and then assembled in a third round of PCR reactions by their own complementarity in a two step-PCR reaction similar to that described above using APCR1 and vla7 as the terminal primers. The fully assembled fragment representing the entire reshaped human 21.6 V*_{L}* region including a leader sequence was digested with *Hind*III and *Bam*HI and cloned into pUC 19 for sequencing. A clone having the correct sequence was designated resh21.6VLa.

The second version of a reshaped human 21.6 V*_{L}* region (Lb) was constructed using PCR primers to make minor modifications in the first version of reshaped human 21.6 V*_{L}* region (La) by the method of Kamman *et al*., 1989, *Nucl*. *Acids Res.* 17: 5404). Two sets of primers were synthesized. Each PCR reaction was essentially carried out under the same conditions as described above. In a first PCR reaction, mutagenic primer 21.6VLb2 was used to destroy a *Sty*I site (Thr-ACC-97 to Thr-ACA-97) to yield resh21.6VLa2. Then, in a second PCR reaction, mutagenic primer 21.6VLb1 (His-49 to Tyr-49) was used with pUC-resh21.6VLa2 as template DNA. The PCR product was cut with *Sty*I and *Bam*HI and subcloned into pUC-resh21.6VLa2, cleaved with the same restriction enzymes. A clone with the correct sequence was designated pUC-resh21.6VLb.

Version "a" of a reshaped human 21.6 V*_{H}* region was constructed using the same PCR methods as described for the construction of version "a" of reshaped human 21.6 V*_{L}* region. The *Hind*III-*Bam*HI DNA fragments coding for version "g" of reshaped human 425 V*_{H}* region (Kettleborough *et al*., *supra*) and version "b" of reshaped human AUK12-20 V*_{H}* region were subcloned into pUC19 vectors yielding pUC-resh425g and pUC-reshAUK12-20b, respectively. (Version "b" of AUK12-20, was derived by PCR mutagenesis of a fragment V*_{H}* a425 described by Kettleborough *et al*., *supra,* and encodes the amino acid sequence: 
QVQLVQSGAEVKKPGASVKVSCKASGYSFT SYYIH WVRQAPGQGLEWVG YIDPFNGGTSYNQKFKG
KVTMTVDTSTNTAYMELSSLRSEDTAVYYCAR GGN-RFAY WGQGTLVTVSS
 (spaces separate FR and CDR regions).

Plasmid pUC-resh425g and pUC-reshAUK12-20b, as well as the pUC vector containing the mouse 21.6 V*_{H}* region as modified for use in the construction of the chimeric 21.6 heavy chain (pUC-chim21.6V*_{H}*), were used as template DNAs in the subsequent PCR reactions. PCR primers were designed and synthesized for the construction of version "a" of reshaped human 21.6 V*_{H}* region. PCR product A was obtained using pUC-reshAUK12-20b as DNA template and APCR1-vha1 as the PCR primer pair. PCR products B and D were obtained using pUC-chim21.6V*_{H}* as DNA template and vha2-vha3 and vha6-APCR4 as PCR primer pairs, respectively. Finally, PCR product C was obtained using pUC-resh425g as DNA template and vla4-vla5 as the PCR primer pair. The final PCR product was subcloned into pUC 19 as a *Hind*III-*Bam*HI fragment for DNA sequencing. A clone with the correct DNA sequence was designated pUC-resh21.6VHa.

The remaining versions of reshaped human 21.6 V*_{H}* region were constructed essentially as described above for the construction of version "b" of reshaped human 21.6 V*_{L}* region. Two sets of primers were synthesized. For the second (Hb) and third (Hc) versions, mutagenic primers 21.6VHb (Arg-44 to Gly-44) and 21.6VHc (Tyr-98 to Phe-98), respectively, were used in PCR reactions with pUC-resh21.6VHa as the template DNA. The PCR products VHb and VHc were cut with restriction enzymes and subcloned into pUC vector pUC-resh21.6VHa as *Msc*I-*Bam*HI and *Pst*T-*Bam*HI fragments, respectively, to yield pUC-resh21.6VHb and pUC-resh21.6VHc.

The first version of a reshaped human 21.6 V*_{H}* region (Ha) was constructed in a similar manner to that used for the construction of the first version of reshaped human 21.6 V*_{L}* region (La). In this case, however, PCR primers were used with three different template DNAs, mouse 21.6 V*_{H}* region as already adapted for expression of chimeric 21.6 heavy chain, humanized 425 V*_{H}* region version "g" (Kettleborough *et al., supra*), and humanized AUK12-20 version "b" V*_{H}* region. The second and third versions of a humanized 21.6 V*_{H}* region (Hb and Hc) were constructed using PCR primers to make minor modifications in the first version of humanized 21.6 V*_{H}* region (Ha).

**TABLE 3**

| Alignment of amino acid sequences leading to the design of reshaped human 21.6 light chain variable regions. | | | | | | | | |
|---|---|---|---|---|---|---|---|---|
| Kabat | # | FR or CDR | mouse 21.6 | mouse kappa 5 | human kappa 1 | human RE1 | RH V_{L} 21.6 | Comment |
| 1 | 1 | FR1 | D | D | D | D | D | |
| 2 | 2 | \| | I | I | I | I | | |
| 3 | 3 | \| | Q | Q | Q | Q | Q | |
| 4 | 4 | \| | M | M | M | M | M | |
| 5 | 5 | \| | T | T | T | T | T | |
| 6 | 6 | \| | Q | Q | Q | Q | Q | |
| 7 | 7 | \| | S | S | S | S | S | |
| 8 | 8 | \| | P | P | P | P | P | |
| 9 | 9 | \| | S | S | S | S | S | |
| 10 | 10 | \| | S | S | S | S | S | |
| 11 | 11 | \| | L | | L | L | L | |
| 12 | 12 | \| | S | S | S | S | S | |
| 13 | 13 | \| | A | A | A | A | A | |
| 14 | 14 | \| | S | S | S | S | S | |
| 15 | 15 | \| | L | | V | V | V | |
| 16 | 16 | \| | G | G | G | G | G | |
| 17 | 17 | \| | G | D | D | D | D | |
| 18 | 18 | \| | K | R | R | R | R | |
| 19 | 19 | \| | V | V | V | V | V | |
| 20 | 20 | \| | T | T | T | T | T | |
| 21 | 21 | \| | I | I | I | I | I | |
| 22 | 22 | \| | T | T | T | T | T | |
| 23 | 23 | FR1 | C | C | C | C | C | |
| 24 | 24 | CDR1 | K | R | R | Q | K | |
| 25 | 25 | \| | T | A | A | A | T* | |
| 26 | 26 | \| | S | S | S | S | S | |
| 27 | 27 | \| | Q | Q | Q | Q | Q* | |
| 27A | | \| | - | D | S | - | - | |
| 27B | | \| | - | - | L | - | - | |
| 27C | | \| | - | - | V | - | - | |
| 27D | | \| | - | - | X | - | | |
| 27E | | \| | - | - | X | - | - | |
| 27F | | \| | - | - | - | - | - | |
| 28 | 28 | \| | D | D | S | D | D* | |
| 29 | 29 | \| | I | I | I | I | I* | |
| 30 | 30 | \| | N | S | S | I | N* | |
| 31 | 31 | \| | K | N | N | K | K* | |
| 32 | 32 | \| | Y | Y | Y | Y | Y* | |
| 33 | 33 | \| | M | L | L | L | M* | |
| 34 | 34 | CDR1 | A | N | A | N | A | |
| 35 | 35 | FR2 | W | W | W | W | W | |
| 36 | 36 | \| | Y | Y | Y | Y | Y | |
| 37 | 37 | \| | Q | Q | Q | Q | Q | |
| 38 | 38 | \| | H | Q | Q | Q | Q | |
| 39 | 39 | \| | K | K | K | T | T | K in CAMPATH-1H |
| 40 | 40 | \| | P | P | P | P | P | |
| 41 | 41 | \| | G | G | G | G | G | |
| 42 | 42 | | K | G | K | K | K | |
| 43 | 43 | \| | R | S | A | A | A | consider R in other versions |
| 44 | 44 | \| | P | P | P | P | P | |
| 45 | 45 | \| | R | K | K | K | R | supports L2 loop, consider K in other versions |
| 46 | 46 | \| | L | L | L | L | L | |
| 47 | 47 | \| | L | L | L | L | L | |
| 48 | 48 | \| | I | I | I | I | I* | |
| 49 | 49 | \| | H | Y | Y | Y | H | in middle of binding site, potential to interact with antigen, consider Y in other versions |
| 50 | 50 | CDR2 | Y | Y | A | E | Y* | |
| 51 | 51 | \| | T | A | A | A | T* | |
| 52 | 52 | \| | S | S | S | S | S* | |
| 53 | 53 | \| | A | R | S | N | A | |
| 54 | 54 | \| | L | L | L | L | L | |
| 55 | 55 | \| | Q | H | E | Q | Q | |
| 56 | 56 | CDR2 | P | S | S | A | P | |
| 57 | 57 | FR3 | G | G | G | G | G | |
| 58 | 58 | \| | I | V | V | V | I | maybe supporting L2, consider V in other versions |
| 59 | 59 | \| | P | P | P | P | P | |
| 60 | 60 | \| | S | S | S | S | S | |
| 61 | 61 | \| | R | R | R | R | R | |
| 62 | 62 | \| | F | F | F | F | F | |
| 63 | 63 | \| | S | S | S | S | S | |
| 64 | 64 | \| | G | G | G | G | G* | |
| 65 | 65 | \| | S | S | S | S | S | |
| 66 | 66 | \| | G | G | G | G | G | |
| 67 | 67 | \| | S | S | S | S | S | |
| 68 | 68 | \| | G | G | G | G | G | |
| 69 | 69 | \| | R | T | T | T | R | adjacent to L1, on the surface near the binding site |
| 70 | 70 | \| | D | D | D | D | D | |
| 71 | 71 | \| | Y | Y | F | Y | Y* | F in CAMPATH-1H |
| 72 | 72 | \| | S | S | T | T | T | |
| 73 | 73 | \| | F | L | L | P | F | |
| 74 | 74 | \| | N | T | T | T | T | |
| 75 | 75 | \| | I | I | I | I | I | |
| 76 | 76 | \| | S | S | S | S | S | |
| 77 | 77 | \| | N | N | S | S | S | |
| 78 | 78 | \| | L | L | L | L | L | |
| 79 | 79 | \| | E | E | Q | Q | Q | |
| 80 | 80 | \| | P | Q | P | P | P | |
| 81 | 81 | \| | E | E | E | E | E | |
| 82 | 82 | \| | D | D | D | D | D | |
| 83 | 83 | \| | I | I | F | I | I | |
| 84 | 84 | \| | A | A | A | A | A | |
| 85 | 85 | \| | T | T | T | T | T | |
| 86 | 86 | \| | Y | Y | Y | Y | Y | |
| 87 | 87 | \| | Y | F | Y | Y | Y | |
| 88 | 88 | FR3 | C | C | C | C | C | |
| 89 | 89 | CDR3 | L | Q | Q | Q | L | |
| 90 | 90 | \| | Q | Q | Q | Q | Q* | |
| 91 | 91 | \| | Y | G | Y | Y | Y* | |
| 92 | 92 | \| | D | N | N | Q | D* | |
| 93 | 93 | \| | N | T | S | S | N* | |
| 94 | 94 | \| | L | L | L | L | L* | |
| 95 | 95 | \| | - | P | P | P | - | |
| 95A | | \| | - | P | E | - | - | |
| 95B | | \| | - | - | - | - | - | |
| 95C | | \| | - | - | - | - | - | |
| 95D | | \| | - | - | - | - | - | |
| 95E | | \| | - | - | - | - | - | |
| 95F | | \| | - | - | - | - | - | |
| 96 | 95 | \| | W | R | W | Y | W* | |
| 97 | 96 | CDR3 | T | T | T | T | T | |
| 98 | 97 | FR4 | F | F | F | F | F | |
| 99 | 98 | \| | G | G | G | G | G | |
| 100 | 99 | \| | G | G | Q | Q | Q | |
| 101 | 100 | \| | G | G | G | G | G | |
| 102 | 101 | \| | T | T | T | T | T | |
| 103 | 102 | \| | K | K | K | K | K | |
| 104 | 103 | \| | L | L | V | L | V | as in CAMPATH-1H |
| 105 | 104 | \| | E | E | E | Q | E | as in CAMPATH-1H |
| 106 | 105 | \| | I | I | I | I | I | |
| 106A | | \| | - | - | - | - | - | |
| 107 | 106 | FR4 | K | K | K | T | K | as in CAMPATH-1H |

| | | | | | | | | |
|---|---|---|---|---|---|---|---|---|
| Legend: (Kabat) numbering according to Kabat *et al., supra*; (#) sequential numbering as used in the molecular modeling; (mouse 21.6) amino acid sequence of the V*_{L}* region from mouse 21.6 antibody; (mouse kappa 5) consensus sequence of mouse kappa V*_{L}* regions from subgroup 5 (Kabat *et al*., *supra*); (human kappa 1) consensus sequence of human V*_{L}* regions from subgroup 1 (Kabat *et al*., *supra);* (human RED amino acid sequence of a human V*_{L}* region (Palm et al., Physiol. Chem. 356: 167-191 (1975)); (RH V*_{L}* 21.6) amino acid sequence of version L1 of reshaped human 21.6 V*_{L}* region; (*) residues that are part of the canonical structures for the CDR loops (Chothia *et al*., *supra*); (underlined) residues in the human FRs where the amino acid residue was changed. | | | | | | | | |

**TABLE 4**

| Alignment of amino acid sequences leading to the design of reshaped human 21.6 heavy chain variable regions. | | | | | | | | |
|---|---|---|---|---|---|---|---|---|
| Kabat | # | FR or CDR | mouse 21.6 | mouse 2c | human 1 | human 21/28'CL | RH V_{H} 21.6 | Comment |
| 1 | 1 | FR1 | E | E | Q | Q | Q | |
| 2 | 2 | \| | V | V | V | V | V | |
| 3 | 3 | \| | Q | Q | Q | Q | Q | |
| 4 | 4 | \| | L | L | L | L | L | |
| 5 | 5 | \| | Q | Q | V | V | V | |
| 6 | 6 | \| | Q | Q | Q | Q | Q | |
| 7 | 7 | \| | S | S | S | S | S | |
| 8 | 8 | \| | G | G | G | G | G | |
| 9 | 9 | \| | A | A | A | A | A | |
| 10 | 10 | \| | E | E | E | E | E | |
| 11 | 11 | \| | L | L | V | V | V | |
| 12 | 12 | \| | V | V | K | K | K | |
| 13 | 13 | \| | K | K | K | K | K | |
| 14 | 14 | \| | P | P | P | P | P | |
| 15 | 15 | \| | G | G | G | G | G | |
| 16 | 16 | \| | A | A | A | A | A | |
| 17 | 17 | \| | S | S | S | S | S | |
| 18 | 18 | \| | V | V | V | V | V | |
| 19 | 19 | \| | K | K | K | K | K | |
| 20 | 20 | \| | L | L | V | V | V | |
| 21 | 21 | \| | S | S | S | S | S | |
| 22 | 22 | \| | C | C | C | C | C | |
| 23 | 23 | \| | T | T | K | K | K | |
| 24 | 24 | \| | A | A | A | A | A | |
| 25 | 25 | \| | S | S | S | S | S | |
| 26 | 26 | \| | G | G | G | G | G* | |
| 27 | 27 | \| | F | F | Y | Y | F* | H1 canonical structure, consider Y in other versions |
| 28 | 28 | \| | N | N | T | T | N* | H1 canonical structure, on the surface |
| 29 | 29 | \| | I | I | F | F | I* | H1 canonical structure, consider F in other versions |
| 30 | 30 | FR1 | K | K | T | T | K* | H1 canonical structure, on the surface |
| 31 | 31 | CDR1 | D | D | S | S | D* | |
| 32 | 32 | \| | T | T | Y | Y | T* | |
| 33 | 33 | \| | Y | Y | A | A | Y | |
| 34 | 34 | \| | I | M | I | M | I* | |
| 35 | 35 | \| | H | H | S | H | H | |
| 35A | | \| | - | - | - | - | - | |
| 35B | | CDR1 | - | - | - | - | - | |
| 36 | 36 | \|FR2 | C | W | W | W | W | buried residue, no obvious special role for C |
| 37 | 37 | \| | V | V | V | V | V | |
| 38 | 38 | \| | K | K | R | R | R | |
| 39 | 39 | \| | Q | Q | Q | Q | Q | |
| 40 | 40 | \| | R | R | A | A | A | |
| 41 | 41 | \| | P | P | P | P | P | |
| 42 | 42 | \| | E | E | G | G | G | |
| 43 | 43 | \| | Q | Q | Q | Q | Q | |
| 44 | 44 | \| | G | G | G | R | R | V_{L} - V_{H} packing, consider G in other |
| 45 | 45 | \| | L | L | L | L | L | versions |
| 46 | 46 | \| | E | E | E | E | E | |
| 47 | 47 | \| | W | W | W | W | W | |
| 48 | 48 | \| | I | I | M | M | M | |
| 49 | 49 | FR2 | G | G | G | G | G | |
| 50 | 50 | CDR2 | R | R | W | W | R | |
| 51 | 51 | \| | I | I | I | I | I | |
| 52 | 52 | \| | D | D | N | N | D | |
| 52A | 53 | \| | P | P | P | A | P* | |
| 52B | | \| | - | - | - | - | - | |
| 52C | | \| | - | - | - | - | - | |
| 53 | 54 | \| | A | A | G | G | A* | |
| 54 | 55 | \| | N | N | N | N | N* | |
| 55 | 56 | \| | G | G | G | G | G* | |
| 56 | 57 | \| | Y | N | D | N | Y | |
| 57 | 58 | \| | T | T | T | T | T | |
| 58 | 59 | \| | K | K | N | K | K | |
| 59 | 60 | \| | Y | Y | Y | Y | Y | |
| 60 | 61 | \| | D | D | A | S | D | |
| 61 | 62 | \| | P | P | Q | Q | P | |
| 62 | 63 | \| | K | K | K | K | K | |
| 63 | 64 | \| | F | F | F | F | F | |
| 64 | 65 | \| | Q | Q | Q | Q | Q | |
| 65 | 66 | CDR2 | G | G | G | G | G | |
| 66 | 67 | FR3 | K | K | R | R | R | |
| 67 | 68 | \| | A | A | V | V | V | |
| 68 | 69 | \| | T | T | T | T | T | |
| 69 | 70 | \| | I | I | I | I | I | |
| 70 | 71 | \| | T | T | T | T | T | |
| 71 | 72 | \| | A | A | A | R | A* | H2 canonical structure, supporting H2 |
| 72 | 73 | \| | D | D | D | D | D | |
| 73 | 74 | \| | T | T | T | T | T | |
| 74 | 75 | \| | S | S | S | S | S | |
| 75 | 76 | \| | S | S | T | A | A | |
| 76 | 77 | \| | N | N | S | S | S | |
| 77 | 78 | \| | T | T | T | T | T | |
| 78 | 79 | \| | A | A | A | A | A | |
| 79 | 80 | \| | Y | Y | Y | Y | Y | |
| 80 | 81 | \| | L | L | M | M | M | |
| 81 | 82 | \| | Q | Q | E | E | E | |
| 82 | 83 | \| | L | L | L | L | L | |
| 82A | 84 | \| | S | S | S | S | S | |
| 82B | 85 | \| | S | S | S | S | S | |
| 82C | 86 | \| | L | L | L | L | L | |
| 83 | 87 | \| | T | T | R | R | R | |
| 84 | 88 | \| | S | S | S | S | S | |
| 85 | 89 | \| | E | E | E | E | E | |
| 86 | 90 | \| | D | D | D | D | D | |
| 87 | 91 | \| | T | T | T | T | T | |
| 88 | 92 | \| | A | A | A | A | A | |
| 89 | 93 | \| | V | V | V | V | V | |
| 90 | 94 | \| | Y | Y | Y | Y | Y | |
| 91 | 95 | \| | F | Y | Y | Y | Y | |
| 92 | 96 | \| | C | C | C | C | C | |
| 93 | 97 | \| | A | A | A | A | A | |
| 94 | 98 | FR3 | R | R | R | R | | |
| 95 | 99 | CDR3 | E | G | A | G | E | |
| 96 | 100 | \| | G | Y | P | G | G | |
| 97 | 101 | \| | Y | Y | G | Y | Y | |
| 98 | 102 | \| | Y | Y | Y | Y | Y | |
| 99 | 103 | \| | G | Y | G | G | G | |
| 100 | 104 | \| | N | D | S | S | N | |
| 100A | 105 | \| | Y | S | G | G | Y | |
| 100B | 106 | \| | G | X | G | S | G | |
| 100C | 107 | \| | V | V | O | - | V | |
| 100D | 108 | \| | Y | G | C | - | Y | |
| 100E | 109 | \| | A | Y | Y | - | A | |
| 100F | 110 | \| | M | Y | R | M | - | |
| 100G | | \| | - | A | 0 | - | - | |
| 100H | | \| | - | M | D | - | - | |
| 100I | | \| | - | - | Y | - | - | |
| 100J | | \| | - | - | - | - | - | |
| 100K | | \| | - | - | F | - | - | |
| 101 | 111 | \| | D | D | D | N | - | |
| 102 | 112 | CDR3 | Y | Y | Y | Y | - | |
| 103 | 113 | FR4 | W | W | W | W | - | |
| 104 | 114 | \| | G | G | G | G | G | |
| 105 | 115 | \| | Q | Q | Q | Q | Q | |
| 106 | 116 | \| | G | G | G | G | G | |
| 107 | 117 | \| | T | T | T | T | T | |
| 108 | 118 | \| | S | X | L | L | L | |
| 109 | 119 | \| | V | V | V | V | V | |
| 110 | 120 | \| | T | T | T | T | T | |
| 111 | 121 | \| | V | V | V | V | V | |
| 112 | 122 | \| | S | S | S | S | S | |
| 113 | 123 | FR4 | S | S | S | S | S | |

| | | | | | | | | |
|---|---|---|---|---|---|---|---|---|
| Legend: (Kabat) numbering according to Kabat *et al*., *supra*; (#) sequential numbering as used in the molecular modeling; (mouse 21.6) amino acid sequence of the V*_{H}* region from mouse 21.6 antibody; (mouse 2c) consensus sequence of mouse V*_{H}* regions from subgroup 2c (Kabat *et al*., *supra*); (human 1) consensus sequence of human V*_{H}* regions from subgroup 1 (Kabat *et al*., *supra*); (human 21/28'CL) amino acid sequence of a human V*_{H}* region (Dersimonian et al., J. Immunol., 139: 2496-2501 (1987)); (RH V*_{H}* 21.6) amino acid sequence of version H1 of reshaped human 21.6 V*_{H}* region; (*) residues that are part of the canonical structures for the CD loops (Chothia *et al*., *supra);* (underlined) residues in the human FRs where the amino acid residue was changed. | | | | | | | | |

### Example 9

### Natalizumab

Natalizumab is a recombinant humanized antibody (rhAb) directed against the α4 integrin molecule and inhibits cell binding mediated by α4β1 (VLA-4) and α4β7 integrins. Natalizumab binds to the α4 subcomponent, which is expressed on leukocytes, predominantly lymphocytes. The binding of the murine monoclonal antibody to α4 integrin blocks the interaction of α4β1 on these leukocytes with its counter receptor on endothelial cells, VCAM-1. The blockade of these cell adhesion molecule interactions is believed to prevent the trafficking of these leukocytes across the vascular endothelium and, subsequently, into the parenchymal tissue.

α₄ integrins bind additional ligands in tissues, including osteopontin and epitopes of fibronectin. A further mechanism of natalizumab includes the suppression of ongoing inflammatory reactions in diseased tissues by inhibition of α4-positive leukocytes with these ligands. Thus, natalizumab acts to suppress existing inflammatory activity present at the disease site, along with inhibition of further recruitment of immune cells into inflamed tissue via interaction with VCAM-1 and MadCAM-1.

Work in inflammatory bowel disease (IBD) has demonstrated the expression of vascular cell adhesion molecule-1 (VCAM-1) and mucosal addressin cell adhesion molecule (MadCAM-1) at active sites of inflammation in both inflamed and non-inflamed bowel of IBD subjects, which suggests that recruitment of leukocytes to the mucosa contributes to the inflammatory response characteristic of IBD. Therefore, an agent which disrupts VCAM-1/α4β1 and MadCAM-1/α4β7 interactions could result in reduction of lymphocyte migration and attenuate the release of cytokines and other substances which cause tissue injury. Studies of anti-α4 integrin antibodies in the cotton-top tamarin (CTT), a primate species that experiences a form of chronic IBD which has a similar pattern of expression of key adhesion molecules in inflamed bowel tissue, have shown highly significant improvement in acute colitis in comparison to placebo.

Single- and multiple-dose toxicity studies have been performed in mice, guinea pigs, and monkeys. All toxicology studies were carried out using natalizumab and included an acute study in guinea pigs, subacute studies in mouse and cynomolgus monkeys, and mutagenicity and tissue cross-reactivity studies. These studies did not demonstrate clinical or postmortem evidence of significant toxicity.

In mice, there is evidence that α4 integrin and VCAM-1 play a role in placental and cardiac development, and they may also play a wider role in fetal development. There is, therefore, a risk of an abortifacient effect or teratogenicity if α4 integrin is blocked by natalizumab. A preliminary reproductive toxicity study exposed groups of five pregnant cynomolgus monkeys to repeated intravenous doses of 0.06, 0.3, or 30 mg/kg natalizumab. One of five pregnant females in the 30 mg/kg group aborted at Day 31 of gestation after receiving five doses of natalizumab. Because the overall rate of abortion fell within the rate of spontaneous abortion in this species, the event was not believed to be related to natalizumab. An ongoing follow-up reproductive toxicity study has exposed groups of 10 to 15 pregnant cynomolgus monkeys to repeated intravenous doses of 3, 10, or 30 mg/kg. Embryo deaths have occurred at similar rates in all treatment groups: two in the control, one in the 3 mg/kg, two in the 10 mg/kg and two in the 30 mg/kg groups respectively. As a precaution, women of childbearing potential must utilize effective contraception throughout the duration of the study and for at least 3 months after the last infusion of study drug, and must have a negative pregnancy test at the time of each natalizumab dosing.

In the six-month multidose toxicity study in primates, minimal to mild lymphoplasmacytic inflammation of the mucosa of the cecum, colon, and/or rectum was noted in about half of the natalizumab-treated animals of all dose groups and was not found in the vehicle- group. The inflammation was characterized by increased numbers of lymphocytes and plasma cells within the lamina propia with occasional crypt abscesses. There was a slightly increased incidence and magnitude of the change in the colon and rectum of animals from the natalizumab 30.0 and 60.0 mg/kg/week groups, indicating a dose-response relationship. However, although there was a possible dose-response relationship, the incidence of inflammation was not related to the natalizumab serum levels. While these changes may reflect some underlying infection of the intestinal tract in the affected animals, the slightly increased incidence and magnitude of the inflammation in the animals of the two highest natalizumab dose groups, combined with the lack of its presence in the control group, indicates natalizumab may possibly have a role in this process.

Earlier studies in Crohn's disease and ulcerative colitis are summarized below. One study was a randomized, double-blind, placebo-controlled, safety, tolerability, and efficacy study of a single infusion of intravenous 3 mg/kg natalizumab in male and female subjects diagnosed with chronic active Crohn's disease. Thirty subjects were enrolled; 18 were treated with natalizumab (3 mg/kg) and 12 with placebo. Two weeks following treatment, 7 natalizumab-treated subjects (39%) and 1 placebo-treated subject (8%) were in clinical remission (Crohn's disease Activity Index (CDAI) <150) (p=0.1). In addition, at Week 2 post-treatment, fewer natalizumab-treated subjects (11%) required rescue therapy compared to placebo-treated subjects (33%). Mean CDAI scores were significantly decreased at both 2 and 4 weeks post-treatment in the natalizumab group only, compared to mean baseline CDAI scores. These effects were not sustained beyond 4 weeks post-treatment and correlate with low natalizumab serum concentrations observed at the Week 4 time point.

Natalizumab treatment with a single, intravenous dose of 3 mg/kg was safe and well tolerated by subjects with CD. No subjects were withdrawn from the study because of the occurrence of an adverse event. Six subjects reported one serious adverse event; all subjects were in the natalizumab-treated group. None of these events were fatal. Five of the six events were admissions for relapses or worsening of the subject's Crohn's disease, the other serious adverse event was admission for anemia. There was no significant difference between natalizumab and placebo groups in the incidence of the most frequently reported adverse events (headache, Crohn's disease and abdominal pain).

A second earlier study was an open-label safety, tolerability, and efficacy study of a single infusion of 3 mg/kg intravenous natalizumab in male and female subjects with active ulcerative colitis. Ten subjects were recruited and treated with natalizumab (3 mg/kg).9 At 2 and 4 weeks post-treatment, 5 subjects (50%) had a good clinical response, defined as a Powell-Tuck Activity Index (PTAI) score of ≤5 and mean PTAI scores decreased from 9.7 at Week 0 to 6.9, 5.7, and 4.9 at 1, 2, and 4 weeks post-treatment, respectively. The mean PTAI scores remained suppressed for the 12-week study period. Seventy percent of subjects received no rescue medication between Weeks 0 and 4.

The most frequently reported adverse events in this study were aggravation of ulcerative colitis, headache, vomiting, lethargy, and sore throat. Of the 30 adverse events reported in this study, only 3 were considered to be related to study drug. These were one incidence each of headache, aggravation of ulcerative colitis, and lethargy. There were two events characterized severe; both events were reports of aggravated ulcerative colitis not considered to be related to treatment. Three subjects reported a serious adverse event. None of these events were fatal. These events were an incidence of *Campylobacter enterits*; a relapse of ulcerative colitis, which resulted in the subject withdrawing from the study; and an episode of rigors, fever, headache, and vomiting.

Another earlier study was a double-blind, placebo-controlled, parallel group, multicenter, efficacy, safety, and tolerability study of either one or two intravenous infusions of placebo, 3 or 6 mg/kg natalizumab in subjects with moderately to severely active Crohn's disease. A total of 248 subjects were randomized of whom 244 received at least one dose of study drug. Sixty-eight subjects were randomized to a single infusion of 3 mg/kg, 66 to two infusions of 3 mg/kg at a 4-week interval, 51 to two infusions of 6 mg/kg at a 4-week interval and 63 to receive placebo. Natalizumab was superior to placebo in inducing remission (CDAI < 150) in at least one of the three active treatment groups at Weeks 4, 6, 8, and 12. The highest remission rate of 46% was observed at Week 6 in the group that received two infusions of 3 mg/kg, remission rates of 41-43% were observed at Weeks 8 and 12 in this and the group that received two infusions of 6 mg/kg. Natalizumab was superior to placebo in inducing a response (≥70 point or ≥100 point drop in CDAI) in at least one of the three active treatment groups at Weeks 2, 4, 6, 8, and 12. The highest response rates of 73% (≥70 point drop) and 56% (≥100 drop) was observed at Week 6 in the group that received two infusions of 3 mg/kg. Statistically significant improvements in quality of life, assessed through the Inflammatory Bowel Disease Questionnaire, and decreases in C-reactive protein were also achieved.

Treatment with natalizumab appeared safe and well tolerated by subjects with active CD. Similar numbers of subjects from each treatment group withdrew due to adverse events: 2, 1, 2, and 3 subjects in the placebo, single 3.0 mg/kg, two 3 mg/kg and two 6 mg/kg infusion dose groups, respectively. A total of 32 subjects reported a serious adverse event during the main phase of the study (9, 8, 8, and 7 subjects in the placebo, single 3.0 mg/kg, two 3 mg/kg, and two 6 mg/kg infusion dose groups, respectively). None of these events were fatal and none were assessed as related to study drug. The majority of these events were admissions for treatment of complications or symptoms of CD. The non-disease-related events which were reported with greater frequency in at least two of the natalizumab treatment groups included chest pain, fever, flu syndrome, dizziness, and conjunctivitis.

### Use of Natalizumab in treatment of Crohn's Disease

The majority of subjects with CD will initially respond to the available medications including 5-ASA formulations (sulfasalazine, mesalazine, olsalazine), oral steroids (e.g., prednisolone, methlyprednisolone, budesonide). More recently, agents directed against tumor necrosis factor (the anti-TNF alpha antibody, infliximab) for the treatment of severe refractory CD and refractory fistulizing disease have been developed. However, some patients continue to have debilitating disease and there is a need for an improved treatment for subjects whose disease is not well controlled by current therapy.

There is evidence of up-regulation of MadCAM-1 and VCAM-1 in subjects with IBD, with evidence that the MadCAM-1/α4β7 interaction mediates the homing of lymphocytes to the gut. The potential role of anti-α4 integrin antibodies in IBD was initially supported by findings from studies in the cotton-top tamarin and more recently by the results of natalizumab in clinical trials.

Two further studies, described in detail below, were planned to confirm the earlier results and is designed to induce response and/or remission in a population of moderately to severely active CD subjects (CDAI ≥ 220, ≤ 450). Subjects from the first study who responded and then had mildly active disease (CDAI score < 220 and ≥ 70 drop) were enrolled in a subsequent study, which was designed to determine whether repeated administration of natalizumab can maintain response and/or remission. Given the chronic nature of CD it is clearly important that new agents are evaluated for their ability to reduce or eliminate disease activity over a longer period of time. In addition, the approach of maintaining an improvement once achieved also reflects aims of current clinical practice.

The primary tool for the assessment of efficacy is the Crohn's disease Activity Index (CDAI). The CDAI was developed for the US National Co-operative Crohn's Study (NCCDS) in 1979 and is the best known of the CD clinical scores. It is widely used in clinical trials of new therapies and has gained general acceptance as an endpoint for clinical activity. A CDAI score of < 150 is generally accepted as remission, scores of ≥ 150 to < 220 are considered mildly active disease whilst scores of ≥220 to < 450 are considered moderately to severely active disease.

Accordingly, a loss of response is defined as a CDAI score of ≥ 220 and a loss of remission as a CDAI score of ≥ 150. These definitions in combination with the use of rescue intervention, were used in the maintenance of response and remission analyses in this study.

Additional endpoints for this study included assessment through the Inflammatory Bowel Disease Questionnaire, a quality of life tool that has been developed for the IBD population, and the SF-3612 which affords a more generic assessment of quality of life and which is favored by some regulatory authorities. Changes in inflammatory markers such as C-reactive protein were also assessed, as will the ability to withdraw concomitant oral steroids in the sub group of subjects receiving them.

The initial dose of natalizumab selected for clinical evaluation was based on non-clinical studies in the guinea pig Experimental Allergic Encephalitis (EAE) model. These studies demonstrated that a dose of 3 mg/kg of natalizumab produced both a significant delay in onset and a reversal of the signs and symptoms of EAE; lower doses of natalizumab were not effective. A single dose of 3 mg/kg appeared to provide serum concentrations of natalizumab associated with α4 integrin receptor blockade for up to approximately 3 weeks, and 6 mg/kg for about 6 weeks.

Natalizumab has been evaluated in all clinical trials to date by administration of dose adjusted for bodyweight. Single dose pharmacokinetic data from completed clinical trials in healthy volunteers and in subjects with MS and IBD showed a 3 mg/kg infusion of natalizumab can maintain natalizumab serum concentrations of 2.5-3.0 µg/mL, levels that are associated with a sufficient degree of receptor saturation and the inhibition of cell adhesion for 3-4 weeks. A single infusion of a higher dose of 6 mg/kg natalizumab produced α4 integrin saturation levels which were slightly higher and more prolonged (approximately 6 weeks).

The pharmacodynamic effects and therapeutic response observed in these Phase II studies were found to be related to natalizumab dose and serum natalizumab concentrations. Based on these findings, along with the knowledge that natalizumab clearance is largely independent of bodyweight, the range of exposures produced by a fixed dose of 300 mg was investigated to determine if fixed dose administration could replace dosing adjusted by bodyweight.

Through pharmacokinetic modeling and assuming that AUC is proportional to total dose, it was demonstrated that a 300 mg fixed dose will produce natalizumab exposures that overlap the exposures observed for the 3 mg/kg and 6 mg/kg doses used in the Phase II trials. Thus, since the 3 mg/kg dose was efficacious in both CD and MS indications, the 6 mg/kg dose resulted in no evidence of dose-limiting toxicities and there was no added benefit of the 6 mg/kg dose over the 3 mg/kg dose, a 300 mg fixed dose is an appropriate choice for Phase III studies.

A double-blind, placebo-controlled study of the efficacy, safety, and tolerability of intravenous Tysabri (natalizumab, 300 mg monthly) in maintaining clinical response and remission in patients with Crohn's Disease (CD) was performed. The objectives were to compare the ability of natalizumab versus placebo to maintain a clinical response in subjects with CD, to compare the ability of natalizumab versus placebo to maintain a clinical remission in subjects with CD, to compare the effects of natalizumab versus placebo on quality of life as measured by the Inflammatory Bowel Disease Questionnaire (IBDQ), and to compare the ability of natalizumab versus placebo to allow subjects to achieve withdrawal of oral steroids.

### Study Design

A Phase III, international, multicenter, randomized, double-blind, placebo-controlled, parallel-group study of subjects with previously active Crohn's disease (CD) (defined as moderately to severely active, CDAI ≥220, <450) who have responded to treatment at Week 10 and maintained that response out to Week 12 in a first study (defined as a ≥70 point decrease in baseline CDAI) and whose disease is mildly active (defined as a CDAI score of <220) was undertaken.

Within this group there was a sub-population who had achieved remission (defined as a CDAI score of <150) at Week 10 of the first study. Subjects who failed to maintain response from Week 10 to Week 12 were not be eligible for the second study and continued in the safety follow-up phase of the first study. Subjects receiving concomitant medications for their CD were permitted to enroll providing that doses remained stable throughout their participation in the first study. All concomitant medications for CD remained stable for the duration of the 12-month treatment phase (up to Month 15) with the exception of oral steroids which were reduced according to a fixed algorithm). At the time this application was filed, only 9 months of data was available.

Natalizumab was administered 300 mg monthly for 12 infusions. The placebo was administered monthly for 12 infusions. Subjects were stratified according to their disease status (remission versus no remission, *i.e*., a CDAI <150 or ≥150), concomitant use of oral steroids and concomitant use of immunosuppressants.

Once randomized, subjects received their first infusion. Thereafter, they returned to the clinic on a monthly basis (where 1 month is defined as a 4-week period) for assessment and infusion. Introduction of any new medication for CD or a dose change to an existing concomitant medication for CD (with the exception of oral steroids withdrawn according to the fixed algorithm) was be permitted unless deemed necessary for purposes of rescue intervention. Once rescued, such a subject was considered a treatment failure.

Subjects will receive up to 12 infusions in this study and will return for the final treatment phase assessment 1 month after the last infusion (*i.e.,* at Month 15).

### Sample Size

Approximately 380 subjects were expected to respond to treatment and have mildly active disease at Week 10 in the first study (defined as ≥ 70 point decrease in CDAI score and a CDAI score of <220 and no use of rescue intervention), maintained to Week 12/Month 3. 285 were expected to enroll into the second study, assuming a 25% drop-out rate of eligible subjects between the two studies. Of these, 200 subjects were expected to have achieved remission (defined as CDAI score of < 150).

A sample size of 285 subjects randomized and dosed (142 per treatment group; ratio 1:1) were given a power of 90% at 5% significance to detect a difference between the natalizumab-treated group and the placebo group in maintenance of response rates (defined as a CDAI score of <220 and no use of rescue intervention), assuming a 65% response rate for natalizumab and a 44% response rate for placebo and allowing for a 10% drop-out rate.

Accordingly, the sub group of 200 subjects in remission, randomized and dosed were given a power of 90% at 5% significance to detect a difference between the natalizumab-treated group and the placebo group in maintenance of remission (defined as a CDAI score of <150 and no use of rescue intervention), assuming a 55% response rate for natalizumab and a 30% response rate for placebo and allowing for a 10% drop-out rate.

Eligible subjects at Week 10 in the first study were consented and enrolled into the second study, in order to allow subjects taking concomitant oral steroids to begin a steroid taper. Subjects who continued to meet the eligibility criteria at Week 12/Month 3, *i.e.,* in time for their next, monthly infusion, were re-randomized and entered the treatment phase which will last up to 12 months in duration (*i.e.,* up to Month 15). Subjects were male or female, eighteen years of age or older.

### Drug Dosage and Formulation

Intravenous natalizumab was administered at a dose of 300 mg. Natalizumab was provided in 5 mL vials at a concentration of 20 mg/mL. All infusions were made up in 100 mL bags of 0.9% saline. Natalizumab vials contained 20 mg/mL natalizumab in 10 mM phosphate buffer, 140 mM NaCl and a 0.02% polysorbate 80, adjusted to pH 6.0 with phosphoric acid.

For the control group, placebo was provided in matching 5-mL vials and comprised 10 mM phosphate buffer, 140 mM NaCl and 0.02% polysorbate 80, adjusted to a pH of 6.0 with phosphoric acid.

### Route of Administration

Natalizumab or placebo was administered by intravenous infusion over approximately 60 minutes, at a flow rate of 2 mL/min. All subjects will be observed for 2 hours post the start of each infusion.

### Procedures

Procedures included physical examination, vital signs, bodyweight, CDAI, IBDQ, SF-36, Subject Global Assessment, blood samples for assessment of hematology, biochemistry, C-reactive protein (CRP), anti-nuclear antibodies (ANA), serum natalizumab levels, anti-natalizumab antibodies and pregnancy testing, urine samples for urinalysis and pregnancy testing, assessment of adverse event, concomitant medications and rescue intervention.

### Primary, Secondary and Tertiary Endpoints

### Primary Endpoint:

The primary endpoint was time to loss of response (defined as a CDAI score 220 or use of rescue intervention) for subjects in response at Week 12.

### Secondary Endpoints:

1. Contingent Primary Endpoint: Time to loss of remission (defined as a CDAI score 150 or use of rescue intervention) for those subjects in remission at Week 12 (defined as a CDAI score <150).
2. Proportion (%) of those subjects in remission at Week 12 (defined as a CDAI score <150) who remained in remission (defined as a CDAI score <150 AND no use of rescue intervention) after 12 months (i.e., at Month 15).
3. Mean change in IBDQ from baseline in CD301, at Month 9.
4. Number (%) not taking oral steroids, at Month 9. Number (%) of subjects in remission (defined as a CDAI score <150 AND no use of rescue intervention) and not taking oral steroids, at Month 9.

### Tertiary Endpoints:

1. Number (%) of subjects with mildly active disease (defined as a CDAI score of <220 AND no use of rescue intervention), at Months 3, 4, 5, 6, 7, 8, 9, 10, 11, 12, 13, 14, and 15.
2. Number (%) of subjects in remission (defined as a CDAI score <150 AND no use of rescue intervention), at Months 3, 4, 5, 6, 7, 8, 9, 10, 11, 12, 13, 14, and 15.
3. Number (%) of subjects with CDAI score <200 AND no use of rescue intervention, at Months 3, 4, 5, 6, 7, 8, 9, 10, 11, 12, 13, 14, and 15.
4. Mean CDAI scores at Months 3, 4, 5, 6, 7, 8, 9, 10, 11, 12, 13, 14, and 15.
5. Mean change in IBDQ from baseline in CD301, at Months 3, 6, 12 and 15.
6. Mean change in SF36 from baseline in CD301, at Months 3, 6, 9, 12 and 15.
7. Mean change in Subject Global Assessment from baseline in CD301, at Months 3, 6, 9, 12, and 15.
8. Number (%) not taking oral steroids, at Months 3, 4, 5, 6, 7, 8, 10, 11, 12, 13, 14, and 15.
9. Number(%) of subjects in remission (defined as a CDAI score <150 AND no use of rescue intervention) and not taking oral steroids, at Months 3, 4, 5,6, 7, 8, 10, 11, 12, 13, 14, and 15.
10. Time to first use of rescue intervention (including surgical intervention).
11. Number (%) of subjects requiring rescue intervention (including surgical intervention), at Months 3, 4, 5, 6, 7, 8, 9, 10, 11, 12, 13, 14, and 15.
12. Mean change in C-reactive protein from baseline in CD301 (in those subjects who had an elevated CRP at baseline in CD301), at Months 3, 6, 9, 12, and 15.
13. Mean change in serum albumin from screening in CD301, at Months 3, 6, 9, 12, and 15.

### Statistical Considerations

The efficacy analyses was based on the intention-to-treat population. The analysis of the primary endpoint was repeated for a per protocol population. Categorical data was presented as counts and percentages. Continuous data was presented as summary statistics. All comparisons made were two-tailed at the 5% level of significance.

The primary analyses adjusted for the factors used for the stratification as well as geographical location and other pre-specified covariates. The Contingent Primary Endpoint, time to loss of remission, were only analyzed if the primary efficacy endpoint was statistically significant at the 5% level.

An administrative analysis will be carried out when 400 patient years of data are available from the first and second studies combined and when every subject will have a minimum of 3 months of data (*i*.*e*., have completed the first study to Week 12 as a minimum or have completed Week 12/Month 3 in the second study).

### Study Drug

Study drug was provided (either natalizumab or placebo) in clear, stoppered, individual 5 mL vials of either 20 mg/mL natalizumab or placebo. Vials were packaged in boxes of 3 vials to protect them from light. Each box was labeled with a unique 6 digit Box Number and will contain sufficient study drug for one infusion.

Natalizumab vials contained 20 mg/mL natalizumab in 10 mM phosphate buffer, 140 mM NaCl and 0.02% polysorbate 80, adjusted to pH 6.0 with phosphoric acid. Placebo was provided in matching 5 mL vials and comprises 10 mM phosphate buffer, 140 mM NaCl and 0.02% polysorbate 80, adjusted to pH 6.0 with phosphoric acid.

### Study Drug Dosage

Subjects randomized to natalizumab received 300 mg monthly (*i*.*e*., every 4 weeks) via an intravenous infusion for up to 12 infusions.

### Study Drug Reparation

Fifteen milliliters of saline, a volume equivalent to that of study drug to be added, were withdrawn from the 100 mL bag of 0.9% saline and discarded. A total of 15 mL of study drug was then drawn from the three vials into a 50 mL syringe via an 18 or 19 gauge needle, and gently added into the saline bag via a 20-23 gauge needle through the medication port diaphragm. The dilution was performed using aseptic technique.

### Study Drug Administration

Administration of study drug occurred every 4 weeks by intravenous infusion. Each infusion took approximately 60 minutes. The infusion was performed using a flow rate of approximately 2 mL/min. Once the 100 mL bag of study drug is empty, it was replaced with the 50 mL bag of saline, in order to flush the infusion line at the same rate.

### Oral Steroid Tapering

All subjects receiving oral steroids were required to undergo a taper immediately upon entry into the second study using the following algorithm. Subjects on doses equivalent to >10 mg of prednisolone will begin their taper at a rate of 5 mg every 7 days until they reach a dose of 10 mg. Subjects on doses equivalent to 10 mg of prednisolone were tapered at a rate of 2.5 mg every 7 days until they are completely withdrawn. Subjects taking budesonide were tapered at a rate of 3 mg every 3 weeks.

### Physical Examination

Complete physical examinations were and will be performed at Month 6, Month 9, Month 12, and Month 15 and as part of the Early Discontinuation Visit for subjects who withdrew before Month 15. Bodyweight was recorded at every visit as part of the assessment of the CDAI score. Vital signs were recorded at every visit. At visits when infusions are administered, vital signs were recorded immediately before (0 minutes) and at the end of the infusion.

### Quality of Life Assessments

Quality of life assessments, consisting of Subject Global Assessment, Inflammatory Bowel Disease Questionnaire (IBDQ), and a health survey were completed by the subject during the Month 3, Month 6, Month 9, Month 12 and Month 15 visits and as part of the Early Discontinuation Visit. Subjects must complete assessments at the beginning of the visit (*i.e.,* before any other assessments or the infusion), completing the Subject Global Assessment first. The Subject Global Assessment is a visual analog scale on which the subject assessed their global impression of how they feel compared to how they felt immediately prior to receiving their first administration of study medication.

### Natalizumab Concentration

Blood samples for natalizumab concentration measurement were collected during Month 3, Month 6, Month 9, Month 12, and Month 15. At visits when infusions were administered, the sample were taken immediately before (0 minutes) the infusion. In addition, at Month 6 and Month 12 only, a second sample was collected at least 1 hour from the end of the infusion.

### Statistical methods

The ability of natalizumab to maintain mildly active disease (defined as a CDAI score of <220 and no use of rescue intervention) and remission (defined as a CDAI score of <150 and no use of rescue intervention) in subjects with CD was assessed. The primary comparison of interest was the time to loss of response between treatment groups (where loss of response is defined as a CDAI score 220 or use of rescue intervention). A contingent sequential analysis was done on the effect of treatment on the time to the loss of remission between treatment groups (defined as CDAI score 150 or use of rescue intervention) in the sub-group of subjects in remission (CDAI <150) at Week 12.

Approximately 380 subjects were expected to respond to treatment and have mildly active disease at Week 10 in the first study (defined as ≥ 70 point decrease in CDAI score and a CDAI score of <220 and no use of rescue intervention) which is maintained to Week 12/Month 3. Of which, 285 were expected to enroll into the second study, assuming a 25% drop-out rate of eligible subjects between the two studies. Of these, 200 subjects were expected to have achieved remission (defined as CDAI score of <150).

A sample size of 285 subjects randomized and dosed (142 per treatment group; ratio 1:1) were given a power of 90% at 5% significance to detect a difference between the natalizumab-treated group and the placebo group in maintenance of response rates (defined as a CDAI score of <220 and no use of rescue intervention), assuming a 65% response rate for natalizumab and a 44% response rate for placebo and allowing for a 10% drop-out rate.

Accordingly, the sub group of 200 subjects in remission, randomized and dosed were given a power of 90% at 5% significance to detect a difference between the natalizumab-treated group and the placebo group in maintenance of remission (defined as a CDAI score of <150 and no use of rescue intervention), assuming a 55% response rate for natalizumab and a 30% response rate for placebo and allowing for a 10% drop-out rate.

### Efficacy Analysis

All efficacy analyses and summaries were based on the intention-to-treat population. A confirmatory analysis of the primary efficacy parameter was carried out using the per protocol population. A sensitivity analysis was carried out on the primary and secondary endpoints using a subset of the intention-to-treat population comprising of those responders from the first study who were randomized to receive natalizumab in the first study.

### Results

In the second study, subjects not taking steroids at month 9 (of those who took steroids at the first study baseline) showed the following results:

**Table 5**

| | Placebo N=76o | Natalizumab | p-value |
|---|---|---|---|
| Not taking steroids | 19 (25%) | 37 (55%) | < 0.001 |
| Remission and not taking steroids | 17 (22%) | 31 (46%) | 0.009 |

The most common side effects in both groups were headache, nausea and abdominal pain. Of serious adverse events (SAEs), 8% versus 7% placebo versus natalizumab.

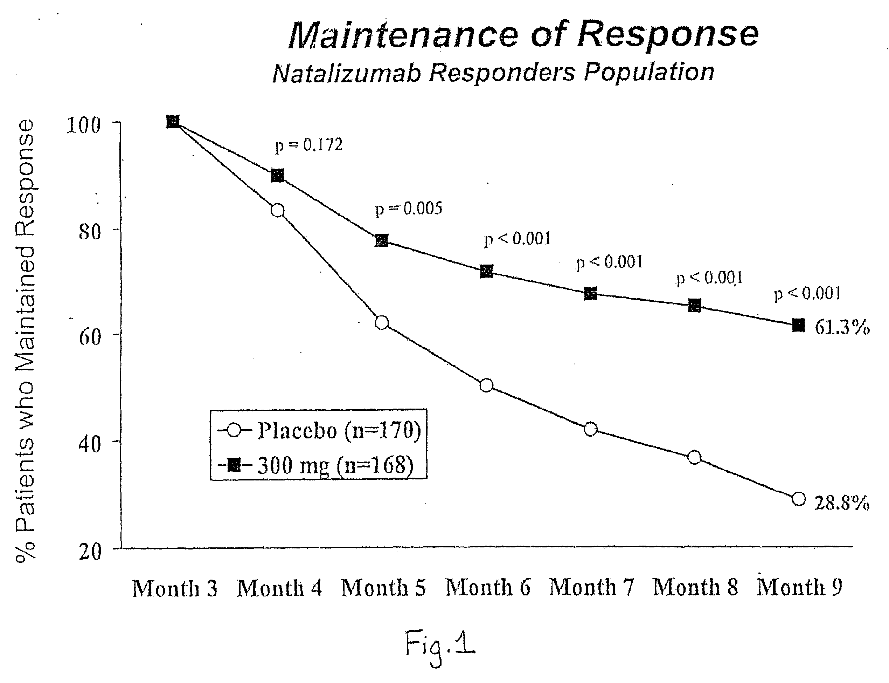

Figure 1 shows a graph of the response to natalizumab when given to patients in a Crohn's disease trial. Of the natalizumab responder population at three months into the trial, 61.3% of the patients maintained a response after 9 months, while only 28.8% of the placebo group maintained a response.

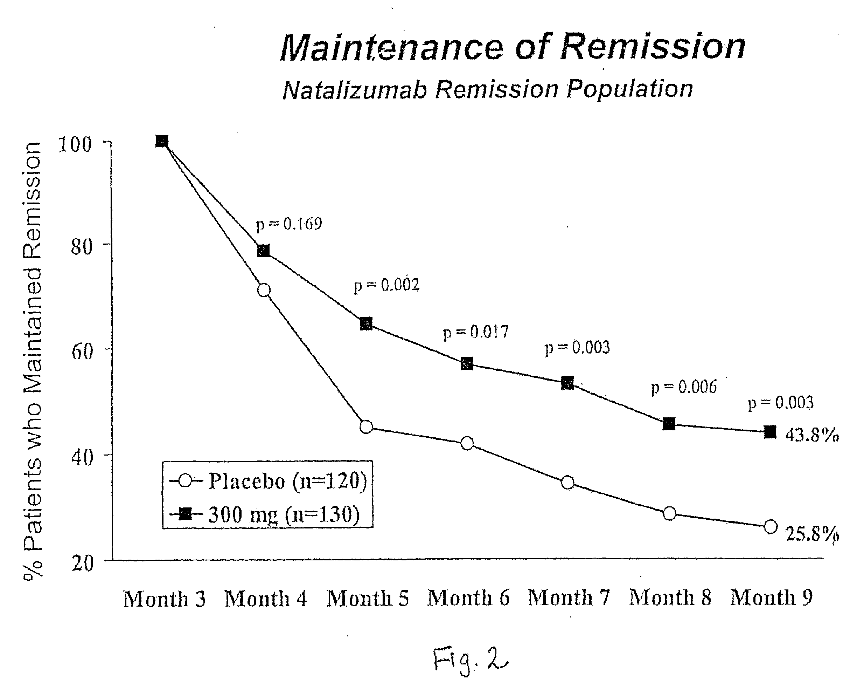

Figure 2 shows a graph of the level of remission in response to natalizumab when given to patients in a Crohn's disease trial. Of the natalizumab remission population at three months into the trial, 43.8% of the patients maintained a response after 9 months, while only 25.8% of the placebo group maintained a response.

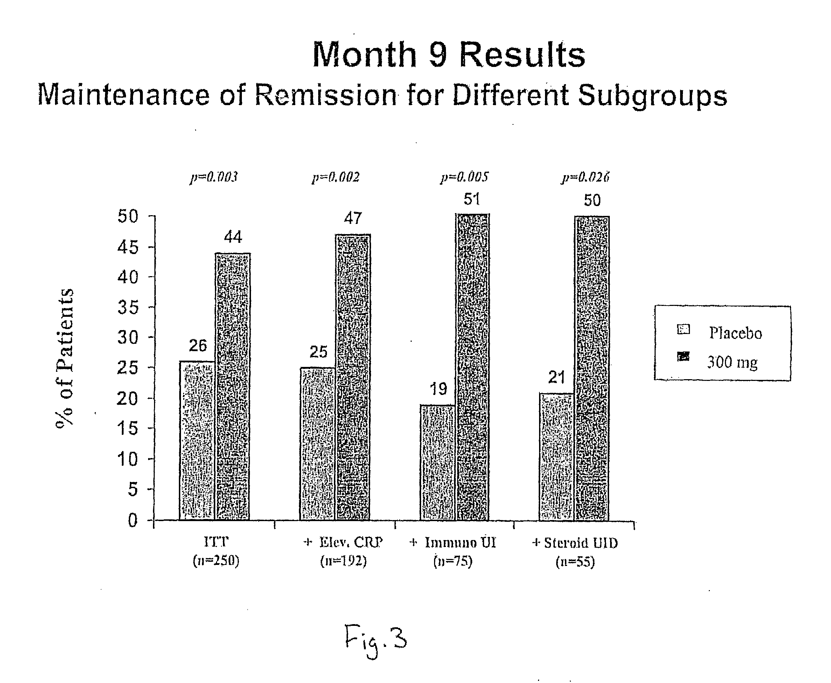

Figure 3 shows a graph of the level of remission in response to natalizumab when given to patients in a Crohn's disease trial in various populations: the intention-to-treat population (ITT), elevated C-reactive protein population (CRP), the population unresponsive or intolerant to immunosuppressives (immuno UI). and the population unresponsive, intolerant to, or dependent upon steroids (steroid UID). These categorizations were based upon patient history of previous use of these medications.

### Efficacy summary

In populations of interest, clinically meaningful differences in remission and response rates were observed with natalizumab compared to placebo in the first study. The second study, (double blind withdrawal study of responders in the first study) confirms the induction effect seen in the first study. Encouraging maintenance data observed after 6 months in the second study. In the second study, natalizumab enabled subjects to be successfully tapered off steroids.

**Table 6 CROHN'S DISEASE ACTIVITY INDEX (CDAI)**

| Variable | Weighting factor |
|---|---|
| Total number of diarrheal stools for each of previous 7 days | × 2 |
| Abdominal pain for each of previous 7 days | × 5 |
| None = 0 | |
| Mild = 1 | |
| Moderate = 2 | |
| Severe = 3 | |
| General well-being for each of previous 7 days | × 7 |
| Well = 0 | |
| Below par = 1 | |
| Poor = 2 | |
| Very poor = 3 | |
| Terrible = 4 | |

All other indices will be assessed by the Doctor at outpatient visit as follows:

**Table 7**

| | |
|---|---|
| Clinical signs during the previous 7 days | × 20 |
| Arthritis or arthralgia = 1 | |
| Skin or mouth lesions = 1 | |
| Iritis or uveitis = 1 | |
| Anorectal lesion = 1 | |
| Other fistulae = 1 | |
| Fever over 38°C during the week = 1 | |
| Lomotil or other anti-diarrheal | × 30 |
| No = 0, yes = 1 | |
| Abdominal mass | ×10 |
| None = 0 | |
| Questionable = 2 | |
| Definite = 5 | |
| Anemia defined by hematocrit less than: | × 6 |
| For males-47% | |
| For females-42% | |
| Standard weight-Actual weight × 100 | × 1 |
| Standard weight* | |
| Crohn's disease Activity Index (CDAI) Total | = |

| | |
|---|---|
| * Obtain from the Standard Height and Weight Tables which will be provided. | |

### Example 10

This experiment was a double-blind, placebo-controlled study of the efficacy, safety, and tolerability of natalizumab in maintaining clinical response and remission in Crohn's Disease.

Natalizumab, a humanized monoclonal IgG4 antibody to α4 integrin, was evaluated in a randomized, controlled study to determine the ability of a six month regimen to maintain clinical response/remission achieved in natalizumab-treated subjects in the Phase III induction of response/remission study.

### Methods

339 adult subjects with Crohn's disease (CD) who achieved response (≥70-joint reduction in baseline Crohn's Disease Activity Index (CDAI)) and/or remission (CDAI <150) and had a CDAI score <220 after receiving three infusions of natalizumab in a first study were re-randomized in a 1:1 ratio to natalizumab (300 mg) (n=168) or placebo (n=171) for up to 12 additional monthly infusions. The primary endpoint was the proportion of subjects that did not lose that response from the first study at every time point for an additional 6 consecutive months in the second study. Loss of response was defined as a CDAI ≥220 and ≥70-point increase from baseline CDAI in the second study or use of rescue intervention. Maintenance of remission was also assessed.

### Results

At 6 months, 61% (103/168) of natalizumab-treated subjects (ITT population) continued to meet the criteria for clinical response versus 29% (49/170) of subjects re-randomized to receive placebo (p<0.001). 44% (57/130) in the natalizumab treatment group maintained clinical remission, compared with 26% (31/120) in the placebo group (p=0.003). In addition, 55% (37/67) of natalizumab-treated subjects taking steroids in the first study re-randomized to natalizumab in the second study were withdrawn from steroids, compared to 25% (19/76) re-randomized to placebo (p<0.001). No notable difference in the rates of serious and non-serious adverse events between treatment groups was observed.

In the second study, natalizumab demonstrated significant superiority over placebo in its ability to sustain response and remission at all consecutive time points over a 6-month period in the first study natalizumab-responders. Monthly administration of natalizumab for 6 months was well tolerated and enabled subjects to be successfully withdrawn from steroids.

## Claims

1. Use of natalizumab or an immunologically active fragment thereof for the preparation of a medicament to be administered to a human subject in a steroid sparing effective amount to reduce and/or eliminate a need for steroid treatment in the human subject with a disease selected from the group consisting of inflammatory bowel disease, asthma, multiple sclerosis, rheumatoid arthritis, graft versus host disease, host versus graft disease, spondyloarthropathies, and combinations thereof, wherein the human subject is under treatment with steroids.

2. The use of claim 1, wherein natalizumab is administered parenterally to the human subject in need thereof.

3. The use of claim 1, wherein natalizumab is administered chronically to the human subject in need thereof.

4. The use of claim 2, wherein the parenteral administration results in an effective blood level of natalizumab of at least 1 ng/mL in said human subject.

5. The use of claim 4, wherein the effective blood level of natalizumab is 1 ng/mL in said human subject.

6. The use of claim 3, wherein the chronic administration of natalizumab is weekly or monthly over a period of at least one year.

7. The use of claim 1, wherein the disease is inflammatory bowel disease, wherein natalizumab is administered in an amount effective to permit the human subject to be tapered from steroid therapy.

8. The use of claim 7, wherein the inflammatory bowel disease is Crohn's disease or ulcerative colitis.

9. The use of claim 1, wherein the disease is multiple sclerosis, wherein natalizumab is administered in an amount to permit the human subject to be tapered from steroid therapy.

10. The use of claim 8 or 9, wherein natalizumab is administered to said human subject in an amount of 2 mg/kg to 8 mg/kg.

11. The use of claim 1, wherein the disease is rheumatoid arthritis, wherein natalizumab is administered in an amount sufficient to permit the human subject to be tapered from steroid therapy.

12. The use of claim 1, wherein the disease is host versus graft disease or graft versus host disease, wherein natalizumab is administered in an amount to permit the human subject to be tapered from steroid therapy.

13. The use of claim 1, wherein the disease is asthma, wherein the natalizumab is administered in an amount to permit the human subject to be tapered from steroid therapy.

14. The use of claim 1, wherein the disease is a spondyloarthropathy, wherein natalizumab is administered in an amount to permit the human subject to be tapered from steroid therapy.

15. The use of claim 14, wherein the spondyloarthropathy is selected from the group consisting of ankylosing spondylitis, psoriatic arthritis, Reiter's syndrome, spondylitis of inflammatory bowel disease, undifferentiated spondyloarthropathy, and juvenile spondylarthropathy.

16. The use of any of claims 7, 10, 11, 12, 13 or 14, wherein the human subject is refractory, intolerant or dependent on steroids.

17. The use of claim 16, wherein the human subject requires a therapeutically effective amount of steroids that is less than would be required in the absence of administering natalizumab.

18. The use of any of claims 7, 10, 11, 12, 13 or 14, wherein the human subject is: a) a patient that is unresponsive or intolerant to treatment with immunosuppressive agents; b) a patient that is unresponsive, intolerant or dependent on treatment with steroids; or c) a patient that is a combination of a) and b).

19. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of an inflammatory bowel disease to be administered to reduce and/or eliminate a need for steroid treatment in the human subject.

20. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of multiple sclerosis to be administered to reduce and/or eliminate a need for steroid treatment in the human subject.

21. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of rheumatoid arthritis to be administered to reduce and/or eliminate a need for steroid treatment in the human subject.

22. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of host versus graft or graft versus host disease to be administered to reduce and/or eliminate a need for steroid treatment in the human subject.

23. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of asthma to be administered to reduce and/or eliminate a need for steroid treatment in the human subject.

24. Use of a steroid sparing effective amount of natalizumab or an immunologically active fragment thereof and a second agent selected from the group consisting of: (i) an immunosuppressant, wherein the immunosuppressant is not a steroid; (ii) an anti-TNF composition; (iii) a 5-ASA composition; and (iv) combinations thereof for preparing a medicament combination for the combination therapy of a spondyloarthropathy to be administered to reduce and/or eliminate a need for steroid treatment in the human subject.

25. The use of any of claims 19-24, wherein the combination therapy comprises a therapeutically effective amount of a second steroid sparing agent.

26. The use of any of claims 19-24, wherein the immunosuppressant is selected from the group consisting of azathioprine, 6-mercaptopurine, methotrexate, and mycophenolate.

27. The use of any of claims 19-24, wherein the anti-TNF composition is infliximab.

28. The use of any of claims 19-24, wherein the 5-ASA composition is selected from the group consisting of sulfasalazine, mesalazine, and osalazine.

## Patentansprüche

1. Verwendung von Natalizumab oder eines immunologisch aktiven Fragments davon für die Herstellung eines Medikaments, das einem Menschen in einer Steroid sparenden wirksamen Menge verabreicht werden soll, um die Notwendigkeit einer Steroid-Behandlung bei einem Menschen mit einer Erkrankung ausgewählt aus der Gruppe, bestehend aus entzündlicher Darmerkrankung, Asthma, multiple Sklerose, rheumatoide Arthritis, Graft-versus-Host-Reaktion, Host-versus-Graft-Reaküon, Spondyloarthropathien und Kombinationen davon, zu reduzieren und/oder zu eliminieren, wobei der Mensch unter Behandlung mit Steroiden ist.

2. Verwendung nach Anspruch 1, wobei Natalizumab parenteral dem Menschen, der dessen bedarf, verabreicht wird.

3. Verwendung nach Anspruch 1, wobei Natalizumab chronisch dem Menschen, der dessen bedarf, verabreicht wird.

4. Verwendung nach Anspruch 2, wobei die parenterale Verabreichung zu einem effektiven Blutspiegel von Natalizumab in dem Menschen von mindestens 1 ng/mL führt.

5. Verwendung nach Anspruch 4, wobei der wirksame Blutspiegel von Natalizumab in dem Menschen 1 ng/mL ist.

6. Verwendung nach Anspruch 3, wobei die chronische Verabreichung von Natalizumab wöchentlich oder monatlich über einen Zeitraum von mindestens einem Jahr erfolgt.

7. Verwendung nach Anspruch 1, wobei die Erkrankung eine entzündliche Darmerkrankung ist, wobei Natalizumab in einer wirksamen Menge verabreicht wird, um dem Menschen ein Ausschleichen aus der Steroid-Therapie zu ermöglichen.

8. Verwendung nach Anspruch 7, wobei die entzündliche Darmerkrankung Morbus Crohn oder Colitis ulcerosa ist.

9. Verwendung nach Anspruch 1, wobei die Erkrankung Multiple Sklerose ist und Natalizumab in einer Menge verabreicht wird, um dem Menschen ein Ausschleichen aus der Steroid-Therapie zu ermöglichen.

10. Verwendung nach Anspruch 8 oder 9, wobei Natalizumab dem Menschen in einer Menge von 2 mg/kg bis 8 mg/kg verabreicht wird.

11. Verwendung nach Anspruch 1, wobei die Erkrankung rheumatische Arthritis ist, wobei Natalizumab in einer Menge verabreicht wird, um dem Menschen ein Ausschleichen aus der Steroid-Therapie zu ermöglichen.

12. Verwendung nach Anspruch 1, wobei die Erkrankung Host-versus-Graft-Reaktion oder Graft-versus-Host-Reaktion ist, wobei Natalizumab in einer Menge verabreicht wird, um dem Menschen ein Ausschleichen aus der Steroid-Therapie zu ermöglichen.

13. Verwendung nach Anspruch 1, wobei die Erkrankung Asthma ist, wobei Natalizumab in einer Menge verabreicht wird, um dem Menschen ein Ausschleichen aus der Steroid-Therapie zu ermöglichen.

14. Verwendung nach Anspruch 1, wobei die Erkrankung Spondyloarthropathie ist, wobei Natalizumab in einer Menge verabreicht wird, um dem Menschen ein Ausschleichen aus der Steroid-Therapie zu ermöglichen.

15. Verwendung nach Anspruch 14, wobei Spondyloarthropathie ausgewählt ist aus der Gruppe bestehend aus Spondylitis ankylosans, psoriatische Arthritis, Reiter-Syndrom, Spondylitis bei entzündlicher Darmerkrankung, undifferenzierte Spondyloarthropathie und juvenile Spondylarthropathien.

16. Verwendung nach einem der Ansprüche 7, 10, 11, 12, 13 oder 14 wobei der Mensch refraktär, intolerant oder abhängig von der Behandlung mit Steroiden ist.

17. Verwendung nach Anspruch 16, wobei der Mensch eine therapeutisch wirksame Menge von Steroiden benötigt, die weniger ist als sie in Abwesenheit der Verabreichung von Natalizumab erforderlich wäre.

18. Verwendung nach einem der Ansprüche 7, 10, 11, 12, 13 oder 14 wobei der Mensch a) ein Patient ist, der nicht-ansprechend oder intolerant gegenüber einer Behandlung mit Immunsuppressiva ist; b) ein Patient, der nicht-ansprechend, intolerant oder abhängig von der Behandlung mit Steroiden ist; oder c) ein Patient, der eine Kombination aus a) und b) ist.

19. Verwendung einer steroidsparenden wirksamen Menge von Natalizumab oder ein immunologisch aktives Fragment davon und ein zweites Mittel ausgewählt aus der Gruppe bestehend aus: (i) ein Immunsuppressivum, wobei das Immunsuppressivum kein Steroid ist; (ii) eine anti-TNF Zusammensetzung; (iii) eine 5-ASA Zusammensetzung; und (iv) eine Kombinationen davon, für die Herstellung einer Medikamentenkombination für die Kombinationstherapie von einer entzündlichen Darmerkrankung, die verabreicht wird, um einen Steroid-Behandlungsbedarf im Menschen zu reduzieren und/oder zu eliminieren.

20. Verwendung einer steroidsparenden wirksamen Menge von Natalizumab oder ein immunologisch aktives Fragment davon und ein zweites Mittel ausgewählt aus der Gruppe bestehend aus: (i) einem Immunsuppressivum, wobei das Immunsuppressivum kein Steroid ist; (ii) eine anti-TNF Zusammensetzung; (iii) eine 5-ASA Zusammensetzung; und (iv) Kombinationen davon für die Herstellung einer Medikamentenkombination für die Kombinationstherapie von Multipler Sklerose, die verabreicht wird, um einen Steroid-Behandlungsbedarf im Menschen zu reduzieren und/oder zu eliminieren.

21. Verwendung einer steroidsparenden wirksamen Menge von Natalizumab oder ein immunologisch aktives Fragment davon und ein zweites Mittel ausgewählt aus der Gruppe bestehend aus: (i) einem Immunsuppressivum, wobei das Immunsuppressivum kein Steroid ist; (ii) eine anti-TNF Zusammensetzung; (iii) eine 5-ASA Zusammensetzung; und (iv) Kombinationen davon für die Herstellung einer Medikamentenkombination für die Kombinationstherapie von rheumatoider Arthritis, die verabreicht wird, um einen Steroid-Behandlungsbedarf im Menschen zu reduzieren und/oder zu eliminieren.

22. Verwendung einer steroidsparenden wirksamen Menge von Natalizumab oder ein immunologisch aktives Fragment davon und ein zweites Mittel ausgewählt aus der Gruppe bestehend aus: (i) einem Immunsuppressivum, wobei das Immunsuppressivum kein Steroid ist; (ii) eine anti-TNF Zusammensetzung; (iii) eine 5-ASA Zusammensetzung; und (iv) Kombinationen davon für die Zubereitung einer Medikamentenkombination für die Kombinationstherapie von Host-versus-Graft-Disease oder Graft-versus-Host-Disease, die verabreicht wird, um einen Steroid-Behandlungsbedarf im Menschen zu reduzieren und/oder zu eliminieren.

23. Verwendung einer steroidsparenden wirksamen Menge von Natalizumab oder ein immunologisch aktives Fragment davon und ein zweites Mittel ausgewählt aus der Gruppe bestehend aus: (i) einem Immunsuppressivum, wobei das Immunsuppressivum kein Steroid ist; (ii) eine anti-TNF Zusammensetzung; (iii) eine 5-ASA Zusammensetzung; und (iv) Kombinationen davon für die Herstellung einer Medikamentenkombination für die Kombinationstherapie von Asthma, die verabreicht wird, um einen Steroid-Behandlungsbedarf im Menschen zu reduzieren und/oder zu eliminieren.

24. Verwendung einer steroidsparenden wirksamen Menge von Natalizumab oder ein immunologisch aktives Fragment davon und ein zweites Mittel ausgewählt aus der Gruppe bestehend aus: (i) einem Immunsuppressivum, wobei das Immunsuppressivum kein Steroid ist; (ii) eine anti-TNF Zusammensetzung; (iii) eine 5-ASA Zusammensetzung; und (iv) Kombinationen davon für die Herstellung einer Medikamentenkombination für die Kombinationstherapie von Spondyloarthropathie, die verabreicht wird, um einen Steroid-Behandlungsbedarf im Menschen zu reduzieren und/oder zu eliminieren.

25. Verwendung nach einem der Ansprüche 19-24, wobei die Kombinationstherapie eine therapeutisch wirksame Menge eines zweiten steroidsparenden Mittels umfasst.

26. Verwendung nach einem der Ansprüche 19-24, wobei das Immunsuppressivum ausgewählt ist aus der Gruppe bestehend aus Azathioprin, 6-Mercaptopurin, Methotrexat und Mycophenolat.

27. Verwendung nach einem der Ansprüche 19-24, wobei die anti-TNF Zusammensetzung Infliximab ist.

28. Verwendung nach einem der Ansprüche 19-24, wobei die 5-ASA Zusammensetzung ausgewählt ist aus der Gruppe, bestehend aus Sulfasalazin, Mesalazin und Osalazine.

## Revendications

1. Utilisation du natalizumab ou d'un fragment actif immunologiquement de ce dernier pour la préparation d'un médicament à administrer à un sujet humain en une quantité efficace permettant d'épargner des stéroïdes, pour réduire et/ou éliminer le besoin d'un traitement aux stéroïdes chez le sujet humain affecté d'une maladie sélectionnée parmi le groupe composé d'une maladie inflammatoire de l'intestin, de l'asthme, de la sclérose en plaques, de l'arthrite rhumatoïde, de la maladie du greffon contre l'hôte, de la maladie de l'hôte contre le greffon, des spondylarthropathies, et de combinaisons de ces dernières, dans laquelle le sujet humain est en traitement par des stéroïdes.

2. Utilisation selon la revendication 1, dans laquelle le natalizumab est administré par voie parentérale au sujet humain qui en a besoin.

3. Utilisation selon la revendication 1, dans laquelle le natalizumab est administré de manière chronique au sujet humain qui en a besoin.

4. Utilisation selon la revendication 2, dans laquelle l'administration par voie parentérale résulte en un niveau sanguin efficace du natalizumab d'au moins 1 ng/ml chez ledit sujet humain.

5. Utilisation selon la revendication 4, dans laquelle le niveau sanguin efficace du natalizumab est de 1 ng/ml chez ledit sujet humain.

6. Utilisation selon la revendication 3, dans laquelle l'administration chronique de natalizumab est hebdomadaire ou mensuelle pendant une période d'au moins un an.

7. Utilisation selon la revendication 1, dans laquelle la maladie est la maladie inflammatoire de l'intestin, dans laquelle le natalizumab est administré en une quantité efficace pour permettre que le sujet humain soit moins soumis au traitement aux stéroïdes.

8. Utilisation selon la revendication 7, dans laquelle la maladie inflammatoire de l'intestin est la maladie de Crohn ou la colite ulcéreuse.

9. Utilisation selon la revendication 1, dans laquelle la maladie est la sclérose en plaques, dans laquelle le natalizumab est administré en une quantité pour permettre que le sujet humain soit moins soumis au traitement aux stéroïdes.

10. Utilisation selon la revendication 8 ou 9, dans laquelle le natalizumab est administré audit sujet humain en une quantité de 2 mglkg à 8 mg/kg.

11. Utilisation selon la revendication 1, dans laquelle la maladie est l'arthrite rhumatoïde, dans laquelle le natalizumab est administré en une quantité suffisante pour permettre que le sujet humain soit moins soumis au traitement aux stéroïdes.

12. Utilisation selon la revendication 1, dans laquelle la maladie est la maladie de l'hôte contre le greffon ou du greffon contre l'hôte, dans laquelle le natalizumab est administré en une quantité pour permettre que le sujet humain soit moins soumis au traitement aux stéroïdes.

13. Utilisation selon la revendication 1, dans laquelle la maladie est l'asthme, dans laquelle le natalizumab est administré en une quantité pour permettre que le sujet humain soit moins soumis au traitement aux stéroïdes.

14. Utilisation selon la revendication 1, dans laquelle la maladie est une spondylarthropathie, dans laquelle le natalizumab est administré en une quantité pour permettre que le sujet humain soit moins soumis au traitement aux stéroïdes.

15. Utilisation selon la revendication 14, dans laquelle la spondylarthropathie est sélectionnée par le da groupe composé de la spondylarthrite ankylosante, de l'arthrite psoriasique, du syndrome de Reiter, de la spondylite de la maladie inflammatoire de l'intestin, de la spondylarthropathie indifférenciée, et de la spondylarthropathie juvénile.

16. Utilisation selon l'une quelconque des revendications 7, 10, 11, 12, 13 ou 14, dans laquelle le sujet humain est résistant, intolérant aux ou dépendant des stéroïdes.

17. Utilisation selon la revendication 16, dans laquelle le sujet humain a besoin d'une quantité thérapeutiquement efficace de stéroïdes qui est inférieure à celle qui serait nécessaire en l'absence de l'administration de natalizumab.

18. Utilisation selon l'une quelconque des revendications 7, 10, 11, 12, 13 ou 14, dans laquelle le sujet humain est: a) un patient qui ne répond pas ou est intolérant au traitement avec des agents immunosuppresseurs; b) un patient qui ne répond pas, est intolérant aux ou dépendant du traitement avec des stéroïdes; ou c) un patient qui est une combinaison de a) et b).

19. Utilisation d'un quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaison d'une maladie inflammatoire de l'intestin à administrer pour réduire et/ou éliminer le besoin d'un traitement aux stéroïdes chez le sujet humain.

20. Utilisation d'une quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaison de la sclérose en plaques à administrer pour réduire et/ou éliminer le besoin d'un traitement aux stéroïdes chez le sujet humain.

21. Utilisation d'une quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaison de l'arthrite rhumatoïde à administrer pour réduire et/ou éliminer le besoin d'un traitement aux stéroïdes chez le sujet humain.

22. Utilisation d'une quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaison de la maladie de l'hôte contre le greffon ou du greffon contre l'hôte à administrer pour réduire et/ou éliminer le besoin d'un traitement aux stéroïdes chez le sujet humain.

23. Utilisation d'une quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaison de l'asthme à administrer pour réduire et/ou éliminer le besoin d'un traitement aux stéroïdes chez le sujet humain.

24. Utilisation d'une quantité de natalizumab efficace permettant d'épargner des stéroïdes, ou d'un fragment immunologiquement actif de ce dernier et d'un deuxième agent sélectionné parmi le groupe composé de: (i) un immunosuppresseur, où l'immunosuppresseur n'est pas un stéroïde; (ii) une composition anti-TNF; (iii) une composition 5-ASA; et (iv) des combinaisons de ces derniers pour la préparation d'une combinaison de médicament pour la thérapie par combinaisond'une spondylarthropathie à administrer pour réduire et/ou éliminer le besoin d'un traitement aux stéroïdes chez le sujet humain.

25. Utilisation selon l'une quelconque des revendications 19 à 24, dans laquelle la thérapie par combinaison comprend une quantité thérapeutiquement efficace d'un deuxième agent permettant d'épargner des stéroïdes.

26. Utilisation selon l'une quelconque des revendications 19 à 24, dans laquelle l'immunosuppresseur est sélectionné parmi le groupe composé de l'azathioprine, de la 6-mercaptopurine, du méthotrexate, et du mycophénolate.

27. Utilisation selon l'une quelconque des revendications 19 à 24, dans laquelle la composition anti-TNF est l'infliximab.

28. Utilisation selon l'une quelconque des revendications 19 à 24, dans laquelle la composition 5-ASA est sélectionnée parmi le groupe composé de la sulfasalazine, de la mésalazine, et de l'osalazine.
